# EUROPEAN PATENT APPLICATION

(11) **EP 3 492 483 A1**
(43) Date of publication of application: **05.06.2019**
(21) Application number: 18214928.6
(22) Date of filing: 22.10.2010
(51) Int. Cl.: C07H 15/18, C07H 15/203, C07H 15/26, C07H 17/00, C07H 17/04, C07H 17/075, A61K 31/70, A61P 31/02, A61P 31/04

(54) **COMPOUNDS AND METHODS FOR TREATING BACTERIAL INFECTIONS**

(30) Priority: 22.10.2009 US 25413509 P; 07.04.2010 US 32173810 P; 20.09.2010 US 38453510 P
(62) Divisional of application: 10825785.8
(71) Applicant: The Washington University, St. Louis, MO 63130 (US)
(72) Inventor: Janetka, James W., St. Louis, MO 63130 (US); Han, Zhenfu, St. Louis, MO 63130 (US); Hultgren, Scott, St. Louis, MO 63130 (US); Pinkner, Jerry, St. Louis, MO 63130 (US); Cusumano, Corinne, St. Louis, MO 63130 (US)
(74) Representative: Greaves Brewster LLP

(57) **Abstract**

The present invention encompasses compounds and methods for treating urinary tract infectons.

## Description

### GOVERNMENTAL RIGHTS

This invention was made with government support under grants numbered 1RC1DK086378, RO1AI029549, P50DK064540 and RO1BK051406-12 each of which were awarded by the National Institutes of Health. The government has certain rights in the invention.

### CROSS REFERENCE TO RELATED APPLICATIONS

This application claims the priority of US provisional application number 61/254,135, filed October 22, 2009; US provisional application number 61/384,535, filed September 20, 2010; and US provisional application number 61/321,738, filed April 7, 2010, which is hereby incorporated by reference in its entirety.

### FIELD OF THE INVENTION

The present invention encompasses compounds and methods for treating urinary tract infections.

### BACKGROUND OF THE INVENTION

Urinary tract infection (UTI) caused by uropathogenic *Escherichia coli* (UPEC) is one of the most common infectious diseases in women. The morbidity and economic impact are enormous, with over $2.5 billion spent annually on treatment. Further, recurrent infections are a significant problem despite appropriate antibiotic therapy of the index case. The high rates of recurrence, and the large numbers of women that end up in urology clinics due to their chronic recurrent UTIs highlights the need for a better understanding of the pathogenic mechanisms involved in this disease and the development of new and better therapies.

Gram-negative bacteria are the causative agents of a wide variety of acute and chronic infectious diseases. Many of these infections are initiated by a critical interaction between host ligands (frequently polysaccharide moieties) and bacterial adhesins (frequently expressed at the distal tip of polymeric pilus fibers assembled by the chaperone/usher pathway). The mannose binding FimH adhesin of type 1 pili is critical for the colonization and invasion into the bladder epithelium. After invasion, UPEC are able to rapidly multiply inside superficial umbrella cells of the bladder forming biofilm-like intracellular bacterial communities (IBCs). Upon maturation, bacteria disperse from the IBC, spread to neighboring cells, and form next generation IBCs. This is the mechanism by which UPEC rapidly amplify in numbers in the urinary tract and cause disease.

The X-ray crystal structure of FimH bound to mannose showed that mannose is bound in a negatively charged pocket on FimH. The mannose binding site is highly conserved as it is invariant in 300 *fimH* genes sequenced from clinical UPEC strains. Thus, FimH is the critical node of the entire UPEC pathogenic cascade.

Recurrence is a serious problem for many women. Women who present with an initial episode of acute UTI have a 25-44% chance of developing a second and a 3% chance of experiencing three episodes within six months of the initial UTI. Recurrence occurs despite appropriate antibiotic treatment and clearance of the initial infection from the urine. A large percentage of recurrent UTI are caused by the same strain of bacteria as the initial infection. One study followed 58 women and found that 68% of recurrences were caused by the same initial index strain of UPEC as determined by restriction fragment length polymorphism (RFLP) analysis. In a separate study, 50% of recurrent strains isolated from female college students appeared genotypically identical to the bacterial strain corresponding to the initial UTI. Another long-term prospective study demonstrated that the same strain of UPEC can cause a recurrent UTI up to 3 years later. The high frequency of same-strain recurrences supports the notion that a UPEC reservoir can exist in the affected individual. The inventors have shown that a quiescent intracellular reservoir (QIR) can form in the bladder tissue itself after acute infection and persist even after antibiotic therapy and urine cultures become sterile. Thus, reactivation of bacteria in QIRs may also be a contributing factor in recurrent UTIs.

Therefore, there is a need for effective treatments that can cure urinary tract infections and prevent the formation of quiescent intracellular reservoir that are the source of so many recurrent infections.

### SUMMARY OF THE INVENTION

One aspect of the present invention encompasses a compound comprising formula (I): 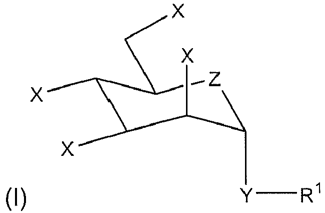 wherein:
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R² is independently selected from the group consisting of hydrogen, COR^{x}, CONR^{x}, hydrocarbyl, and substituted hydrocarbyl;
R³, R⁴, R⁵ are independently selected from the group consisting of hydrogen, hydrocarbyl, and substituted hydrocarbyl;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, NR⁵R⁵, and an optionally substituted alkyl, cycloalkyl, heterocycle, or aryl;
n is an integer from 1 to 10; and
R¹ is selected from the group comprising Formulas (IIIB), (IV), (V), (VI), (VII) and (VIII): 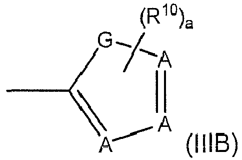 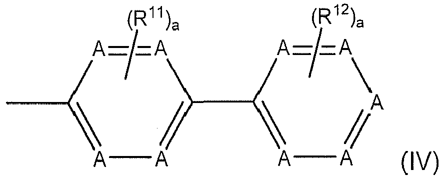 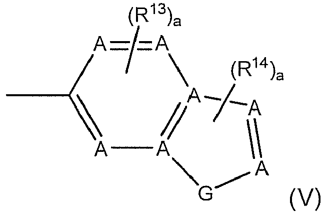 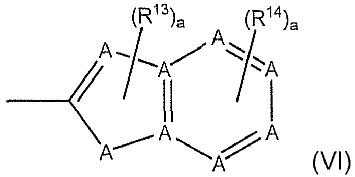 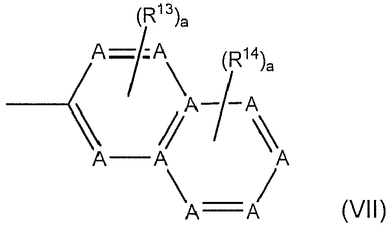 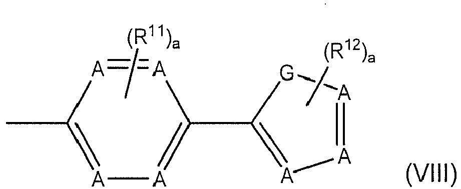 wherein:
   A is independently selected from the group consisting of CR⁶ and N;
   G is selected from the group consisting of S, O, CR⁸, and NR⁹;
   R⁶, R⁸ and R⁹ are independently selected from the group consisting of hydrogen, hydrocarbyl, and substituted hydrocarbyl;
   R¹⁰, R¹¹, R¹², R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, alkenyl, alkynyl, hydrocarbyl, and substituted hydrocarbyl;
   R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
   a is either the integer 1 or the integer 2.

Another aspect of the present invention encompasses a compound of formula (II): 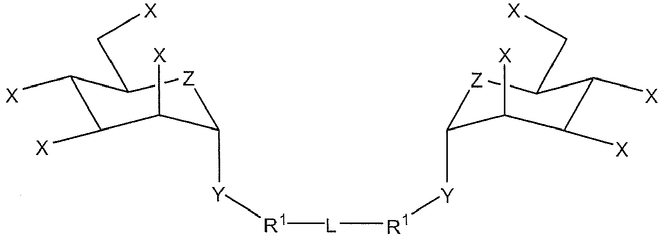
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
L is selected from -O(CH₂)ₙO-, -S(CH₂)ₙS-, -N(CH₂)ₙN-, -(CH₂)ₙ-, -O[(CH₂)ₘO(CH₂)ₙ]ₓO-, -N(CH₂)ₘO(CH₂)ₙN-, heterocycle, alkene, alkyne, -CON[(CH₂)ₘO(CH₂)ₙ]ₓNCO-, -SO₂N[(CH₂)ₘO(CH₂)ₙ]ₓNO₂S-, and -NCO[(CH₂)ₘO(CH₂)ₙ]ₓCON-, where L is bound to a ring of R¹ at a meta or para position;
R² is independently selected from the group consisting of hydrogen, COR^{x}, CONR^{x}, hydrocarbyl, and substituted hydrocarbyl;
R³, R⁴, R⁵ are independently selected from the group consisting of hydrogen, hydrocarbyl, and substituted hydrocarbyl,
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, NR⁵R⁵, and an optionally substituted alkyl, cycloalkyl, heterocycle, or aryl;
m, n, and x are integers from 1 to 10; and
R¹ is selected from the group comprising Formulas (IIIA), (IIIB), (IV), (V), (VI), (VII) and (VIII): 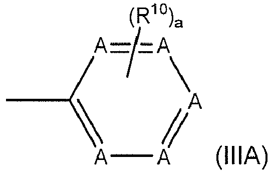 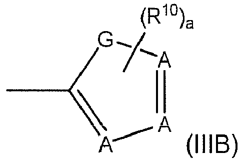 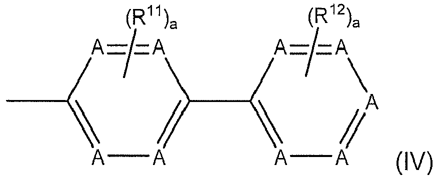 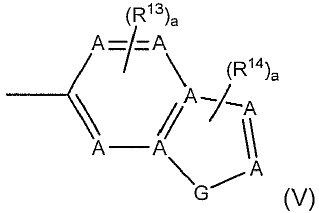 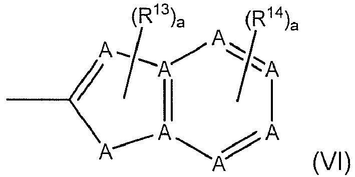 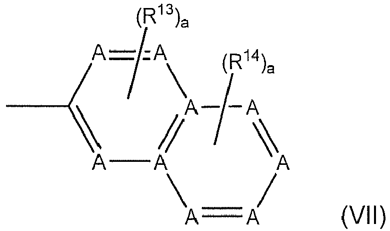 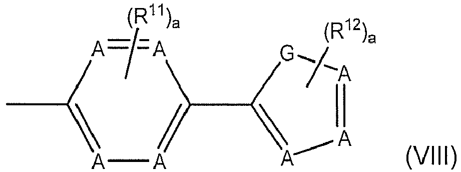 wherein:
   A is independently selected from the group consisting of CR⁶ and N;
   G is selected from the group consisting of S, O, CR⁸, and NR⁹;
   R⁶, R⁸ and R⁹ are independently selected from the group consisting of hydrogen, hydrocarbyl, and substituted hydrocarbyl;
   R¹⁰, R¹¹, R¹², R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, alkenyl, alkynyl, hydrocarbyl, and substituted hydrocarbyl;
   R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
   a is either the integer 1 or the integer 2.

Yet another aspect of the present invention encompasses a method of treating a urinary tract infection. The method comprises administering a compound of the invention to a subject in need thereof.

Still another aspect of the invention encompasses a method of reducing the resistance of a bacterium to a bactericidal compound. The method comprises administering a compound of the invention to a subject in need thereof.

Other aspects and iterations of the invention are described below.

### REFERENCE TO COLOR FIGURES

The application file contains at least one photograph executed in color. Copies of this patent application publication with color photographs will be provided by the Office upon request and payment of the necessary fee.

### BRIEF DESCRIPTION OF THE FIGURES

**Fig. 1** depicts images and diagrams detailing uropathogenic E. coli pathogenesis.
**Fig. 2** depicts chaperone/usher mediated pilus biogenesis as shown for the pilus system.

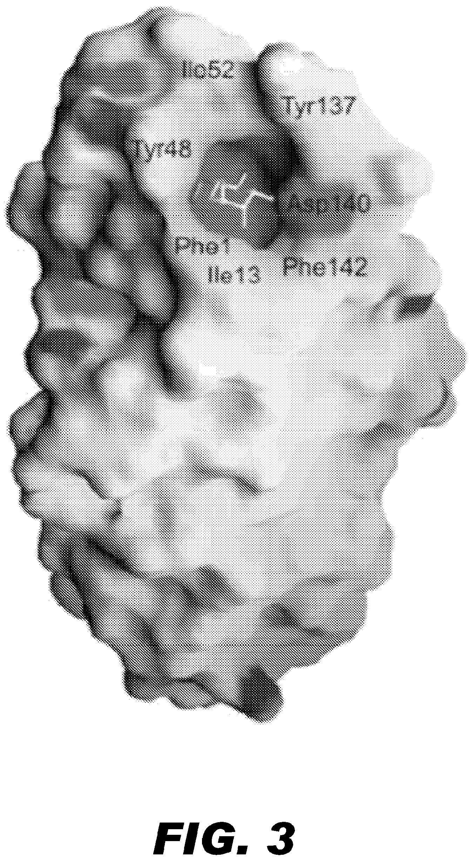

**Fig. 3** depicts a GRASP view of the FimH adhesion domain bound to a D-mannose residue (shown in stick). Red = acidic residues. Blue = basic residues.
**Fig. 4** depicts a ribbon representation of the receptor binding pocket in complex with the mannose. The D-mannose (pink), the mannose interacting residues (green) and the residues of the hydrophobic ridge around the pocket (white) are shown as ball-and-stick model.
**Fig. 5** depicts a graph showing the reduction of bacterial load in the bladder upon incubation with heptyl mannose at 6 hours post-infection. HM=heptyl mannose, MM=methyl mannose, p value obtained by Mann-Whitney test.
**Fig. 6** depicts FimH residues under positive selection. Green represents the adhesin FimH and gray represents the chaperone FimC. Residues in red are under positive selection. Residues in blue are not under positive selection. All residues are located outside the mannose binding pocket.
**Fig. 7** depicts the alignment of FimH sequence from a panel of clinical isolates previously examined. 18 clinical isolates examined for IBC formation were used to assess the different FimH sequences. The bold columns represent the residues identified as under positive selection. The residues highlighted in aqua represent residues that differ from UT189.
**Fig. 8** depicts bacterial load and IBC formation of isolate acute4 with its native FimH and UT189's FimH. The FimH sequence from acute4 was replaced with the fimH sequence of UT189 on the chromosome. **(A)** Depicts results obtained using bacterial titers, and **(B)** depicts results obtained using IBC formation.
**Fig. 9** depicts the structure of FimH with the compound number 15 in Table 1, illustrating interactions formed by compound number 15 with FimHA that are not shared with monomannose. Compound number 15 is shown in the final refined electron density calculated to 2.55Å with 2Fo-Fc coefficients and contoured at 1σ.
**Fig. 10** depicts a graph showing that mannosides number 7 and number 15 (Table 1) reduced intracellular bacteria at 1 hour post-infection. A 1 hour gentamicin protection assay was used to evaluate the amount of bacteria present in the luminal versus intracellular fraction in the presence of mannosides number 7 and number 15. A small decrease was seen in the luminal fraction in the presence of mannoside number 7 (p<0.05) whereas a significant decrease was seen in the intracellular fraction in the presence of either mannoside (p<0.0001).
**Fig. 11** depicts graphs showing that mannosides number 7 and number 15 reduce infection capacity *in vivo.* **(A)** 6 hour LacZ reveals significantly reduced IBCs at 1mM and 0.1mM mannoside for both mannosides number 7 and number 15 (p<0.0001). **(B)** 6 hour bacterial load quantification reveals significantly reduced colonization with 1mM mannoside number 7 and 1mM mannoside number 15 treated bacteria, p<0.001 and p<0.01, respectively.

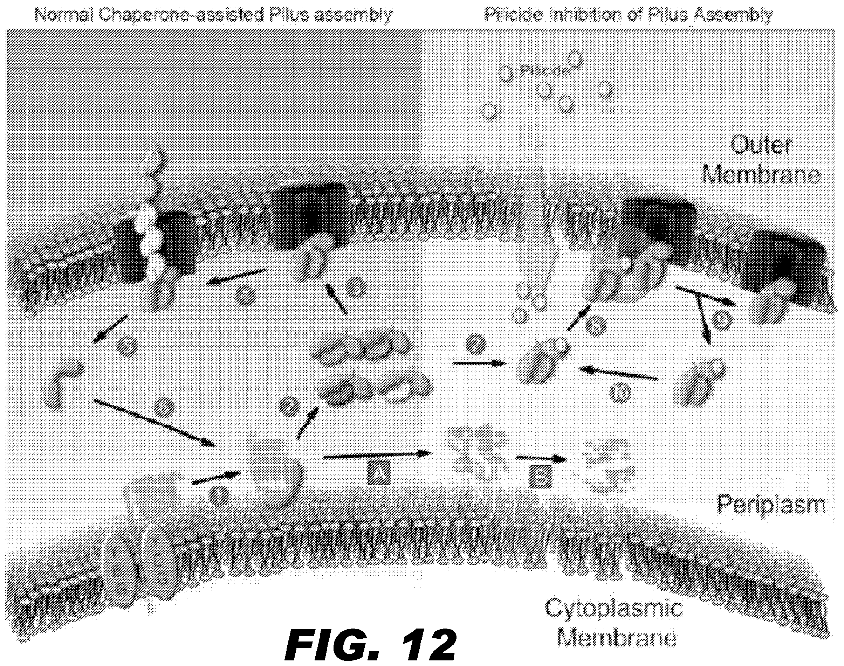

**Fig. 12** depicts a diagram showing pilicide inhibit bacterial pilus biogenesis. The schematic diagram shows the model of chaperone-assisted pilus assembly and the mode of pilicide action. During normal pilus biogenesis, newly synthesized pilus subunits cross into the periplasm via the Sec secretion pathway. These pilus subunits bind to their periplasmic chaperone proteins (orange molecules) and fold into correct confirmations (1). In the absence of the chaperones **[A],** pilus subunits are degraded **[B].** Chaperone-subunit complexes (2) are transported to the outer membrane usher proteins (blue cylinders) for assembly (3). Pilus assembly follows an ordered process with the chaperone-adhesin complexes as the initiatiors (4). As subunits are assembled into the growing pilus, accompanying chaperones disassociate from the membrane complexes (5), which could potentially be recycled to bind newly synthesized subunits. Pilicides (yellow circles) can cross freely through bacterial outer membranes and bind to periplasmic chaperones (7). Pilicides block the interaction of chaperone-subunit complexes with the outer membrane usher (8) and prevent pilus assembly (9 & 10).

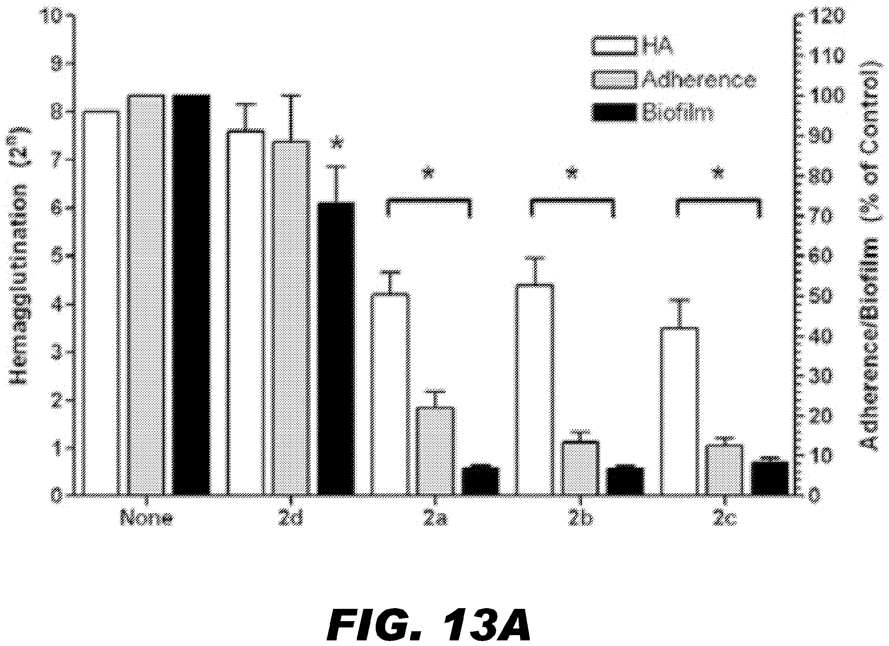

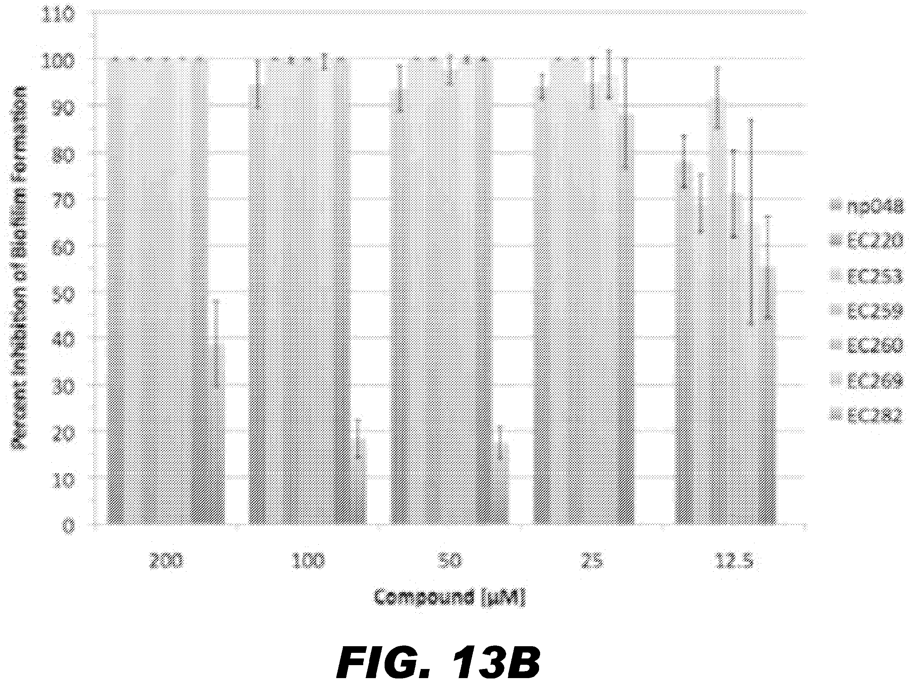

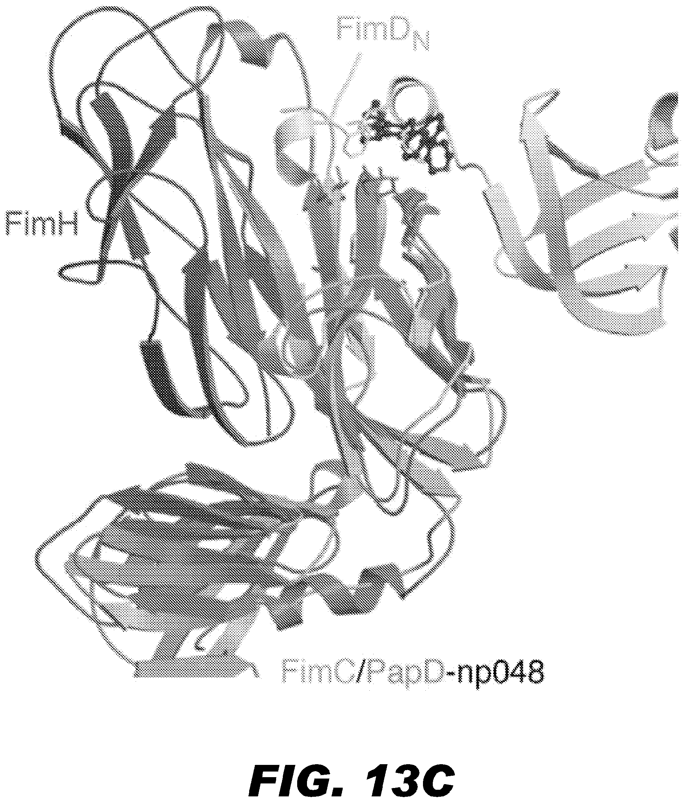

**Fig. 13** Effect of pilicides on pilus function and pilicide interaction with the PapD chaperone. **(A)** *E. coli* NU14 was grown in the presence of pilicides 2d, 2a, 2b and 2c (np048) (3.5 mM each). HA titers, adherence to 5637 bladder cells, and the ability to form biofilms was determined. **(B)** New pilicdes EC220-EC282 exhibit significant improvement in biofilm inhibitory activity compared to the original pilicide, np048. **(C)** PapD-np048 complex in overlay with the FimD1-125 N-terminal usher domain in complex with the FimC-FimH158-279 chaperone/adhesin complex. PapD and np048 are shown in light blue ribbon and ball-and-stick representation, respectively. FimC, FimH158-279 and FimD1-125 N-terminal domain are shown in magenta, green and yellow, respecitvely.
**Fig. 14** depicts a graph showing that UTI89 bacteria, when inoculated in the presence of FN075, makes significantly less IBCs in the bladder **(A)** and has a significantly reduced bacterial load within the bladder at 6 hours post-infection **(B).** p<0.0001.
**Fig. 15** depicts a representation of an efficacy study in mice using mannoside alone, antibiotic treatment alone, or dual mannoside/antibiotic treatment.
**Fig. 16** depicts a graph showing that dual treatment of mice inoculated with bacteria showed significantly lower levels of bacteria in the bladder than antibiotics alone.

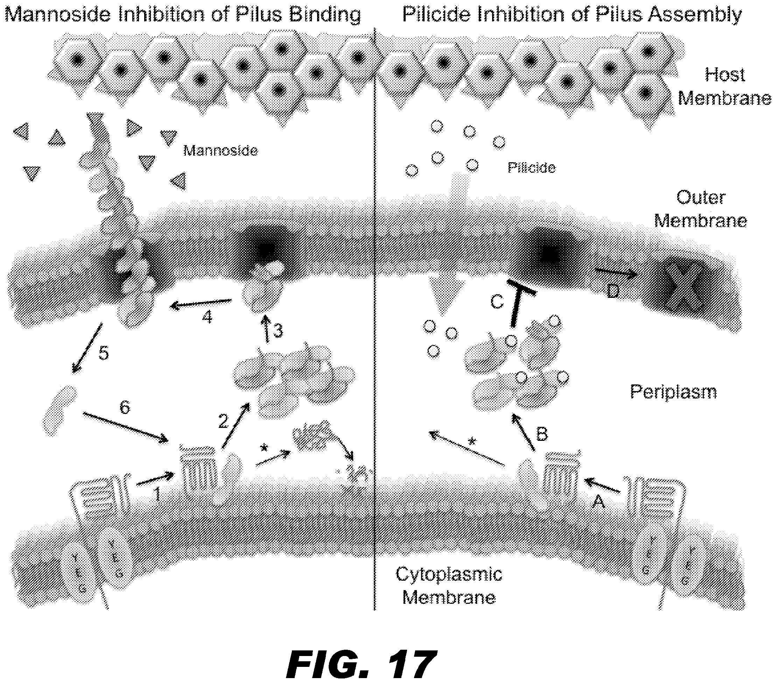

**Fig. 17** depicts a diagram illustrating two strategies to inhibit bacterial binding to host cells. The schematic diagram shows the model of chaperone-assisted pilus assembly and the mode of mannoside (left panel) and pilicide (right panel) action. During normal pilus biogenesis, newly synthesized pilus subunits cross into the periplasm via the Sec secretion pathway. These pilus subunits bind to their periplasmic chaperone proteins (orange molecules) and fold into correct confirmations (1). In the absence of the chaperones, pilus subunits are degraded (*). Chaperone-subunit complexes (2) are transported to the outer membrane usher proteins (blue cylinders) for assembly (3). Pilus assembly follows an ordered process with the chaperone-adhesin complexes as the initiators (4). As subunits are assembled into the growing pilus, accompanying chaperones disassociate from the membrane complexes (5), which could potentially be recycled to bind newly synthesized subunits. Mannosides (teal triangles) interact with the pilus adhesin and prevent binding to mannosylated residues (light brown triangles) on the surface of epithelial cells. Pilicides (yellow circles) can cross freely through bacterial outer membranes and bind to periplasmic chaperones (B). Pilicides block the interaction of chaperone-subunit complexes with the outer membrane usher (C) and prevent pilus assembly (D).

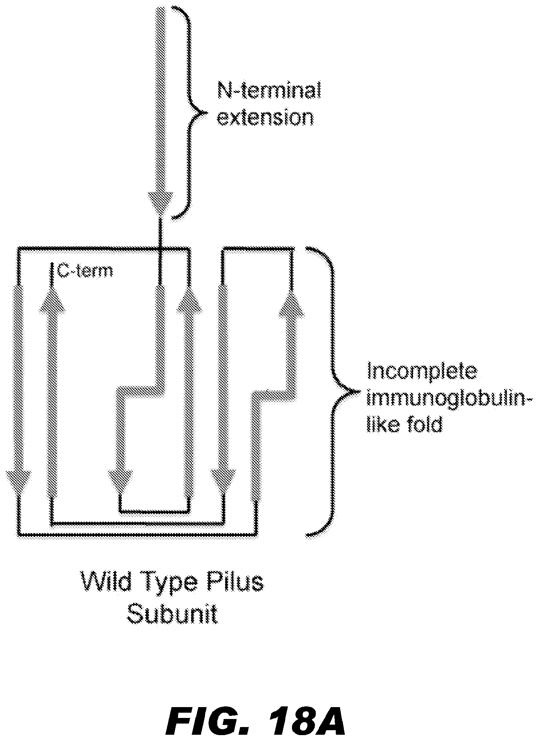

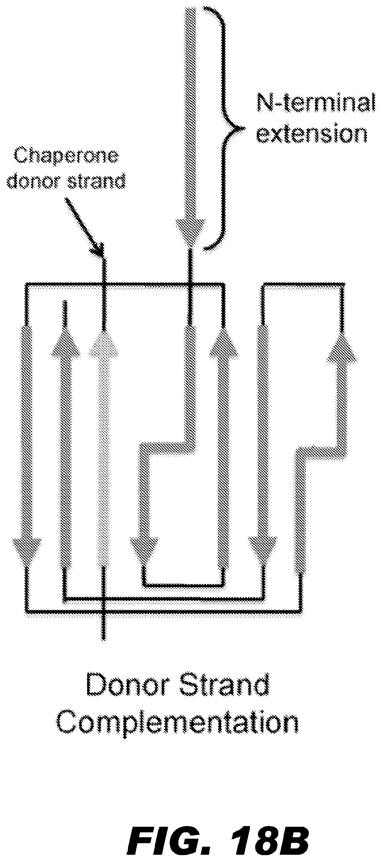

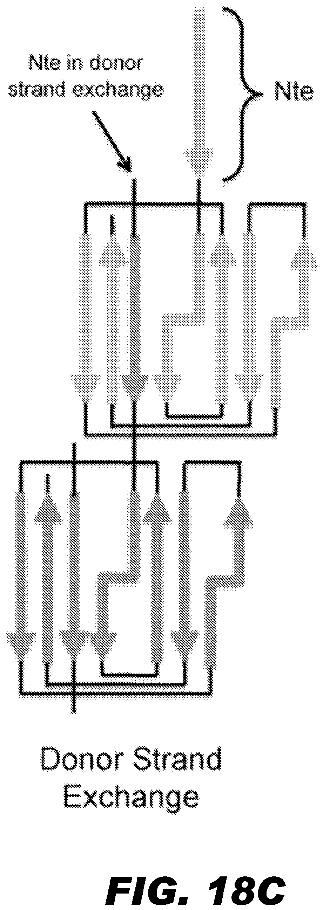

**Fig. 18** depicts diagrams illustrating chaperone-subunit donor strand exchange (DSE) and donor strand complementation (DSC). (A) Each subunit consists of six of the seven strands of a standard immunoglobulin (Ig)-fold and an N-terminal extension (Nte). (B) In order to form a stable structure prior to incorporation into the growing fiber, the chaperone donates the missing seventh strand of the subunit in a process called donor strand complementation (DSC). (C) During pilus assembly, the free Nte of one subunit displaces the chaperone bound to another subunit and serves as the seventh strand of the Ig-like fold in a process called donor strand exchange (DSE).

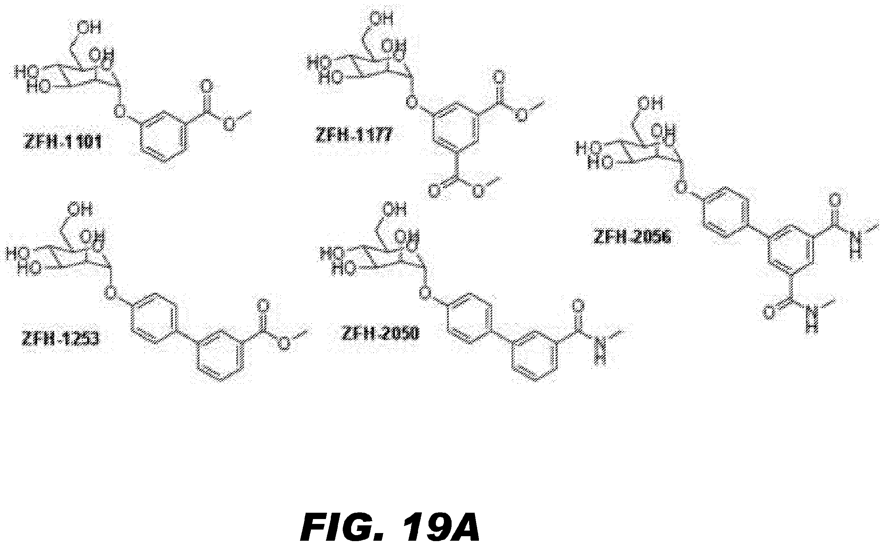

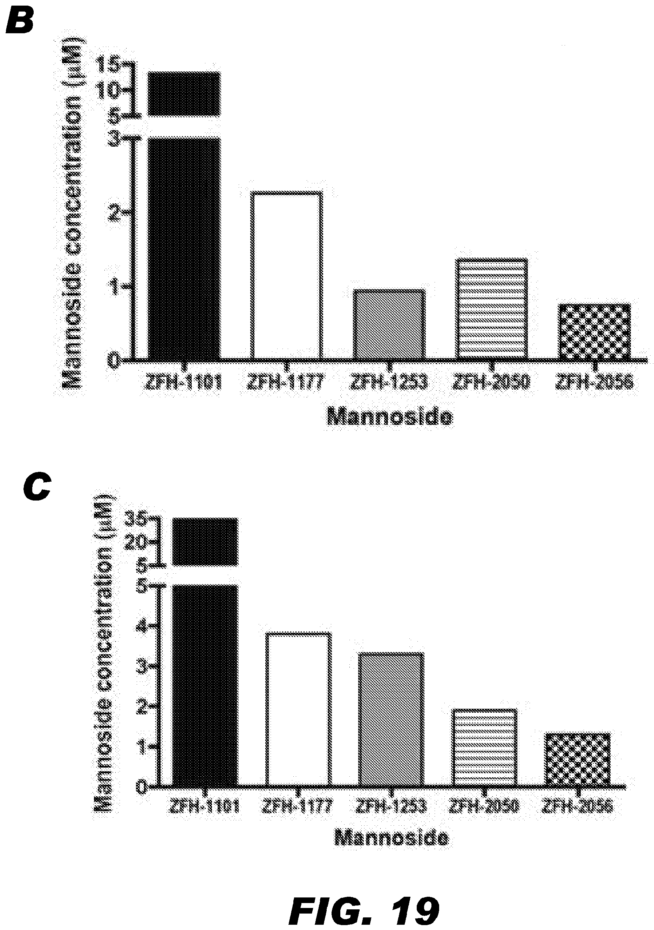

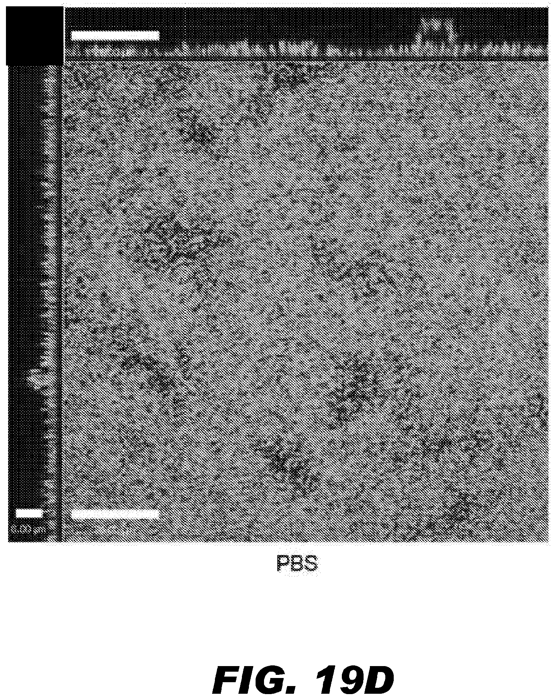

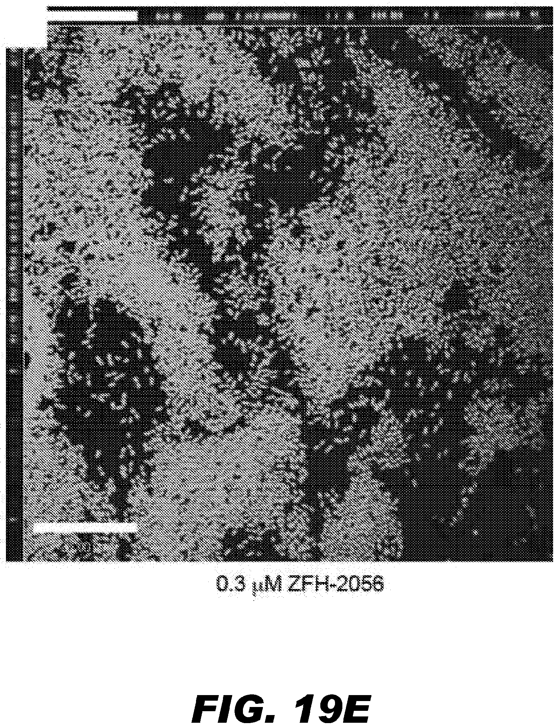

**Fig. 19** depicts the inhibition and disruption of UTI89 biofilm by mannoside and phase switching of UTI89 in the presence of mannoside. (a) Structure of mannosides. (b) The median inhibitory concentration (IC₅₀) of mannoside on UTI89 biofilm formation. (c) The IC₅₀ of mannoside on UTI89 biofilm disruption. After treatment with mannoside, the amount of UTI89 biofilm was measured. Bars show the mean value of the experiments (n = 3). (d and e) ZFH-2056 (Compound **50**) dispersed biofilm as measured by confocal microscopy of UTI89 biofilms grown for 24 h, then incubated for an additional 16 h in the absence (d) or presence (e) of 0.3 µM ZFH-2056.

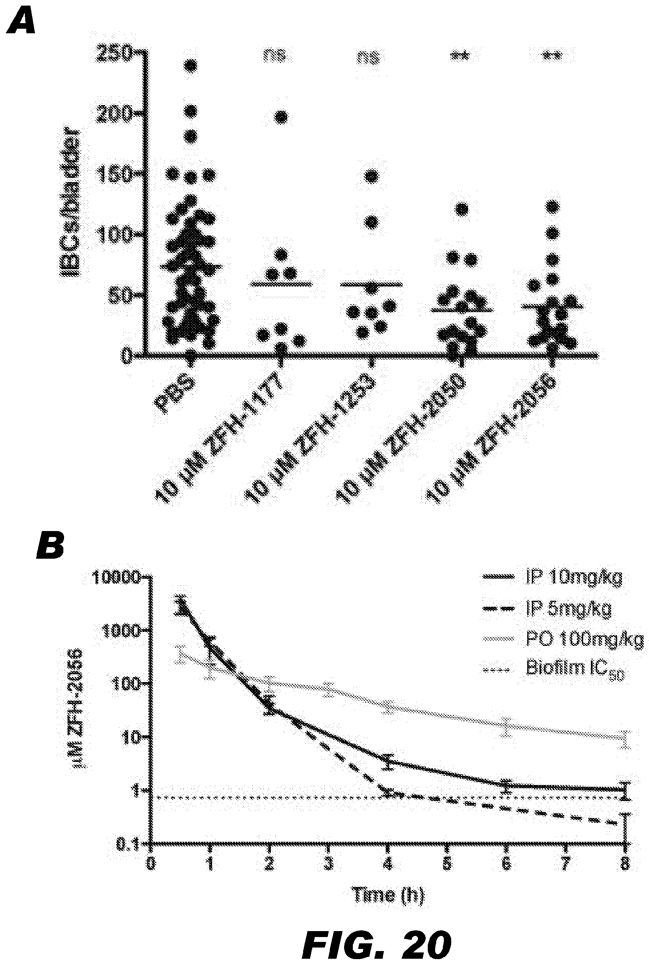

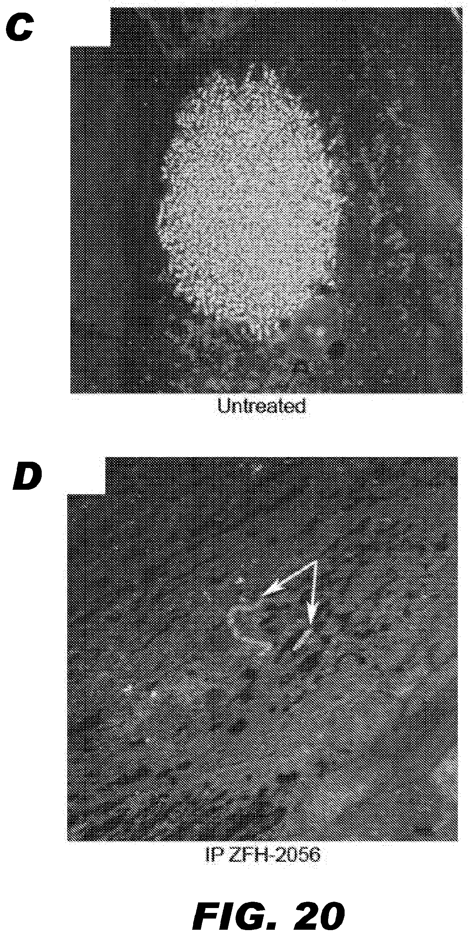

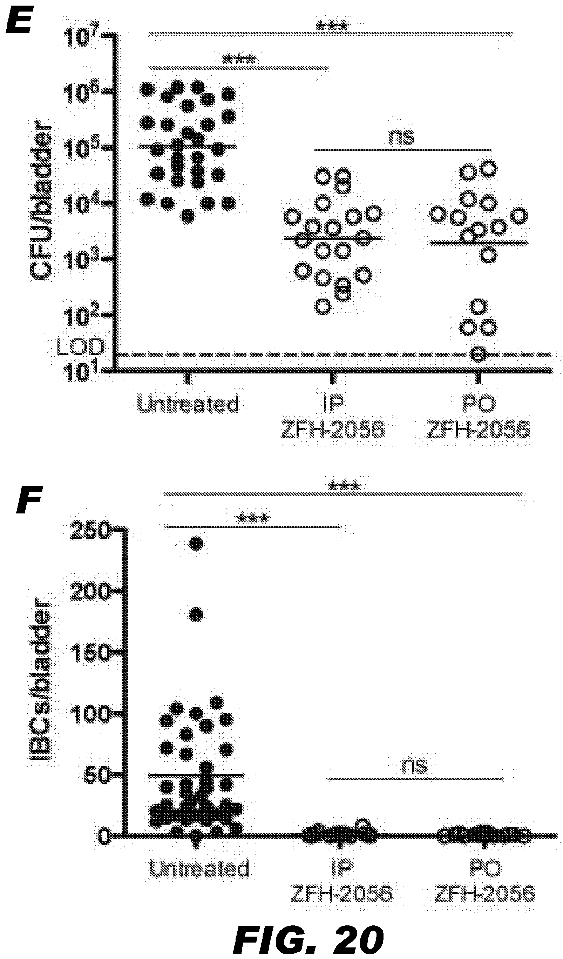

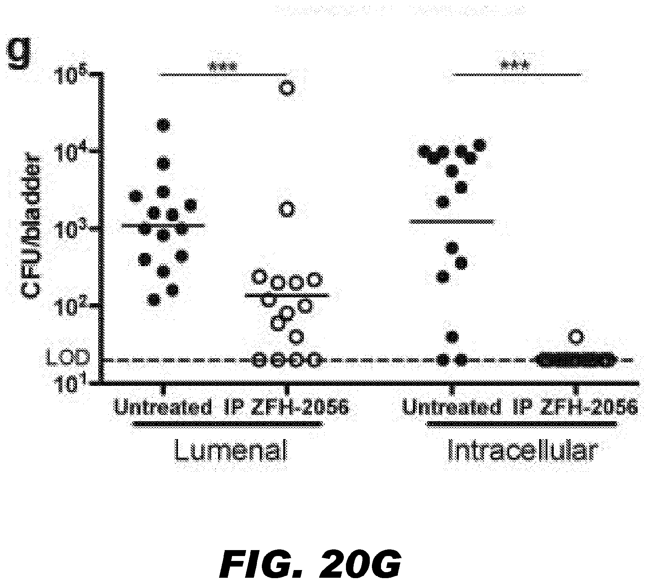

**Fig 20** depicts the mannoside effect on UTI89 colonization. (a) Pretreatment of UTI89 with mannosides ZFH-2050 (Compound **49**) and ZFH-2056 (Compound **50**) resulted in reduced IBC formation at 6 hpi. (b) Urine PK analysis of ZFH-2056 (Compound **50**) (n ≥ 3 mice) showing amounts in urine over time for each dosing regimen indicated. Horizontal dashed line is at IC₅₀ (0.74 µM) as determined by the biofilm inhibition assay. (c and d) Confocal microscopy of bladders from untreated (c) and ZFH-2056-treated (d) mice. Bacteria were stained with SYTO9 (green) and the bladder luminal surface was stained with WGA (red). The image in c shows a normal, robust IBC whereas the arrows in d indicate luminal bacteria. (e) Total bacterial CFUs at 6 hpi from untreated mice or mice treated with ZFH-2056 either by IP (5 mg/kg) or oral (100 mg/kg) dosing 30 min prior to inoculation of UTI89. (f) IBC quantification at 6 hpi from untreated mice or mice treated with ZFH-2056 either by IP (5 mg/kg) or oral (100 mg/kg) dosing 30 min prior to inoculation of UTI89. (g) 6 hpi ex vivo gentamicin protection assay revealed both luminal and intracellular bacteria are significantly reduced upon IP (5 mg/kg) pretreatment of mice with ZFH-2056. Bars indicate geometric mean. Statistical significance according to Mann-Whitney is at **P < 0.01, ***P < 0.0001. ns, not significant; LOD, limit of detection.

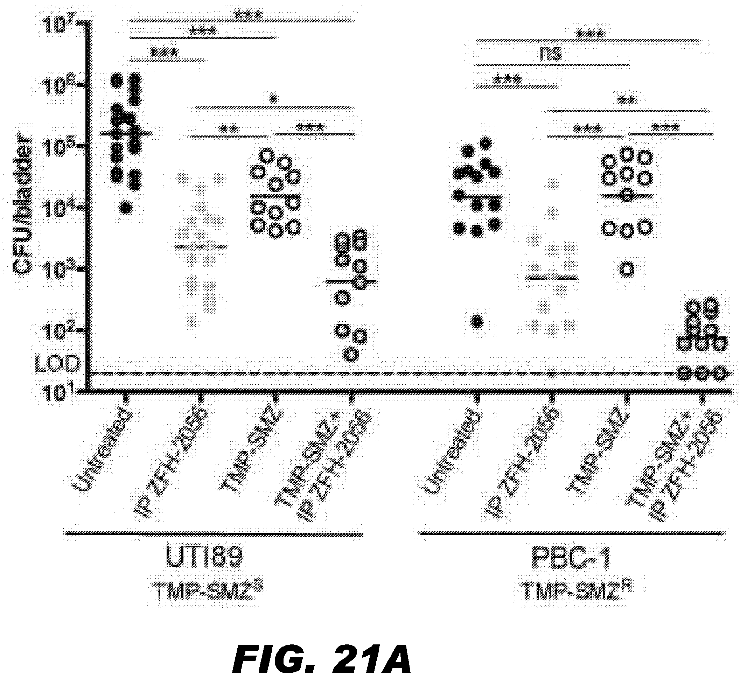

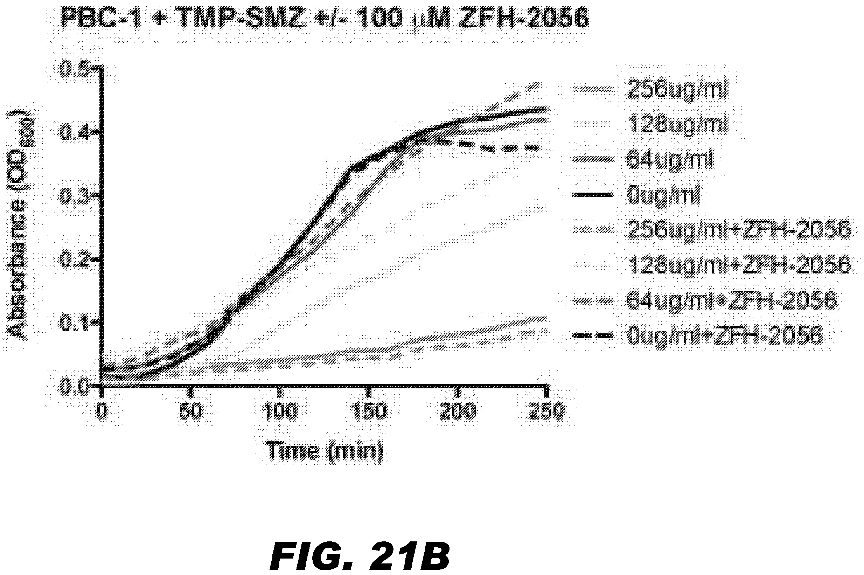

**Fig. 21** depicts mannoside synergy with TMP-SMZ at reducing UTI89 colonization. (a) Total bacterial CFUs were quantitated 6 hpi. UTI89 colonization was reduced in mice treated with ZFH-2056 (Compound **50**), TMP-SMZ and TMP-SMZ+ZFH-2056. There was further decreased colonization in TMP-SMZ+ZFH-2056-treated mice over ZFH-2056 or TMP-SMZ alone. PBC-1 colonization was reduced in mice treated with ZFH-2056 and TMP-SMZ+ZFH-2056, but not TMP-SMZ alone. Enhanced efficacy as measured by bacterial CFUs was observed upon treatment with TMP-SMZ+ZFH-2056 over ZFH-2056 or TMP-SMZ treatment alone. Bars indicate geometric mean. Statistical significance according to Mann-Whitney is at *P < 0.05, **P < 0.01, ***P < 0.0001. ns, not significant; LOD, limit of detection. (b) Growth curve of PBC-1 with TMP-SMZ in the absence (solid line) or presence (dashed line) of 100 □M ZFH-2056. SMZ concentration is 5x TMP concentration listed.
**Fig. 22** depicts X-ray structure of biphenyl mannoside meta-methyl ester (Compound **29**) with electron density (mesh) calculated with 2Fo-Fc coefficients, contoured at 1 sigma. Interaction with the Arg-98-Glu-50 salt bridge (dashes), pi-pi stacking with Tyr-48 and hydrophobic interaction with Tyr-137 are shown. Surface electrostatic potential of FimH, calculated with APBS, is displayed such that pure blue and red would be +4kT/e and -4kT/e respectively.
**Fig. 23** depicts the correlation of HA Titer and Binding Data.
**Fig. 24** depicts mannoside esters and their amidated counterparts with enhanced solubility.
**Fig. 25** depicts results of hemagglutination (A) and LacZ quantification of IBCs (B).
**Fig 26** depicts the pharmacokinetic results of ZFH56 (Compound **50**) injected IP and PO into mice.
**Fig. 27** depicts IBC numbers and lacZ staining in mice treated with ZFH56 (Compound **50**).
**Fig. 28** depicts fluorescent microscopy imaging of infected bladders treated with ZFH56 (Compound **50**).
**Fig. 29** depicts efficacy studies of mannosides against TMP-SMZ resistant strains.
**Fig. 30** depicts efficacy of ZFH56 (Compound **50**) under various doses of inoculum.
**Fig. 31** depicts efficacy studies of ZFH56 (Compound **50**) with compound dose boosting.
**Fig. 32A** depicts time course of infection and treatment with mannosides.
**Fig. 32B** depicts inhibition of preformed biofilm by ZFH56 (Compound **50**).

### DETAILED DESCRIPTION

Compounds that inhibit the function of type I pili of bacteria have been developed. The compounds may be useful for the treatment of urinary tract infections. Significantly, the compounds may prevent bacterial colonization and invasion of the bladder tissue to prevent infection and the establishment of reservoirs that can serve as a source of recurrent infections. The invention also encompasses methods of use of a compound of the invention.

### I. Compounds

One aspect of the invention is a compound of Formula (I): 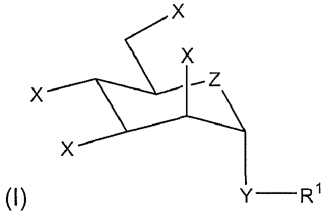 wherein:
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R² is independently selected from the group consisting of hydrogen, COR^{x}, CONR^{x}, hydrocarbyl, and substituted hydrocarbyl;
R³, R⁴, R⁵ are independently selected from the group consisting of hydrogen, hydrocarbyl, and substituted hydrocarbyl;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, NR⁴R⁵, and an optionally substituted alkyl, cycloalkyl, heterocycle, or aryl;
n is an integer from 1 to 10; and
R¹ is selected from the group comprising Formulas (IIIA), (IIIB), (IV), (V), (VI), (VII) and (VIII): 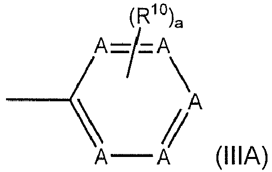 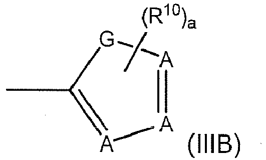 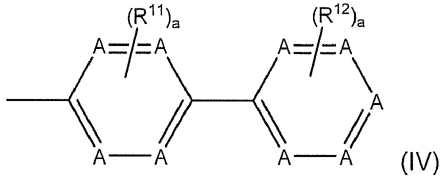 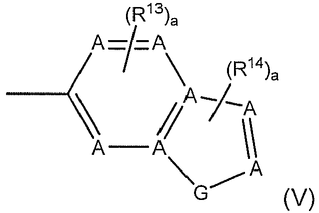 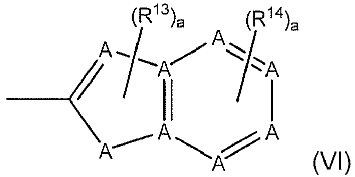 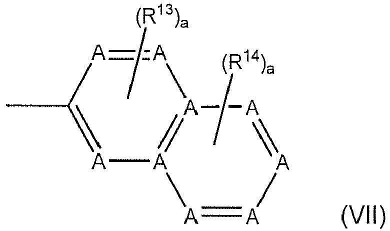 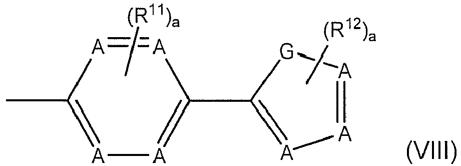 wherein:
   A is independently selected from the group consisting of CR⁶ and N;
   G is selected from the group consisting of S, O, CR⁸, and NR⁹;
   R⁶, R⁸ and R⁹ are independently selected from the group consisting of hydrogen, hydrocarbyl, and substituted hydrocarbyl;
   R¹⁰, R¹¹, R¹², R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, alkenyl, alkynyl, hydrocarbyl, and substituted hydrocarbyl;
   R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
   a is either the integer 1 or the integer 2.

One embodiment of formula (I), where R¹ is formula (IIIA), comprises formula (IX): 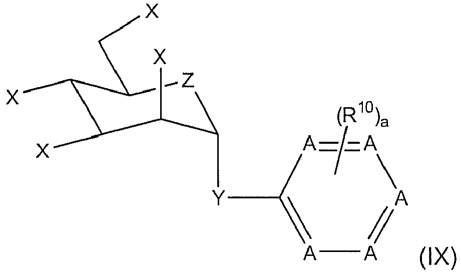 wherein
X is selected from the group consisting of hydrogen, OR², SR², NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
R⁶, R⁸ and R⁹ are independently selected from the group consisting of hydrogen, hydrocarbyl, and substituted hydrocarbyl;
R¹⁰ is independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

In an exemplary embodiment of a compound of formula (IX), R¹⁰ is aryl or heterocyle.

Another embodiment of formula (I), where R¹ is formula (IIIB), comprises formula (X): 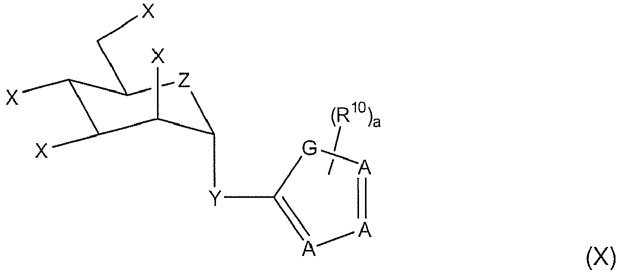 wherein
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R² is independently selected from the group consisting of hydrogen, -COR^{x}, and -CONR^{x};
R³, R⁴, R⁵ are independently selected from the group consisting of hydrogen, hydrocarbyl, and substituted hydrocarbyl;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl;
n is an integer from 1 to 10;
A is independently selected from the group consisting of CR⁶ and N;
G is selected from the group consisting of S, O, CR⁸, and NR⁹;
R⁶, R⁸ and R⁹ are independently selected from the group consisting of hydrogen, hydrocarbyl, and substituted hydrocarbyl;
R¹⁰ is independently selected from the group consisting of hydrogen, OR¹⁵, NR¹⁵R¹⁶, NR¹⁵COR¹⁶, NR¹⁵CO₂R¹⁶, NR¹⁵CONR¹⁶, NR¹⁵SO₂R¹⁶, COR¹⁵, SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl; and
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
a is either the integer 1 or the integer 2.

Yet another embodiment of formula (I), where R¹ is formula (IV), comprises formula (XI): 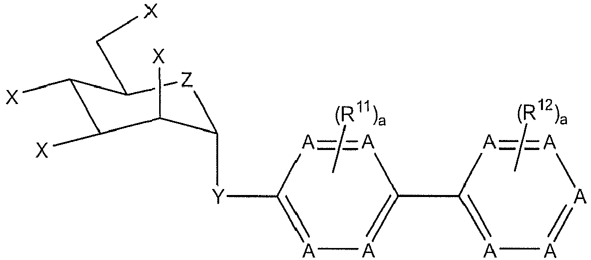 wherein
X is selected from the group consisting of hydrogen, OR², SR², NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, -N(CH₂)ₙ-, -(CH₂)ₙ-, NR⁵, and an optionally substituted alkyl, alkene, alkyne, or heterocycle;
R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
R⁶, R⁸ and R⁹ are independently selected from the group consisting of hydrogen, hydrocarbyl, and substituted hydrocarbyl;
R¹¹ and R¹² are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶ -COR¹⁵, -SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

Still another embodiment of formula (I), where R¹ is formula (V), comprises formula (XII): 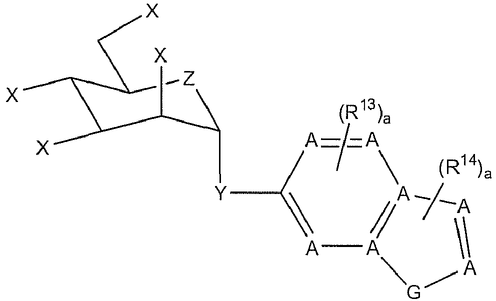 wherein
X is selected from the group consisting of hydrogen, OR², SR², NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
G is selected from the group consisting of S, O, CR⁸, and NR⁹;
R⁶, R⁸ and R⁹ are independently selected from the group consisting of hydrogen, hydrocarbyl, and substituted hydrocarbyl;
R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

Yet still another embodiment of formula (I), where R¹ is formula (VI), comprises formula (XIII): 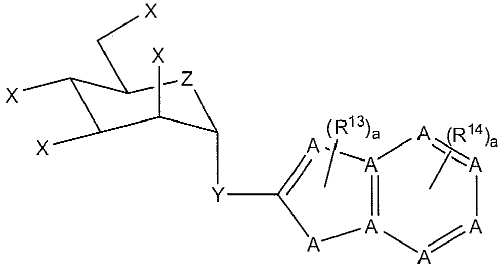 wherein
X is selected from the group consisting of hydrogen, OR², SR², NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
R⁶ is independently selected from the group consisting of hydrogen, hydrocarbyl, and substituted hydrocarbyl;
R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

Still another embodiment of formula (I), where R¹ is formula (VII), comprises formula (XIV): 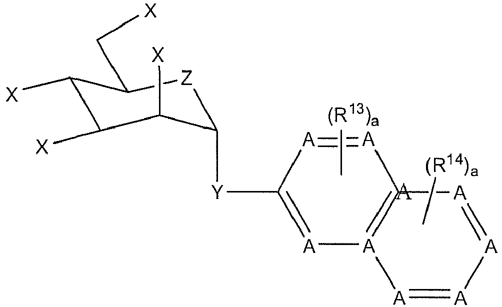 wherein
X is selected from the group consisting of hydrogen, OR², SR², NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
R⁶ is independently selected from the group consisting of hydrogen, hydrocarbyl, and substituted hydrocarbyl;
R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

A further embodiment of formula (I), where R¹ is formula (VIII), comprises formula (XV): 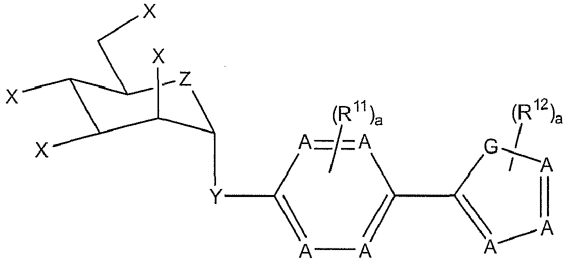 wherein:
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
R², R³, R⁴, R⁵ are independently selected from the group consisting of hydrogen, hydrocarbyl, and substituted hydrocarbyl;
n is an integer from 1 to 10;
A is independently selected from the group consisting of CR⁶ and N;
G is selected from the group consisting of S, O, CR⁸, and NR⁹;
R⁶, R⁸ and R⁹ are independently selected from the group consisting of hydrogen, hydrocarbyl, and substituted hydrocarbyl;
R¹¹ and R¹² are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, COOR¹⁵, CONR¹⁶R¹⁷, SO₂NR¹⁸R¹⁹, alkenyl, alkynyl, hydrocarbyl, and substituted hydrocarbyl;
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring;
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, -NR⁴R⁵, and an optionally substituted alkyl, cycloalkyl, heterocycle, or aryl; and
a is either the integer 1 or the integer 2.

A still further another embodiment of formula (I), where R¹ is formula (IV), comprises formula (XVI): 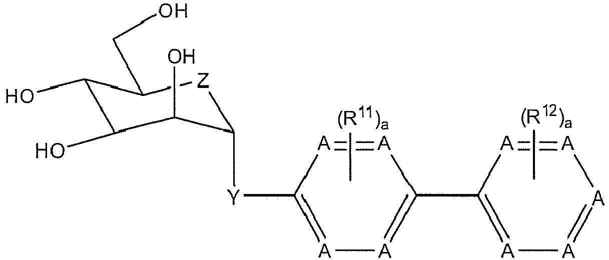 wherein:
Z is selected from the group consisting of O, S, CR³ and NR⁴;
Y is selected from the group consisting of O, S, CR³, and NR⁴;
A is independently selected from the group consisting of CR⁶ and N;
R³, R⁴, and R⁶ are independently selected from the group consisting of hydrogen hydrocarbyl, and substituted hydrocarbyl;
R¹¹ is independently selected from the group consisting of hydrogen, -NR⁵, -OR⁵, nitro, cyano, chloro, bromo, iodo, fluoro, -COOR¹⁵, -CONR¹⁶R¹⁷, -SO₂R¹⁵, -SO₂NR¹⁸R¹⁹, methyl, hydrocarbyl, and substituted hydrocarbyl;
R¹² is independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, SO₂NR¹⁸R¹⁹, hydrocarbyl, and substituted hydrocarbyl; and
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
a is either the integer 1 or the integer 2.

A still further another embodiment of formula (I), where R¹ is formula (IV), comprises formula (XVII): 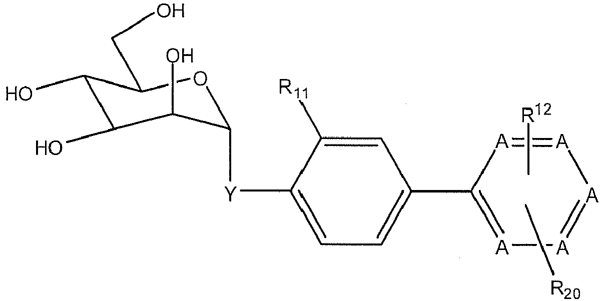 wherein:
Y is selected from the group consisting of O, S, CR³, and NR⁴;
A is independently selected from the group consisting of CR⁶ and N;
R³, R⁴, and R⁶ are independently selected from the group consisting of hydrogen, hydrocarbyl, and substituted hydrocarbyl;
R¹¹ is selected from the group consisting of hydrogen, -NR⁵, -OR⁵, nitro, cyano, chloro, bromo, iodo, fluoro, -COOR¹⁵, -CONR¹⁶R¹⁷, -SO₂R¹⁵, -SO₂NR¹⁸R¹⁹, methyl, hydrocarbyl, and substituted hydrocarbyl;
R¹² is selected from the group consisting of hydrogen, -OR¹⁵, - NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, hydrocarbyl, and substituted hydrocarbyl;
R²⁰ is selected from the group consisting of -NR¹⁵SO₂R¹⁶, -COOR¹⁵, -NR¹⁵CONR¹⁶, -CONR¹⁶R¹⁷, -SO₂NR¹⁸R¹⁹; and
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring.

In a preferred embodiment of formula (XVII), R¹¹ is selected from the group consisting of chloro, bromo, iodo, and fluoro, R¹² is selected from the group consisting of nitro, cyano, -COOR¹⁵, -CONR¹⁶R¹⁷, and -SO₂NR¹⁸R¹⁹, and R²⁰ is selected from the group consisting of -COOR¹⁵, -NR¹⁵CONR¹⁶, -CONR¹⁶R¹⁷, and -SO₂NR¹⁸R¹⁹.

In another preferred embodiment of formula (XVII), R¹¹ is chloro, R¹² is selected from the group consisting of nitro, cyano, -COOR¹⁵, -CONR¹⁶R¹⁷, and - SO₂NR¹⁸R¹⁹, and R²⁰ is selected from the group consisting of -COOR¹⁵, -NR¹⁵CONR¹⁶, - CONR¹⁶R¹⁷, and -SO₂NR¹⁸R¹⁹.

A yet further embodiment of formula (I), where R¹ is formula (VIII), comprises formula (XVIII): 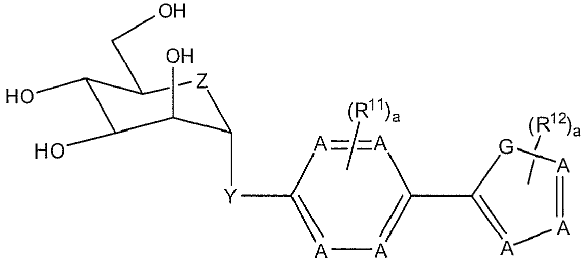 wherein
Z is selected from the group consisting of O, S, CR³ and NR⁴;
Y is selected from the group consisting of O, S, CR³, and NR⁴;
A is independently selected from the group consisting of CR⁶ and N;
G is selected from the group consisting of S, O, CR⁸, and NR⁹;
R³, R⁴, R⁶, R⁸, and R⁹ are independently selected from the group consisting of hydrogen, hydrocarbyl, and substituted hydrocarbyl;
R¹¹ is independently selected from the group consisting of hydrogen, -NR⁵, -OR⁵, nitro, cyano, chloro, bromo, iodo, fluoro, -COOR¹⁵, -CONR¹⁶R¹⁷, -SO₂R¹⁵, -SO₂NR¹⁸R¹⁹, methyl, hydrocarbyl, and substituted hydrocarbyl;
R¹² is independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, SO₂NR¹⁸R¹⁹, hydrocarbyl, and substituted hydrocarbyl; and
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
a is either the integer 1 or the integer 2.

In some embodiments of formula (I), where R¹ is formula (VIII), comprises formula (XIX): 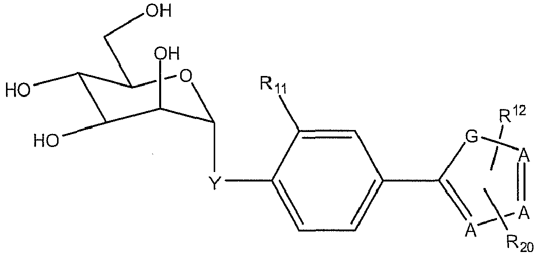 wherein
Y is selected from the group consisting of O, S, CR³, and NR⁴;
A is independently selected from the group consisting of CR⁶ and N;
G is selected from the group consisting of S, O, CR⁸, and NR⁹;
R³, R⁴, R⁶, R⁸, and R⁹ are independently selected from the group consisting of hydrogen, hydrocarbyl, and substituted hydrocarbyl;
R¹¹ is independently selected from the group consisting of hydrogen, -NR⁵, -OR⁵, nitro, cyano, chloro, bromo, iodo, fluoro, -COOR¹⁵, -CONR¹⁶R¹⁷, -SO₂R¹⁵, -SO₂NR¹⁸R¹⁹, methyl, hydrocarbyl, and substituted hydrocarbyl;
R¹² is independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, SO₂NR¹⁸R¹⁹, hydrocarbyl, and substituted hydrocarbyl; and
R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring.

In a preferred embodiment of formula (XIX), R¹¹ is selected from the group consisting of chloro, bromo, iodo, and fluoro, R¹² is selected from the group consisting of -COOR¹⁵, and -CONR¹⁶R¹⁷, and R²⁰ is selected from the group consisting of -COOR¹⁵, -NR¹⁵CONR¹⁶, -CONR¹⁶R¹⁷, and -SO₂NR¹⁸R¹⁹.

In another preferred embodiment of formula (XVII), R¹¹ is chloro, R¹² is selected from the group consisting of -COOR¹⁵, and -CONR¹⁶R¹⁷, and R²⁰ is selected from the group consisting of -COOR¹⁵, -NR¹⁵CONR¹⁶, -CONR¹⁶R¹⁷, and - SO₂NR¹⁸R¹⁹.

Another aspect of the present invention is a compound of formula (II): 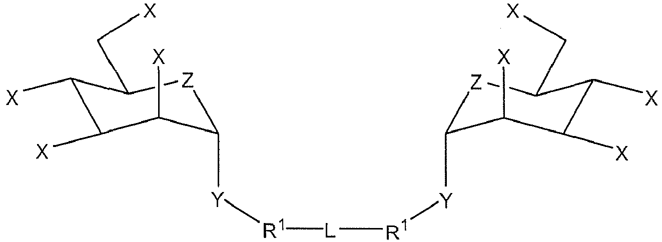
X is selected from the group consisting of hydrogen, OR², SR², and NR^{z};
Z is selected from the group consisting of O, S, CR³ and NR⁴;
Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, - CH₂S-, CO, -CON(R⁵)-, -SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
L is selected from -O(CH₂)ₙO-, -S(CH₂)ₙS-, -N(CH₂)ₙN-, -(CH₂)ₙ-, -O[(CH₂)ₘO(CH₂)ₙ]ₓO-, -N(CH₂)ₘO(CH₂)ₙN-, heterocycle, alkene, alkyne, -CON[(CH2)ₘO(CH2)ₙ]ₓNCO-, -SO₂N[(CH2)ₘO(CH2)ₙ]ₓNO₂S-, and -NCO[(CH₂)ₘO(CH₂)ₙ]ₓCON-, where L is bound to a ring of R¹ at a meta or para position;
R² is independently selected from the group consisting of hydrogen, COR^{x}, CONR^{x}, hydrocarbyl, and substituted hydrocarbyl;
R³, R⁴, R⁵ are independently selected from the group consisting of hydrogen, hydrocarbyl, and substituted hydrocarbyl,
R^{z} is independently selected from the group consisting of hydrogen hydrocarbyl, substituted hydrocarbyl, -COR^{x}, -CONR^{x}R^{x}SO₂R^{x}, and -CO₂R^{x};
R^{x} is independently selected from the group consisting of hydrogen, NR⁴R⁵, and an optionally substituted alkyl, cycloalkyl, heterocycle, or aryl;
m, n, and x are integers from 1 to 10; and
R¹ is selected from the group comprising Formulas (IIIA), (IIIB), (IV), (V), (VI), (VII) and (VIII): 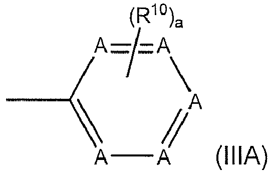 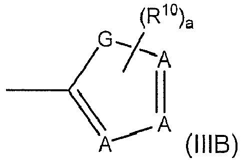 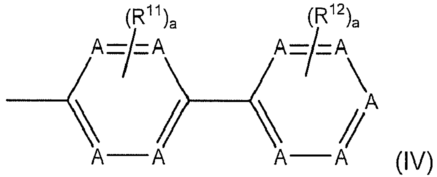 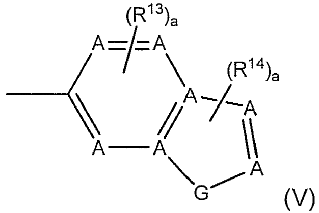 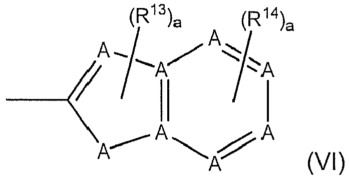 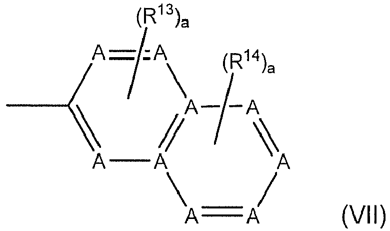 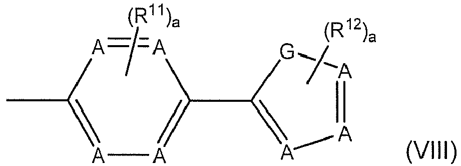 wherein:
   A is independently selected from the group consisting of CR⁶ and N;
   G is selected from the group consisting of S, O, CR⁸, and NR⁹;
   R⁶, R⁸ and R⁹ are independently selected from the group consisting of hydrogen, hydrocarbyl, and substituted hydrocarbyl;
   R¹⁰, R¹¹, R¹², R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, alkenyl, alkynyl, hydrocarbyl, and substituted hydrocarbyl;
   R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
   a is either the integer 1 or the integer 2.

In an exemplary alternative of each of the foregoing embodiments, a compound comprising formula (I) is a compound comprising any of the Formulas in **Table 15.**

In a further exemplary alternative of each of the foregoing embodiments, a compound comprising formula (I) is compound number 50, 75, 76, or 77.

In certain embodiments, the sugar residue of the above compounds may encompass any stereoisomer of mannose. In other embodiments, the sugar residue of the above compounds may encompass any stereoisomer of mannose other than glucose. In an exemplary embodiment, the sugar residue of the above compounds is alpha D mannose.

Exemplary methods of synthesizing a compound of the invention are detailed in the Examples.

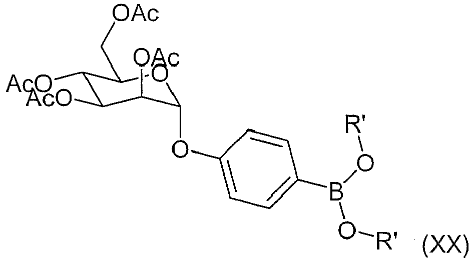

A compound of the invention may also be an intermediate in the synthesis of a compound of formula (I) - (XIX). For instance, in one embodiment, a compound of the invention may be an ester intermediate in the synthesis of a compound of formula (I) - (XIX). In another embodiment, a compound of the invention may be a boronate ester of a mannoside or a boronic acid ester of a mannoside. In yet another embodiment, a compound of the invention may have the formula (XX), wherein R' is selected from H, alkyl, or both R' groups may together form a ring (for instance, see the synthesis of compound 77 detailed in the Examples below). In still another embodiment, a compound of the invention may be a compound illustrated in Schemes 1, 2, 3, 1^{a}, 2^{a}, 3^{a}, 4^{a}, or 5^{a} in the Examples below.

A compound of the invention may also comprise an imaging agent, such as a fluorescent moiety. For example, see compounds 98 and 99 of Table 15. In an exemplary embodiment, the imaging agent is bound to the sugar portion of a compound of the invention, either directly, or via a linker.

Compounds of the invention may block the function of FimH of the type I pili of pathogenic bacteria and prevent bacterial adherence and invasion and thus prevent bacterial amplification in the IBC and subsequent spreading and repeated rounds of amplification via new generation IBCs.

FimH functional assays used to measure activity of the compounds are known to individuals skilled in the art. Non-limiting examples of functional assays include hemmagglutination titer using guinea pig red blood cells, affinity of binding to FimH, and the ability of the compounds to prevent biofilm formation.

In some embodiments, activity of the compound is measured using hemmagglutination titer of guinea pig red blood cells. Hemagglutination of guinea pig red blood cells by type1 piliated UPEC is dependent upon FimH mannose binding ability and serial dilutions allow a quantitative analysis. Hemagglutination titer may generally be defined as the amount of compound required for decreasing hemagglutination by 75%. In some embodiments, the hemmagglutiantion titer of the compound of the invention may be less than about 14, 13, 12, 11, 10, 9, 8, 7, 6, 5, 4, 3, 2, or 1µM. In a preferred alternative of the embodiments, the hemmagglutiantion titer of the compound of the invention may be less than about 9µM. In another preferred alternative of the embodiments, the hemmagglutiantion titer of the compound of the invention may be less than about 7µM. In yet another preferred alternative of the embodiments, the hemmagglutiantion titer of the compound of the invention may less than about 1µM.

In other embodiments, activity of the compound may be measured using affinity of binding of the compound of the invention to FimH. In some embodiments of the invention, the Kd value for a compound of the invention and FimH may be less than about 5µM. For instance, the Kd value may be about 0.2, 0.3, 0.5, 0.6, 0.7, or 3.5µM.

In yet other embodiments, activity of the compound may be measured using the ability of the compound to prevent biofilm formation. In general, titration curves measuring the ability of a compound inhibit biofilm formation may be performed to determine the IC₅₀. In some embodiments, the IC₅₀ of the compound may be less than about 700, 600, 500, 400, 300, 200 or 100µM. In other embodiments, the IC₅₀ of the compound may be less than about 500, 400, 300, 200, 100, 50, 40, 30, 20 10, 9, 8, 7, 6, or 5µM. In preferred embodiments, the IC₅₀ of the compound may be less than about 20µM.

### II. Combinations

Another aspect of the present invention encompasses a combination of a compound of the invention (described in Section I above) with one or more bactericidal compounds. In some embodiments, a compound of the invention may comprise a combination with 1, 2, 3, 4, or 5 bactericidal compounds. In one embodiment, the bactericidal compound is an antibiotic. Suitable antibiotics are known in the art, and may include Amikacin, Gentamicin, Kanamycin, Neomycin, Netilmicin, Tobramycin, Paromomycin, Geldanamycin, Herbimycin, Carbacephem, Loracarbef, Ertapenem, Doripenem, Imipenem/Cilastatin, Meropenem, Cefadroxil, Cefazolin, Cefalotin, Cefalexin, Cephalosporins, Cefaclor, Cefamandole, Cefoxitin, Cefprozil, Cefuroxime, Cefixime, Cefdinir, Cefditoren, Cefoperazone, Cefotaxime, Cefpodoxime, Ceftazidime, Ceftibuten, Ceftizoxime, Ceftriaxone, Cefepime, Ceftobiprole, Teicoplanin, Vancomycin, Telavancin, Clindamycin, Lincomycin, Azithromycin, Clarithromycin, Dirithromycin, Erythromycin, Roxithromycin, Troleandomycin, Telithromycin, Spectinomycin, Aztreonam, Furazolidone, Nitrofurantoin, Amoxicillin, Ampicillin, Azlocillin, Carbenicillin, Cloxacillin, Dicloxacillin, Flucloxacillin, Mezlocillin, Methicillin, Nafcillin, Oxacillin, Penicillin G, Penicillin V, Piperacillin, Temocillin, Ticarcillin, Bacitracin, Colistin, Polymyxin B, Ciprofloxacin, Enoxacin, Gatifloxacin, Levofloxacin, Lomefloxacin, Moxifloxacin, Nalidixic acid, Norfloxacin, Ofloxacin, Trovafloxacin, Grepafloxacin, Sparfloxacin, Temafloxacin, Mafenide, Sulfonamidochrysoidine, Sulfacetamide, Sulfadiazine, Silver sulfadiazine, Sulfamethizole, Sulfamethoxazole (SMZ), Sulfanilimide, Sulfasalazine, Sulfisoxazole, Trimethoprim (TMP), Trimethoprim-Sulfamethoxazole (such as Bactrim, Septra), Demeclocycline, Doxycycline, Minocycline, Oxytetracycline, Tetracycline, Clofazimine, Dapsone, Capreomycin, Cycloserine, Ethambutol, Ethionamide, Isoniazid, Pyrazinamide, Rifampicin, Rifabutin, Rifapentine, Streptomycin, Arsphenamine, Chloramphenicol, Fosfomycin, Fusidic acid, Linezolid, Metronidazole, Mupirocin, Platensimycin, Quinupristin/Dalfopristin, Rifaximin, Thiamphenicol, or Tinidazole. In an exemplary embodiment, the antibiotic is TMP, SMZ, or a combination thereof.

### III. Pharmaceutical compositions

Yet another aspect of the invention encompasses a pharmaceutical composition. A compound of the invention described in section I above may exist in tautomeric, geometric or stereoisomeric forms. The present invention contemplates all such compounds, including cis- and trans-geometric isomers, E- and Z-geometric isomers, R- and S-enantiomers, diastereomers, d-isomers, I-isomers, the racemic mixtures thereof and other mixtures thereof. Pharmaceutically acceptable salts of such tautomeric, geometric or stereoisomeric forms are also included within the invention. The terms "cis" and "trans", as used herein, denote a form of geometric isomerism in which two carbon atoms connected by a double bond will each have a hydrogen atom on the same side of the double bond ("cis") or on opposite sides of the double bond ("trans"). Some of the compounds described contain alkenyl groups, and are meant to include both cis and trans or "E" and "Z" geometric forms. Furthermore, some of the compounds described contain one or more stereocenters and are meant to include R, S, and mixtures of R and S forms for each stereocenter present.

In a further embodiment, the inhibitors of the present invention may be in the form of free bases or pharmaceutically acceptable acid addition salts thereof. The term "pharmaceutically-acceptable salts" are salts commonly used to form alkali metal salts and to form addition salts of free acids or free bases. The nature of the salt may vary, provided that it is pharmaceutically acceptable. Suitable pharmaceutically acceptable acid addition salts of compounds for use in the present methods may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, hydroxybutyric, salicylic, galactaric and galacturonic acid. Suitable pharmaceutically-acceptable base addition salts of compounds of use in the present methods include metallic salts made from aluminum, calcium, lithium, magnesium, potassium, sodium and zinc or organic salts made from N, N'-dibenzylethylenediamine, chloroprocaine, choline, diethanolamine, ethylenediamine, meglumine- (N-methylglucamine) and procaine. All of these salts may be prepared by conventional means from the corresponding compound by reacting, for example, the appropriate acid or base with any of the compounds of the invention.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid are useful in the preparation of injectables. Dimethyl acetamide, surfactants including ionic and non-ionic detergents, and polyethylene glycols can be used. Mixtures of solvents and wetting agents such as those discussed above are also useful.

Solid dosage forms for oral administration may include capsules, tablets, pills, powders, and granules. In such solid dosage forms, the compound is ordinarily combined with one or more adjuvants appropriate to the indicated route of administration. If administered per os, the compound can be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets can contain a controlled-release formulation as can be provided in a dispersion of active compound in hydroxypropylmethyl cellulose. In the case of capsules, tablets, and pills, the dosage forms can also comprise buffering agents such as sodium citrate, or magnesium or calcium carbonate or bicarbonate. Tablets and pills can additionally be prepared with enteric coatings.

For therapeutic purposes, formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art. For instance, a compound of the invention may be administered with a carrier. Non-limiting examples of such a carrier include protein carriers and lipid carriers.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The amount of the compound of the invention that may be combined with the carrier materials to produce a single dosage of the composition will vary depending upon the subject and the particular mode of administration. Those skilled in the art will appreciate that dosages may also be determined with guidance from Goodman & Goldman's The Pharmacological Basis of Therapeutics, Ninth Edition (1996), Appendix II, pp. 1707-1711 and from Goodman & Goldman's The Pharmacological Basis of Therapeutics, Tenth Edition (2001), Appendix II, pp. 475-493.

A compound of the invention may also be formulated as a prodrug. Such a prodrug formulation may increase the bioavailability of a compound of the invention. In one embodiment, the sugar portion of a compound of the invention may encompass a prodrug. In another embodiment R¹ may comprise a prodrug. Non-limiting examples of a compound of the invention formulated as a prodrug include the compounds below:

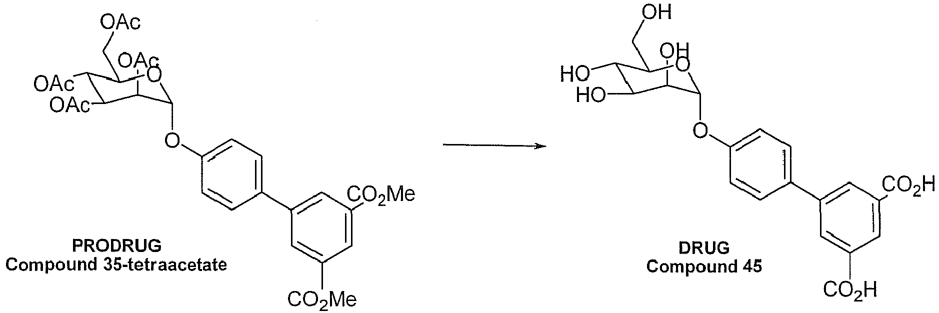

### IV. Methods of the invention

Compounds of the invention may be used in methods of treating a bacterial infection and methods of reducing resistance to a bactericidal compound in a bacterium.

### (a) methods of treating a bacterial infection

One embodiment of the invention encompasses a method for treating bacterial infections. As used herein, "treating" refers to preventing infection in a subject not currently infected, and reducing or eliminating infection in a subject that is currently infected. Generally, such a method comprises administering a pharmaceutical composition comprising a compound of the invention to a subject. As used herein, "subject" includes any mammal prone to urinary tract infections by E. coli. In one embodiment, a subject is prone to recurring UTIs. In some embodiments, a subject may not have clinical symptoms of a UTI. In such embodiments, the subject may have a latent infection. In other embodiments, a subject may have clinical symptoms of a UTI.

In some embodiments, a compound of the invention may be administered to a subject in combination with a bactericidal compound as described in Section II above. When administered in a combination, a compound of the invention may be administered before, simultaneously, or after administration of a bactericidal compound. When administered before or after a bactericidal compound, the time between administration of a compound of the invention and a bactericidal compound may be about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 min. In another embodiment, the time between administration of a compound of the invention and a bactericidal compound may be about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72 hours.

A compound or pharmaceutical composition of the invention may be administered by several different means that will deliver a therapeutically effective dose. Such compositions may be administered orally, parenterally, by inhalation spray, rectally, intradermally, intracisternally, intraperitoneally, transdermally, bucally, as an oral or nasal spray, topically (i.e. powders, ointments or drops), or via a urinary cathetar in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous, intravenous, intramuscular, or intrasternal injection, or infusion techniques. In an exemplary embodiment, the pharmaceutical composition will be administered in an oral dosage form. Formulation of drugs is discussed in, for example, Hoover, John E., Remington's Pharmaceutical Sciences, Mack Publishing Co., Easton, Pa. (1975), and Liberman, H. A. and Lachman, L., Eds., Pharmaceutical Dosage Forms, Marcel Decker, New York, N.Y. (1980).

The amount of a compound of the invention that constitutes an "effective amount" can and will vary. The amount will depend upon a variety of factors, including whether the administration is in single or multiple doses, and individual subject parameters including age, physical condition, size, and weight. Those skilled in the art will appreciate that dosages may also be determined with guidance from Goodman & Goldman's The Pharmacological Basis of Therapeutics, Ninth Edition (1996), Appendix II, pp. 1707-1711 and from Goodman & Goldman's The Pharmacological Basis of Therapeutics, Tenth Edition (2001), Appendix II, pp. 475-493.

In order to selectively control the release of an inhibitor to a particular region of the gastrointestinal tract for release, the pharmaceutical compositions of the invention may be manufactured into one or several dosage forms for the controlled, sustained or timed release of one or more of the ingredients. In this context, typically one or more of the ingredients forming the pharmaceutical composition is microencapsulated or dry coated prior to being formulated into one of the above forms. By varying the amount and type of coating and its thickness, the timing and location of release of a given ingredient or several ingredients (in either the same dosage form, such as a multi-layered capsule, or different dosage forms) may be varied.

In an exemplary embodiment, the coating may be an enteric coating. The enteric coating generally will provide for controlled release of the ingredient, such that drug release can be accomplished at some generally predictable location in the lower intestinal tract below the point at which drug release would occur without the enteric coating. In certain embodiments, multiple enteric coatings may be utilized. Multiple enteric coatings, in certain embodiments, may be selected to release the ingredient or combination of ingredients at various regions in the lower gastrointestinal tract and at various times.

As will be appreciated by a skilled artisan, the encapsulation or coating method can and will vary depending upon the ingredients used to form the pharmaceutical composition and coating, and the desired physical characteristics of the microcapsules themselves. Additionally, more than one encapsulation method may be employed so as to create a multi-layered microcapsule, or the same encapsulation method may be employed sequentially so as to create a multi-layered microcapsule. Suitable methods of microencapsulation may include spray drying, spinning disk encapsulation (also known as rotational suspension separation encapsulation), supercritical fluid encapsulation, air suspension microencapsulation, fluidized bed encapsulation, spray cooling/chilling (including matrix encapsulation), extrusion encapsulation, centrifugal extrusion, coacervation, alginate beads, liposome encapsulation, inclusion encapsulation, colloidosome encapsulation, sol-gel microencapsulation, and other methods of microencapsulation known in the art. Detailed information concerning materials, equipment and processes for preparing coated dosage forms may be found in Pharmaceutical Dosage Forms: Tablets, eds. Lieberman et al. (New York: Marcel Dekker, Inc., 1989), and in Ansel et al., Pharmaceutical Dosage Forms and Drug Delivery Systems, 6th Ed. (Media, Pa.: Williams & Wilkins, 1995).

A bacterium may be contacted with a compound of the invention in vivo, in vitro, in situ, or ex vivo. In some embodiments, a bacterium may be directly contacted with the compound of the invention. In other embodiments, an intracellular bacterium may be contacted with a compound of the invention. Suitable cells comprising one or more bacteria may be grown, sub-cultured, stored and manipulated using standard techniques known to individuals skilled in the art. Cell culture and microbiological techniques for growing, culturing, storing, and manipulating cells comprising one or more bacteria are commonly known in the art.

### (b) methods of reducing bactericidal resistance

Another method of the invention comprises reducing the resistance of a bacterium to a bactericidal compound. Such a method comprises contacting a bacterium resistant to a bactericidal compound with a compound of the invention. For instance, a subject infected with a bacterium resistant to a bactericidal compound may be administered a compound of the invention, as described in section IV(a) above. In an exemplary embodiment, a method comprises contacting a bacterium resistant to an antibiotic with a compound of the invention. In a further exemplary embodiment, a method comprises contacting a bacterium resistant to TMP or SMZ with a compound of the invention.

Methods of measuring resistance of a bacterium to an antibiotic are known in the art. For more details, see the examples.

### DEFINITIONS

The term "acyl," as used herein alone or as part of another group, denotes the moiety formed by removal of the hydroxyl group from the group --COOH of an organic carboxylic acid, e.g., RC(O)--, wherein R is R', R₁O--, R'R₂ N--, or R₁S--, R₁ is hydrocarbyl, heterosubstituted hydrocarbyl, or heterocyclo and R₂ is hydrogen, hydrocarbyl or substituted hydrocarbyl.

The term "acyloxy," as used herein alone or as part of another group, denotes an acyl group as described above bonded through an oxygen linkage (-O--), e.g., RC(O)O-- wherein R is as defined in connection with the term "acyl."

Unless otherwise indicated, the alkyl groups described herein are preferably lower alkyl containing from one to eight carbon atoms in the principal chain and up to 20 carbon atoms. They may be straight or branched chain or cyclic and include methyl, ethyl, propyl, isopropyl, butyl, hexyl and the like.

Unless otherwise indicated, the alkenyl groups described herein are preferably lower alkenyl containing from two to eight carbon atoms in the principal chain and up to 20 carbon atoms. They may be straight or branched chain or cyclic and include ethenyl, propenyl, isopropenyl, butenyl, isobutenyl, hexenyl, and the like.

Unless otherwise indicated, the alkynyl groups described herein are preferably lower alkynyl containing from two to eight carbon atoms in the principal chain and up to 20 carbon atoms. They may be straight or branched chain and include ethynyl, propynyl, butynyl, isobutynyl, hexynyl, and the like.

The terms "aryl" or "ar" as used herein alone or as part of another group denote optionally substituted homocyclic aromatic groups, preferably monocyclic or bicyclic groups containing from 6 to 12 carbons in the ring portion, such as phenyl, biphenyl, naphthyl, substituted phenyl, substituted biphenyl or substituted naphthyl. Phenyl and substituted phenyl are the more preferred aryl.

As used herein, the term "functional group" includes a group of atoms within a molecule that is responsible for certain properties of the molecule and/or reactions in which it takes part. Non-limiting examples of functional groups include, alkyl, carboxyl, hydroxyl, amino, sulfonate, phosphate, phosphonate, thiol, alkyne, azide, halogen, and the like.

The terms "halogen" or "halo" as used herein alone or as part of another group refer to chlorine, bromine, fluorine, and iodine.

The terms "heterocyclo" or "heterocyclic" as used herein alone or as part of another group denote optionally substituted, fully saturated or unsaturated, monocyclic or bicyclic, aromatic or nonaromatic groups having at least one heteroatom in at least one ring, and preferably 5 or 6 atoms in each ring. The heterocyclo group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heterocyclo include heteroaromatics such as furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "heteroaromatic" as used herein alone or as part of another group denote optionally substituted aromatic groups having at least one heteroatom in at least one ring, and preferably 5 or 6 atoms in each ring. The heteroaromatic group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heteroaromatics include furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The terms "hydrocarbon" and "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The "substituted hydrocarbyl" moieties described herein are hydrocarbyl moieties which are substituted with at least one atom other than carbon, including moieties in which a carbon chain atom is substituted with a hetero atom such as nitrogen, oxygen, silicon, phosphorous, boron, sulfur, or a halogen atom. These substituents include halogen, carbocycle, aryl, heterocyclo, alkoxy, alkenoxy, alkynoxy, aryloxy, hydroxy, protected hydroxy, keto, acyl, acyloxy, nitro, amino, amido, nitro, cyano, thiol, ketals, acetals, esters and ethers.

The following examples are included to demonstrate preferred embodiments of the invention. It should be appreciated by those of skill in the art that the techniques disclosed in the examples that follow represent techniques discovered by the inventors to function well in the practice of the invention. Those of skill in the art should, however, in light of the present disclosure, appreciate that many changes can be made in the specific embodiments that are disclosed and still obtain a like or similar result without departing from the spirit and scope of the invention, therefore all matter set forth or shown in the accompanying drawings is to be interpreted as illustrative and not in a limiting sense.

### EXAMPLES

The following examples illustrate various iterations of the invention.

### Example 1. Uropathogenic E. coli (UPEC) pathogenesis in the urinary tract.

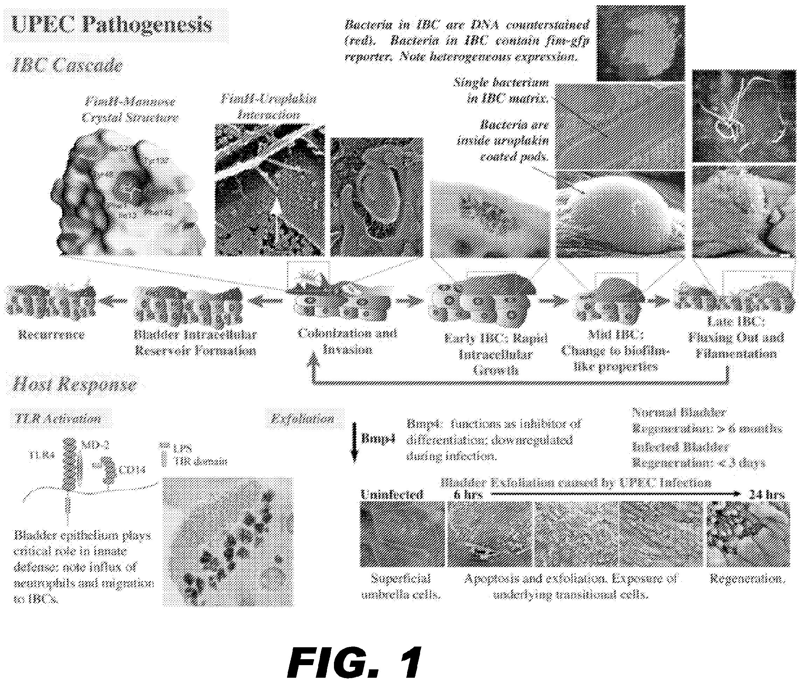

Clinically, it has been presumed that UPEC infection consists of a relatively simple extracellular colonization of the luminal surface after inoculation of fecal flora into the bladder via the urethra. In contrast, using a murine model of UPEC infection of the UT, the inventors have detailed an unexpectedly complex UPEC pathogenesis cycle that involves both intracellular and extracellular niches (**Fig. 1**). Using genetic, biochemical and cell biological approaches together with a variety of imaging techniques including transmission, quick freeze-deep etch and scanning electron microscopy, as well as confocal and time lapse video microscopy, the inventors discovered that UPEC invade bladder facet cells via a FimH-dependent mechanism (see below). After invasion, cytoplasmic intracellular bacterial communities (IBCs) are formed. Rapid replication of the initial invading bacteria results in the formation of an early IBC of loosely-packed rod-shaped bacteria. The bacteria continue to replicate and progress to form a large densely packed mid-stage IBC of morphologically coccoid bacteria, with biofilm-like characteristics including positive periodic acid-Schiff (PAS) staining and differential gene expression throughout the community. After the IBC matures, bacteria detach from the biomass, often become filamentous, and spread to neighboring cells forming new generation IBCs. Thus, the IBC pathway facilitates massive expansion of the invading bacteria in a niche protected from host defenses. Translational studies have shown that the majority of UPEC isolates form IBCs when introduced into the murine bladder and that IBCs and filamentous bacteria occur in the urine of human UTI patients. Population dynamic studies conducted by the inventors using *ex vivo* gentamicin protection assays demonstrated that ∼10⁴ UPEC of an initial 10⁷ inoculum invaded the bladder tissue within 15 minutes after infection and that one percent of the invaded bacteria went on to form IBCs, resulting in an average of 100 IBCs per infected mouse bladder. If this is extrapolated to the human situation, innate defenses in the bladder most likely prevent the majority of bacterial inoculation events into the bladder from leading to disease. However, the ramifications of the IBC cascade are striking. Invasion of a single infecting bacterium can lead to rapid expansion of the infection via IBC formation, replicating within hours to 10⁴ bacteria and even higher numbers followed by dispersal of the bacteria from the biomass and spreading to neighboring cells to reinitiate the IBC cascade. This process allows the bacteria to gain a critical foothold. Bacterial descendents of the acute IBC cascade have been shown using a murine model, to be able to form a quiescent intracellular reservoir (QIR) that can persist, protected from antibiotics and seemingly undetected by the host immune system even after the acute infection is resolved and bacteria are no longer detectable in the urine. Bacteria in the QIR can later seed a recurrent infection, manifested by IBC formation, bacteruria and inflammation.

### Example 2. FimH as a therapeutic target.

There are several key implications from understanding UPEC pathogenesis. Mannosides and pilicides that block FimH function will prevent bacterial adherence and invasion and thus prevent bacterial amplification in the IBC and subsequent spreading and repeated rounds of amplification via new generation IBCs. These compounds will have potent therapeutic activity by preventing bacterial expansion which may also have the consequence of eliminating or significantly reducing the QIR thus reducing predisposition to recurrent infection.

### Type 1 pili / FimH are critical for UPEC pathogenesis in the UT.

Type 1 pili are essential cystitis virulence determinants. Using scanning and high-resolution EM and the mouse cystitis model developed by the inventors, it was shown that adhesive type 1 piliated bacteria are able to bind and invade host superficial umbrella cells, while UPEC lacking type 1 pili are not. Colonization and invasion of the bladder epithelium is dependent on the FimH adhesin located at the distal end of the pilus that binds mannose residues on bladder epithelial cells. High-resolution freeze-dry/deep-etch EM revealed that FimH interacts directly with receptors on the luminal surface of the bladder (**Fig. 1**). Standard gentamicin protection assays of infected tissue culture cells and *ex vivo* gentamicin treatment of infected bladders demonstrated that *fimH*⁺ type 1 piliated clinical cystitis isolates, but not *fimH⁻* mutants, could invade bladder epithelial cells. Using immunohistochemistry and *Pfim-gfp* transcriptional fusions it was demonstrated that type 1 pili are expressed within IBCs. Using high-resolution EM, pilus-like fibers radiating from bacteria and interacting with matrix material within the intracellular IBC were also visualized (**Fig 1**). These results combined with work showing that type 1 pili are required for biofilm formation in *in vitro* systems led to the hypothesis that type 1 pili promote IBC formation and/or maintenance. Therefore, an anhydrotetracycline (AHT) inducible *fim* strain was constructed which can be "pre-piliate" UTI89 *in vitro* by growth in AHT before infecting mouse bladders, allowing the initial invasion event to normally. However, once inoculated into the mouse, AHT is no longer present, *fim* transcription ceases and piliation is diluted upon each bacterial division. Using this system, the earliest events of colonization and invasion were identical between the wild type and conditional strain. However, the inability of the conditional strain to produce type 1 pili intracellularly abolished its ability to form IBCs, as shown by confocal microscopy, and thus dramatically attenuated virulence as determined by CFUs at later time points. These results strongly suggest that type 1 pili are required for the survival and proliferation of UPEC within superficial facet cells. Additionally, this conditional mutant is significantly impaired in its ability to form QIRs, arguing that the bacteria in QIRs are descendents and thus dependent on the acute IBC cascade.

### Structural studies of FimH and its ligand.

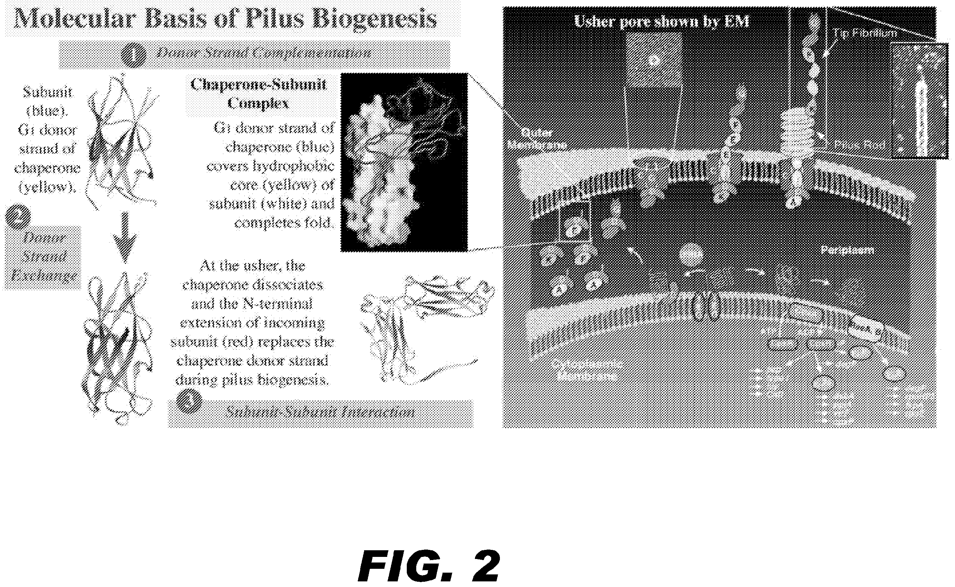

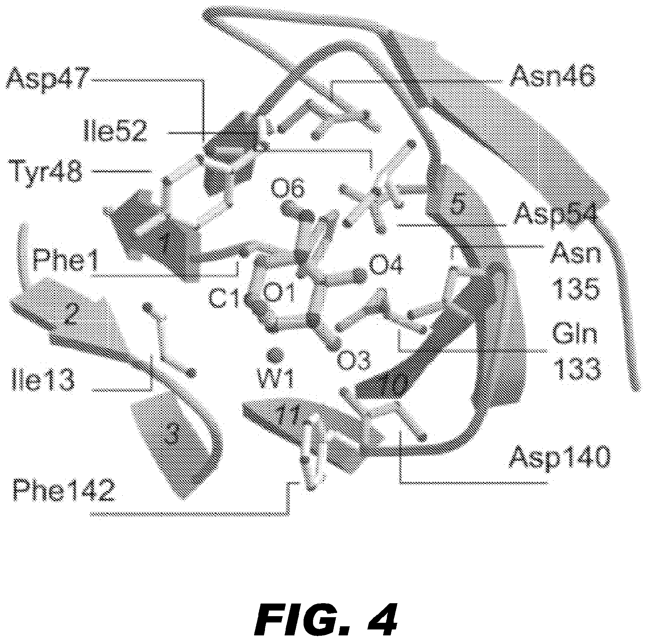

Adhesive type 1 pili are prototypic structures of a family of adhesive fibers produced by diverse Gram-negative bacteria via the chaperone/usher assembly pathway. Using biochemistry, mutational studies, nuclear magnetic resonance, and x-ray crystallography, the molecular basis of pili assembled by the chaperone/usher pathway in gram-negative bacteria, including type 1 pili of UPEC, were delineated (**Fig. 2**). The three dimensional structure of FimH bound to its mannose receptor was solved in order to gain a molecular snapshot of a critical initial event in UTI pathogenesis (**Fig**. **3**). FimH is a two domain protein, with a receptor binding domain linked to a typical pilin domain that joins the adhesin to the pilus fiber. The structure of the complex of the FimC chaperone bound to FimH (which was bound to D-mannopyranoside) was determined to 2.8Å resolution. The mannose binding site of FimH is a deep negatively charged pocket at the tip of its receptor-binding domain. The FimH pocket engages in extensive hydrogen bonding to mannose (**Fig. 4**), which are abundant in the oligosaccharide moieties of uroplakins that coat the lumenal surface of the bladder epithelium. A hydrophobic ridge surrounds the mannose binding pocket in a manner that may facilitate polar interactions within the FimH pocket. Mutational studies revealed that each residue is critical in mannose binding and pathogenesis, emphasizing why the pocket is invariant among UPEC isolates.

### Devlopment of anti-adhesives.

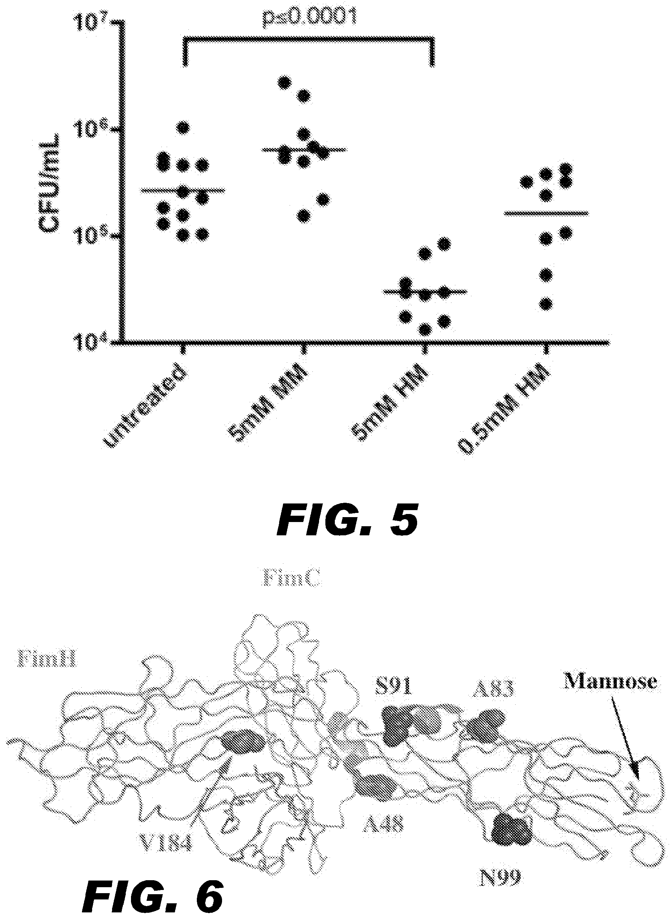

The FimH-mannose interaction was further investigated in an effort to develop potential ligand-based antagonists of UTIs. The chitobiose unit on oligomannose was found to bridge various mannose derivatives to the asparagine in the Asn-X-Ser/Thr motif of FimH resulting in higher affinity binding. Crystallization of FimH in complex with oligomannose-3 revealed the mechanism of this higher affinity binding. The non-reducing Man4 anchors into the mannose-binding pocket while the GlcNAc folds over Thr51 allowing specific interactions with a hydrophobic tyrosine gate. Heptyl mannoside mimics the GlcNAc tail of oligomannose-3 and extends it further to increase interactions outside the binding pocket resulting in high affinity binding (Kd = 5 nM). Based on the high affinity of heptyl mannose for FimH, the ability of heptyl mannose to reduce bacterial infection in our mouse model of UTI was tested. First, biofilm formation as a surrogate for IBCs formed in the bladder was evaluated. Heptyl mannose at 1mM inhibited UPEC biofilm formation *in vitro,* suggesting that the mannose binding properties of the FimH adhesin is required for biofilm formation. Thus, UPEC strain UTI89 was incubated with heptyl mannose prior to inoculation into the bladders of mice. This resulted in a significant attenuation of virulence at 6 hours post-infection at 5 mM heptyl mannose (**Fig. 5**). The ability of these compounds to significantly attenuate virulence establishes mannosides as a potential treatment for UTI. Therefore, more potent mannosides that mimic the natural receptor for FimH but with increased affinity and avidity in order to ultimately block bacterial colonization, invasion, IBC formation and disease were developed as described below.

### Example 3. Genetic analysis of UPEC baterial isolates.

Due to the necessity of FimH, it was tested to see if this gene was under positive selection. Through sequencing of more than 200 isolates, the majority of which were UTI isolates, it was discovered that residues on FimH are evolving under positive selection, none of which were in the mannose binding pocket. These residues are A48, A83 and V184 in UT189 (**Fig 6**). Mutants were constructed containing the other naturally occurring amino acids commonly found at the positively selected positions. A mutation of A83 to a serine resulted in a defect in binding *in vitro,* however only a log drop in bacterial titers were observed *in vivo.* The double mutant containing a valine at position 48 and an alanine at position 184 resulted in a severe defect *in vivo* based on bacterial titers and no defect in the ability to bind mannose *in vitro.* Mutations in the non-positively selected amino acids had no detectable effect. This study shows that residues outside the binding pocket are also important to FimH's function *in vivo* suggesting there is more to FimH than just its ability to bond to the surface of the bladder through its mannose binding pocket.

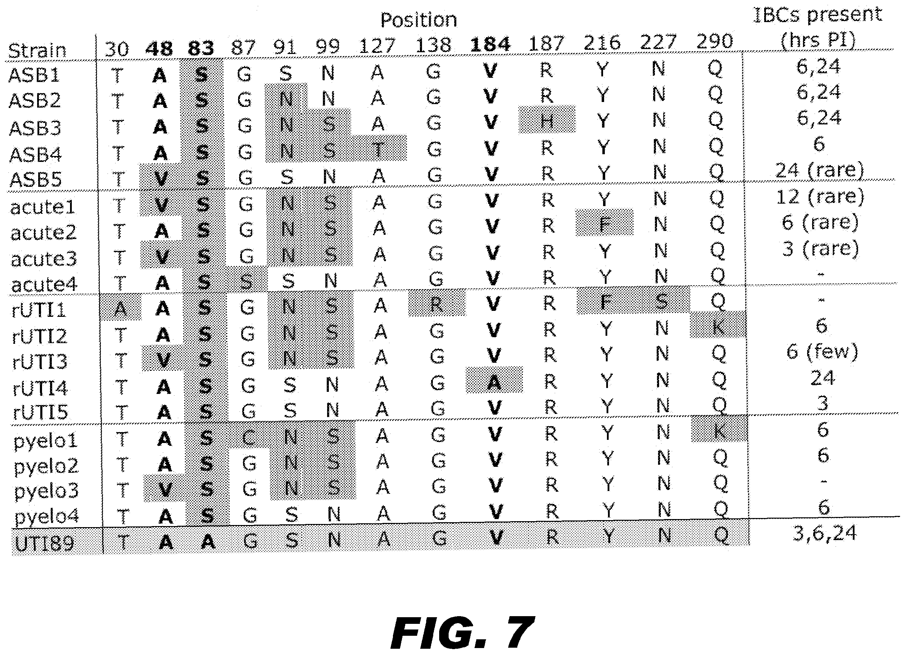

Since FimH has been the only essential virulence factor determined to date, it was investigated if the FimH from UT189 would result in establishing a more robust infection if it was put into a clinical isolate containing an altered FimH. A panel of clinical isolates was examined for their ability to form IBCs (**Fig. 7**). Isolates with a valine at position 48 seemed to perform worse in vivo in regards to IBC formation.

### Example 4. Role of FimH in infection

To assess FimH's role in infection, the native FimH of the clinical strain acute 4 (**Fig. 7**) was replaced with the FimH sequence from UT189 under the control of the native promoter. This was done through lambda red recombinase. The acute4 FimH was first deleted and replaced with a chloramphenicol (Cm) cassette. The Cm cassette was then replaced with UT189 FimH. The isolate was then sequenced to confirm proper sequence.

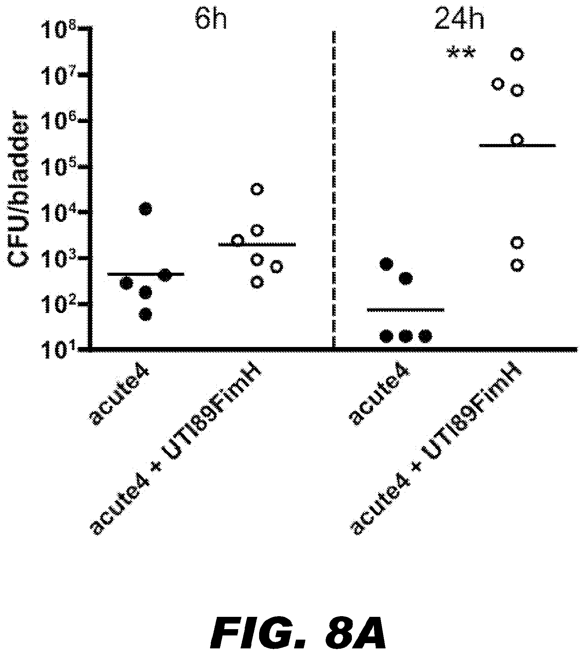

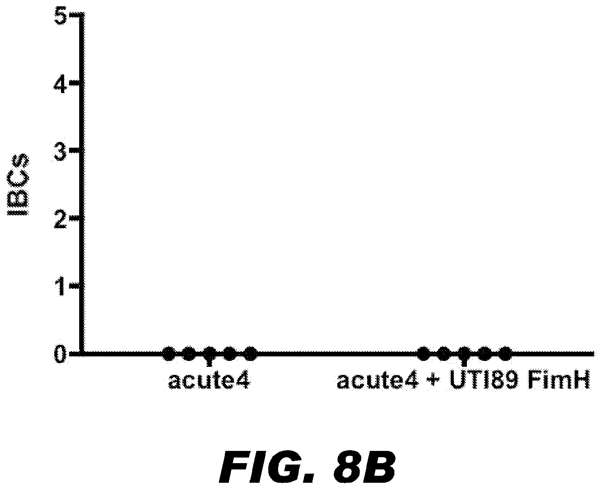

Acute4 and acute4 + UT189FimH were grown 2x24 hours to obtain type 1 expression. C3H/HeN mice were then inoculated with 10⁷ of either strain. For bacterial titers (**Fig. 8A**), bladders were harvested at 6 and 24 hours post-infection. At 6 hours post-infection there was no significant difference in bacterial load between the two stains and both strains were much lower that a typical UT189 6 hour titer (∼10⁶). However, at 24 hours post-infection, acute4 + UT189FimH significantly colonized the murine bladder more than actue 4 (p<0.001 by Mann-Whitney). 24 hour titers in the acute4 + UT189FimH isolate were equivalent to what is typically observed with UT189. For IBC formation (**Fig. 8B**), LacZ staining was performed at 6 hours post-infection. No IBCs were observed in either isolate.

### Example 5. Synthesis of compounds

The compounds of the present invention may be prepared in a number of ways well known to one skilled in the art of organic synthesis. More specifically, the novel compounds of this invention may be prepared using the reactions and techniques described herein. In the description of the synthetic methods described below, it is to be understood that all proposed reaction conditions, including choice of solvent, reaction atmosphere, reaction temperature duration of the experiment and workup procedures, are chosen to be the conditions standard for that reaction. It is understood by one skilled in the art of organic synthesis that the functionality present on various portions of the molecule must be compatible with the reagents and reactions proposed. Such restrictions to the substituents, which are not compatible with the reaction conditions, will be apparent to one skilled in the art and alternate methods must then be used. Unless otherwise stated, the starting materials for the examples contained herein are either commercially available or are readily prepared by standard methods from known materials. The compounds of Formula (I) and (II) may be synthesized through standard organic chemistry methodology and purification known to those trained in the skill and art by using commercially available starting materials and reagents. General procedures for synthesizing the compounds of the invention are as follows:

The chemical synthesis of compounds with the general Formula (I) and (II) is achieved using the routes described in **Scheme 1** starting from commercially available α-D-mannose, α-D-mannose pentaacetate (Route A), or α-methyl-D mannose (Route B). Shown in Scheme 1A, Lewis-acid mediated glycosidation of α-D-mannose pentaacetate followed by acetate ester hydrolysis with sodium methoxide yields glycosides of Formula (I). Further chemical transformation of the substituent R¹ may be accomplished using standard synthetic organic procedures such as, but not limited to, metal-mediated aryl Suzuki coupling reactions through aryl bromide or aryl boronate ester glycosides, shown in **Scheme 2A and 2B,** respectively, to produce exemplified compounds of Formula (I) described in **Tables 1-9.** All compounds are also listed in **Table 15.**

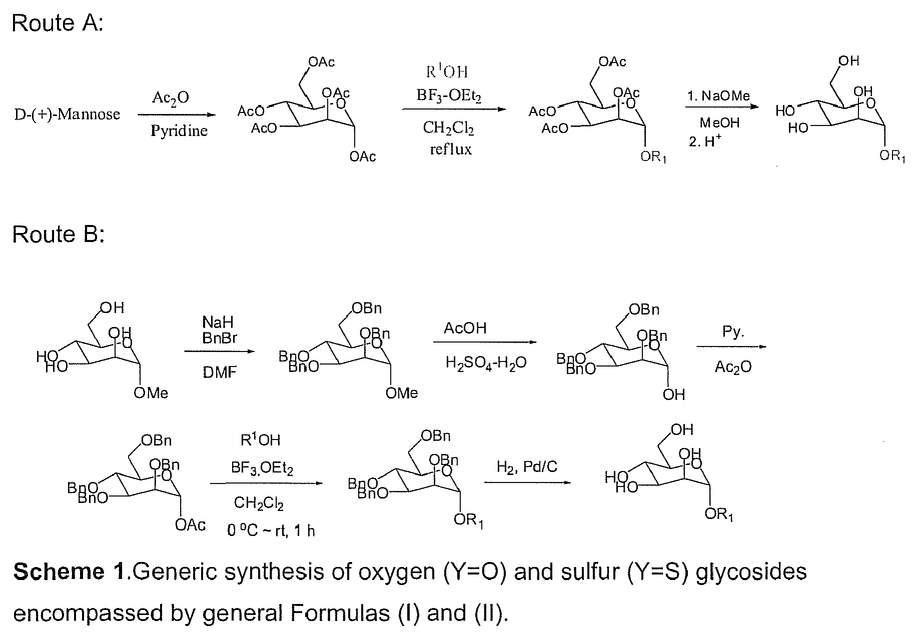

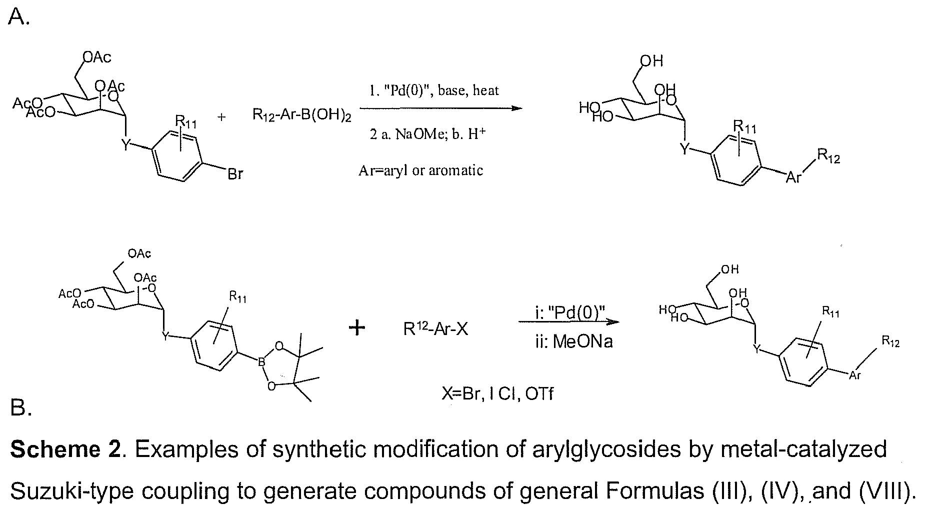

The compounds of Formula (I) may be transformed into compounds of Formula (II) as described in Scheme 3 by employing various functional groups (R¹) and linkers(L), such as, but not limited to, alkyl ethers, esters, amines and amides.

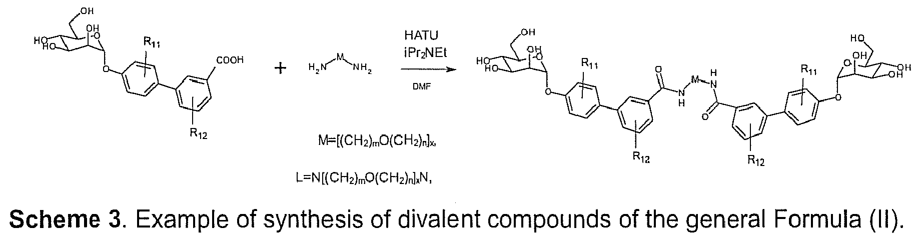

The invention is further described with reference to the following illustrative examples in which, unless stated otherwise:
(i) temperatures are given in degrees Celsius (°C); operations are carried out at roomtemperature or ambient temperature, that is, in a range of 18-25 °C, unlessotherwise stated;
(ii) solutions are dried over anhydrous sodium sulphate or magnesium sulphate;
   evaporation of organic solvent is carried out using a rotary evaporator under reduced pressure (4.5-30 mmHg) with a bath temperature of up to 60 °C;
(iii) chromatography means flash chromatography on silica gel; thin layer chromatography (TLC) is carried out on silica gel plates;
(iv) in general, the course of reactions are followed by TLC or liquid chromatography/mass spectroscopy (LC/MS) and reaction times are given for illustration only;
(v) final products have satisfactory proton nuclear magnetic resonance (NMR) spectra and/or mass spectra data;
(vi) yields are given for illustration only and are not necessarily those which can be obtained by diligent process development; preparations are repeated if more material is required;
(vii) when given, nuclear magnetic resonance (NMR) data is in the form of delta (δ)values for major diagnostic protons, given in part per million (ppm) relative to tetramethylsilane (TMS) as an internal standard, determined at 300 MHz ind₃-MeOD unless otherwise stated;
(viii) chemical symbols have their usual meanings;
(ix) solvent ratio is given in volume: volume (v/v) terms;
(x) Purification of the compounds is carried out using one or more of the following methods:
   a) flash chromatography on regular silica gel;
   b) flash chromatography on silica gel using an MPLC separation system (Teledyne ISCO):prepackednormal phase flash column cartridge, flow rate, 10-80 ml/min;
   c) Preparatory HPLC system using a reverse-phase C18 column, 100 x 20 mm, 5 uM (or larger) and eluting with combinations of water (0.1% TFA) and MeCN (0.1% TFA) as the mobile phase;
(xi) the following abbreviations have been used:
   - Ac: acetyl;
   - CIV: concentrated in vacuo;
   - RTandrt: room temperature;
   - BOC: tert-butoxycarbonyl;
   - HATU: 2-(7-Aza-1H-benzotriazole-1-yl)-1,1,3,3-tetramethyluronium hexafluorophosphate;
   - DIPEA: N,N-diisopropylethylamine;
   - CBZ: benzyloxycarbonyl;
   - Bn: benzyl;
   - DCM: dichloromethane;
   - DMF: N,N-dimethylformamide;
   - DMSO: dimethylsulfoxide;
   - NMP: N-methyl-2-pyrrolidinone;
   - EtOAc: ethyl acetate;
   - ether: diethyl ether;
   - EtOH: ethanol;
   - THF: tetrahydrofuran;
   - MeOH: methanol;
   - MeCN: acetontrile;
   - TFA: trifluoracetic acid; and
   - TEA: triethylamine.

### 1. General procedures for the preparation of mannosides when α-D-mannose pentaacetate is used as the starting material: Methyl 3-[4-[(2R,3S,4S,5S,6R)-3,4,5-trihydroxy-6-(hydroxymethyl)tetrahydropyran-2-yl]oxyphenyl]benzoate (29)

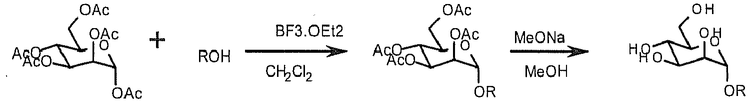

**1.1 Methyl 3-[4-[(2R,3S,4S,5R,6R)-3,4,5-triacetoxy-6-(acetoxymethyl)tetrahydropyran-2-yl]oxyphenyl]benzoate:** Under nitrogen atmosphere, at 0 °C boron trifluoride diethyl etherate (0.128 g, 0.90 mmol) was added dropwise into the solution of α-D-mannose pentaacetate (0.120 g, 0.3mmol) and methyl 3-(4-hydroxyphenyl)benzoate (0.140 g, 0.6mmol) in 6 ml of CH₂Cl₂. After a few mins the mixture was heated to reflux and kept stirring for more than 36 hrs. The reaction was then quenched with water and extracted with CH₂Cl₂. The CH₂Cl₂ layer was collected, dried with Na₂SO₄, concentrated. The resulting residue was purified by silica gel chromatography with hexane/ethyl acetate combinations as eluent, giving rise to methyl 3-[4-[(2R,3S,4S,5R,6R)-3,4,5-triacetoxy-6-(acetoxymethyl)tetrahydropyran-2-yl]oxyphenyl]benzoate (0.136 g) in 81% yield. ¹H NMR(300 MHz, CDCl₃) δ ppm8.23 (m, 1H), 7.99 (m, 1H), 7.74 (m, 1H), 7.57 (m, 2H), 7.50 (t, *J* = 7.8 Hz, 1H), 7.18 (m, 2H), 5.59 (dd, *J* = 3.6, 9.9 Hz, 1H), 5.58 (d, *J* = 1.5 Hz, 1H),5.48 (dd, *J* = 2.1, 3.3 Hz, 1H),5.39 (t, *J* = 10.2 Hz, 1H), 4.30 (dd, *J* = 5.4, 12.3 Hz, 1H), 4.06∼4.15 (m, 2 H), 3.95 (s, 3H), 2.15 (s, 3H), 2.06 (s, 3H),2.05 (s, 3H),2.04 (s, 3H); MS (ESI): found: [M + H]⁺, 559.1.

**1.2 Methyl 3-[4-[(2R,3S,4S,5S,6R)-3,4,5-trihydroxy-6-(hydroxymethyl)tetrahydropyran-2-yl]oxyphenyl]benzoate (29):** Methyl 3-[4-[(2R,3S,4S,5R,6R)-3,4,5-triacetoxy-6-(acetoxymethyl)tetrahydropyran-2-yl]oxyphenyl]benzoate (0.120 g) was stirred in 6 mL of methanol with catalytic amount of sodium methoxide (0.02 M) at room temperature overnight. H⁺ exchange resin (DOWEX 50WX4-100) was added to neutralize the mixture. The resin was filtered off and the filtrate was concentrated, then dried in vacuo giving rise to pure product **29** (0.084 g) in quantitative yield. ¹H NMR(300 MHz, CD₃OD) δ ppm 8.21 (m, 1H), 7.95 (m, 1H), 7.83 (m, 1H), 7.59 (m, 2H), 7.53 (m, 1H), 7.23 (m, 2H), 5.55 (d, *J* = 1.8 Hz, 1H), 4.03 (dd, *J* = 1.8, 3.3 Hz, 1H), 3.91∼3.96 (m, 4H), 3.70∼3.82 (m, 3H), 3.59∼3.65 (m, 1H); MS (ESI): found: [M + H]⁺, 391.1. (see Table 1)

### 2. Procedures for the preparation of mannosides when 2,3,4,6-tetra-O-benzyl-1-acetyl-α-D-mannopyranoseis used as the starting material:[3-[(2R,3S,4S,5S,6R)-3,4,5-trihydroxy-6-(hydroxymethyl)tetrahydropyran-2-yl]oxyphenyl] acetate (42):

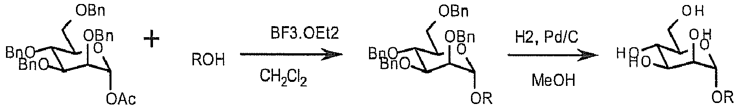

**2.1 [3-[(2R,3S,4S,5R,6R)-3,4,5-Tribenzyloxy-6-(benzyloxymethyl)tetrahydropyran-2-yl]oxyphenyl] acetate:** Under nitrogen atmosphere, at 0 °C boron trifluoride diethyl etherate (0.051 g, 0.36 mmol) was added dropwise into the solution of 2,3,4,6-tetra-O-benzyl-1-acetyl-α-D-mannopyranose (0.106 g, 0.18mmol) and resorcinol monoacetate (0.055 g, 0.36 mmol) in 7 ml of CH₂Cl₂. The mixture was stirred at 0 °C and monitored by TLC. The reaction was then quenched with water and extracted with CH₂Cl₂. The CH₂Cl₂ layer was collected, dried with Na₂SO₄, concentrated. The resulting residue was purified by silica gel chromatography with hexane/ethyl acetate combinations as eluent to give [3-[(2R,3S,4S,5R,6R)-3,4,5-tribenzyloxy-6-(benzyloxymethyl)tetrahydropyran-2-yl]oxyphenyl] acetate (0.093g) in 75% yield. ¹H NMR(300 MHz, CDCl₃) δ ppm 7.15∼7.40 (m, 21H), 6.88∼6.91 (m, 1H), 6.81 (t, *J* = 2.4 Hz, 1H), 6.73∼6.76 (m, 1H), 5.58 (d, *J* = 1.8 Hz, 1H), 4.90 (d, *J* = 10.8 Hz, 1H), 4.78 (s, 2H), 4.64∼4.69(m, 3H), 4.52 (d, *J* = 10.8 Hz, 1H), 4.45 (d, *J* = 12.3 Hz, 1H), 4.05∼4.18 (m, 2H), 3.94 (t, *J* = 2.4 Hz, 1H), 3.77∼3.85 (m, 2H), 3.64∼3.70 (m, 1H), 2.27 (s, 3H). MS (ESI): found: [M + Na]⁺, 697.2.

**2.2 [3-[(2R,3S,4S,5S,6R)-3,4,5-trihydroxy-6-(hydroxymethyl)tetrahydropyran-2-yl]oxyphenyl] acetate (42):** Under hydrogen atmosphere [3-[(2R,3S,4S,5R,6R)-3,4,5-tribenzyloxy-6-(benzyloxymethyl) tetrahydropyran-2-yl]oxyphenyl] acetate (0.085 g, 0.13 mmol) was stirred with Pd/C(10 wt.%) (0.132 g, 0.063 mmol) in ethanol (6 mL) and ethyl acetate (6 mL). The reaction was monitored by TLC until it went into completion. The mixture was filtered through a celite plug. The filtrate was concentrated then dried in vacuo furnishing pure **42** (0.040 g) in quantitative yield. ¹H NMR(300 MHz, CD₃OD) δ ppm 7.30 (t, *J* = 8.1 Hz, 1H), 7.00 (m, 1H), 6.90 (t, *J* = 2.1 Hz, 1H), 6.76 (m, 1H), 5.47 (d, *J* = 1.8 Hz, 1H), 4.00 (dd, *J* = 1.8, 3.3 Hz, 1H), 3.88 (dd, *J* = 3.3, 9.3 Hz, 1H), 3.68∼3.79 (m, 3H), 3.54∼3.61 (m, 1H). MS (ESI): found: [M + H]⁺, 314.9. (see Table 2)

### 3. Procedure for the preparation of 5-[4-[(2R,3S,4S,5S,6R)-3,4,5-Trihydroxy-6-(hydroxymethyl)tetrahydropyran-2-yl]oxyphenyl]benzene-1,3-dicarboxylic acid (45):

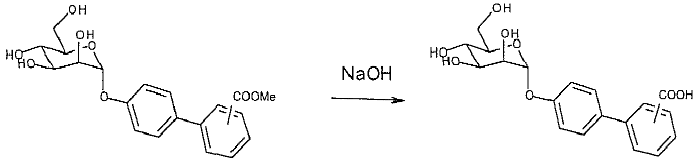

**35** (0.025 g, 0.056 mmol) was added into 7 mL methanol. Then 0.20 M NaOH aqueous (3 mL) was added. The mixture was stirred at rt overnight. H⁺ exchange resin (DOWEX 50WX4-100) was added to neutralize the mixture. The resin was filtered off and the filtrate was concentrated, then dried in vacuo giving rise to pure product **45** (0.023 g) in quantitative yield. ¹H NMR (300 MHz, METHANOL-*d*₄) δ ppm 3.59 - 3.67 (m, 1 H) 3.70 - 3.84 (m, 3 H) 3.94 (dd, *J*=3.30, 9.30 Hz, 1 H) 4.05 (dd, *J*=3.30, 1.80 Hz, 1 H) 5.56 (d, *J*=1.80 Hz, 1 H) 7.26 (m, 2 H) 7.63 (m, 2 H) 8.41 (d, *J*=1.50 Hz, 2 H) 8.57 (t, *J*=1.50 Hz, 1 H). MS (ESI): found: [M+Na]⁺, 443.0. (see Table 3)

### 4. Procedure for the preparation of N1,N3-dimethyl-5-[4-[(2R,3S,4S,5S,6R)-3,4,5-trihydroxy-6-(hydroxymethyl)tetrahydropyran-2-yl]oxyphenyl]benzene-1,3-dicarboxamide (50):

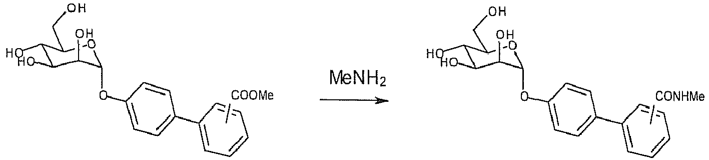

**35** (0.050 g, 0.11 mmol) was stirred with 15 mL of MeNH₂/EtOH (33 wt.%) at rt for 40 hrs. The solvent was removed and the residue was dried in vacuo to afford pure **50** (0.050 g) in quantitative yield. ¹H NMR (300 MHz, METHANOL-*d*₄) δ ppm 2.96 (s, 6 H) 3.61 - 3.66 (m, 1 H) 3.67 - 3.84 (m, 3 H) 3.86 - 3.97 (m, 1 H) 4.04 (dd, *J*=3.30, 1.92 Hz, 1 H) 5.56 (d, *J*=1.92 Hz, 1 H) 7.21 - 7.34 (m, 2 H) 7.63 - 7.74 (m, 2 H) 8.13 - 8.26 (m, 3 H). MS (ESI): found: [M+H]⁺, 447.4. (see Table 4)

### 5. Procedures for the preparation of biphenyl mannoside derivatives through Suzuki coupling reaction: 3-[4-[(2R,3S,4S,5S,6R)-3,4,5-trihydroxy-6-(hydroxymethyl)tetrahydropyran-2-yl]oxyphenyl]benzonitrile (62)

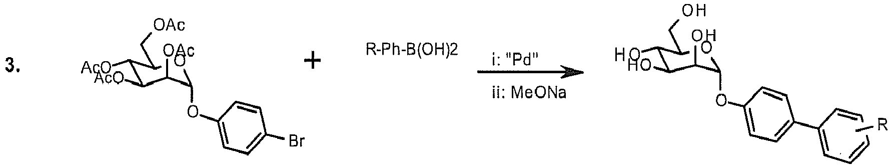

**5.1 [(2R,3S,4S,5R,6R)-3,4,5-Triacetoxy-6-[4-(3-cyanophenyl)phenoxy]tetrahydropyran-2-yl]methyl acetate:** Under nitrogen atmosphere, the mixture of acetyl-protected 4-bromophenyl α-D-mannoside (0.101 g, 0.2 mmol), m-cyanophenylboronic acid (0.044g, 0.3 mmol), cesium carbonate (0.196 g, 0.6 mmol) and tetrakis(triphenylphosphine)palladium (0.023 g, 0.02 mmol) in dioxane/water (5 mL/1 mL) was heated at 80 °C with stirring for 1 h. The solvent was removed and the resulting residue was purified by silica gel chromatography with hexane/ethyl acetate combinations as eluent to give [(2R,3S,4S,5R,6R)-3,4,5-triacetoxy-6-[4-(3-cyanophenyl)phenoxy]tetrahydropyran-2-yl]methyl acetate (0.080 g) in 76% yield. ¹H NMR(300 MHz, CDCl₃) δ ppm 7.82 (t, *J* = 1.5 Hz, 1H), 7.76 (dt, *J* = 7.69, 1.65 Hz, 1H), 7.58∼7.65 (m, 1H), 7.47∼7.57 (m, 3H), 7.16∼7.24 (m, 2H), 5.55∼5.65 (m, 2H), 5.47 (dd, *J* = 3.57, 1.92 Hz, 1H), 5.39 (t, *J* = 9.9 Hz, 1H), 4.25∼4.34 (m, 1H), 4.04∼4.17 (m, 2H), 2.22 (s, 3H), 2.06 (s, 3H), 2.05 (s, 3H), 2.04 (s, 3H). MS (ESI): found: [M + Na]⁺, 548.7.

**5.2 3-[4-[(2R,3S,4S,5S,6R)-3,4,5-Trihydroxy-6-(hydroxymethyl)tetrahydropyran-2-yl]oxyphenyl]benzonitrile (62):** [(2R,3S,4S,5R,6R)-3,4,5-Triacetoxy-6-[4-(3-cyanophenyl)phenoxy]tetrahydropyran-2-yl]methyl acetate (0.075 g) was stirred in 6 mL of methanol with catalytic amount of sodium methoxide (0.02 M) at room temperature overnight. H⁺ exchange resin (DOWEX 50WX4-100) was added to neutralize the mixture. The resin was filtered off and the filtrate was concentrated, then dried in vacuo giving rise to pure product **62** (0.045 g) in 88% yield. ¹H NMR(300 MHz, CD₃OD) δ ppm 3.55∼3.65 (m, 1H), 3.67∼3.83 (m, 3H), 3.84∼3.99 (m, 1H), 4.03 (dd, *J* = 3.43, 1.79 Hz, 1H), 5.55 (*J* = 1.65 Hz, 1H), 7.16∼7.33 (m, 2H), 7.54∼7.75 (m, 4H), 7.83∼8.01 (m, 2H). MS (ESI): found: [M + H]⁺, 358.3. (see Table 5)

### 6. Procedures for the preparation of biphenyl mannoside derivatives through Suzuki coupling reaction: Methyl 5-[4-[(2R,3S,4S,5S,6R)-3,4,5-trihydroxy-6-(hydroxymethyl)tetrahydropyran-2-yl]oxyphenyl]pyridine-3-carboxylate (77)

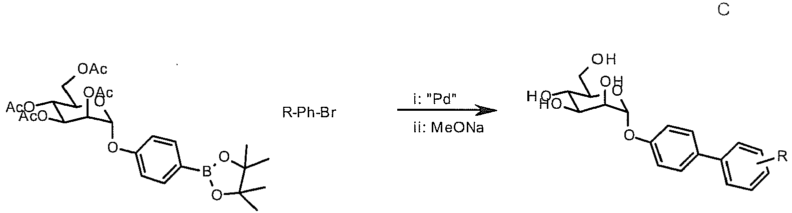

**6.1 [(2R,3S,4S,5R,6R)-4,5-diacetoxy-6-(acetoxymethyl)-2-[4-(4,4,5,5-tetramethyl-1,3,2-dioxaborolan-2-yl)phenoxy]tetrahydropyran-3-yl] acetate:** Under nitrogen atmosphere, the mixture of acetyl-protected 4-bromophenyl α-D-mannoside (2.791 g, 5.55 mmol), bis(pinacolato)diboron (1.690g, 6.66 mmol), potassium acetate (2.177 g, 22.18 mmol) and (1.1'-bis(diphenylphosohino)ferrocene)dichioropalladium(II) (0.244 g, 0.33 mmol) in DMSO (50 ml) was heated at 80 °C with stirring for 2.5 h. The solvent was removed and the resulting residue was purified by silica gel chromatography with hexane/ethyl acetate combinations as eluent to give [(2R,3S,4S,5R,6R)-4,5-diacetoxy-6-(acetoxymethyl)-2-[4-(4,4,5,5-tetramethyl-1,3,2-dioxaborolan-2-yl)phenoxy]tetrahydropyran-3-yl] acetate (2.48 g) in 81% yield. ¹H NMR (300 MHz, CHLOROFORM-*d*) δ ppm 1.33 (s, 12 H) 1.98 - 2.12 (m, 9 H) 2.20 (s, 3 H) 3.93 - 4.19 (m, 2 H) 4.21 - 4.36 (m, 1 H) 5.32 - 5.42 (m, 1 H) 5.45 (dd, *J*=3.57, 1.92 Hz, 1 H) 5.51 - 5.62 (m, 2 H) 7.00 - 7.15 (m, 2 H) 7.67 - 7.84 (m, 2 H). MS (ESI): found: [M + Na]⁺, 573.2.

**6.2 Methyl 5-[4-[(2R,3S,4S,5S,6R)-3,4,5-trihydroxy-6-(hydroxymethyl)tetrahydropyran-2-yl]oxyphenyl]pyridine-3-carboxylate (77):** Under nitrogen atmosphere, the mixture of [(2R,3S,4S,5R,6R)-4,5-diacetoxy-6-(acetoxymethyl)-2-[4-(4,4,5,5-tetramethyl-1,3,2-dioxaborolan-2-yl)phenoxy]tetrahydropyran-3-yl] acetate (0.132 g, 0.24 mmol), methyl 5-bromonicotinate (0.043g, 0.2 mmol), cesium carbonate (0.196 g, 0.6 mmol) and tetrakis(triphenylphosphine)palladium (0.023 g, 0.02 mmol) in dioxane/water (5 mL/1 mL) was heated at 80 °C with stirring for 1 h. After cooling down, the mixture was filtered through silica gel column to remove the metal catalyst and salts with hexane/ethyl acetate (2/1) containing 2% triethylamine as eluent. The filtrate was concentrated, and then dried in vacuo. Into the residue, 6 mL of methanol with catalytic amount of sodium methoxide (0.02 M) was added and the mixture was stirred at room temperature overnight. H⁺ exchange resin (DOWEX 50WX4-100) was added to neutralize the mixture. The resin was filtered off and the filtrate was concentrated. The resulting residue was purified by silica gel chromatography with CH₂Cl₂/MeOH combinations containing 2% NH₃/H₂O as eluent, giving rise to **77** (0.031 g) in 40% yield. ¹H NMR(300 MHz, CD₃OD) δ ppm 3.53 - 3.65 (m, 1 H) 3.67 - 3.83 (m, 3 H) 3.89 - 3.96 (m, 1 H) 3.99 (s, 3 H) 4.04 (dd, J=3.43, 1.79 Hz, 1 H) 5.57 (d, J=1.92 Hz, 1 H) 7.22 - 7.37 (m, 2 H) 7.58 - 7.73 (m, 2 H) 8.54 (t, J=2.06 Hz, 1 H) 8.97 (d, J=2.20 Hz, 1 H) 9.04 (d, J=1.92 Hz, 1 H). MS (ESI): found: [M + H]⁺, 392.1. (see Table 6)

### 7. Procedure for the preparation of mannosides via amide coupling reaction:

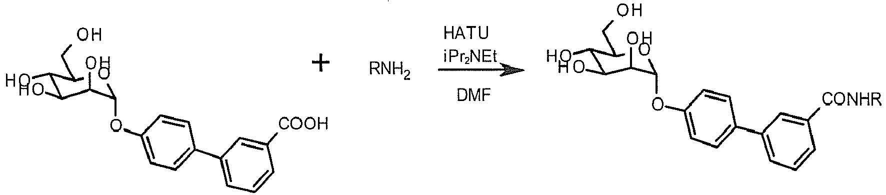

**3-[4-[(2R,3S,4S,5S,6R)-3,4,5-trihydroxy-6-(hydroxymethyl)tetrahydropyran-2-yl]oxyphenyl]-N-[2-[2-[2-[[3-[4-[(2R,3S,4S,5S,6R)-3,4,5-trihydroxy-6-(hydroxymethyl)tetrahydropyran-2-yl]oxyphenyl]benzoyl]amino]ethoxy]ethoxy]ethyl]benzamide (82):** Under nitrogen atmosphere, at 0 °C anhydrous DMF (5 mL) was added into the RB flask containing **44** (0.062 g, 0.165 mmol) and HATU (0.069 g, 0.182 mmol). After stirring for 10 min, 1,2-bis(2-aminoethoxy)ethane (0.0123 g, 0.083 mmol), then *N,N*-diisopropylethylamine (0.024 g, 0.182 mmol) were added. The mixture was stirred overnight while being warmed to rt naturally. The solvent was removed and the residue was purified by HPLC (C18, 15*150 mm column; eluent: acetonitrile/water (0.1% TFA)) to give **82**(0.044 g) in 82% yield. ¹H NMR (300 MHz, METHANOL-*d*₄) δ ppm 3.51 - 3.65 (m, 6 H), 3.65 - 3.82 (m, 14 H), 3.88 - 3.96 (m, 2 H), 4.03 (dd, *J*=3.57, 1.92 Hz, 2 H), 5.52 (d, *J*=1.65 Hz, 2 H), 7.14 - 7.24 (m, 4 H), 7.40 - 7.50 (m, 2 H), 7.53 - 7.63 (m, 4 H), 7.66 - 7.77 (m, 4 H), 8.00 (t, *J*=1.65 Hz, 2 H). MS (ESI): found: [M + H]⁺, 865.9. (see Table 7)

### 8. Procedures for the preparation of fluorescent mannoside(99 and 100):

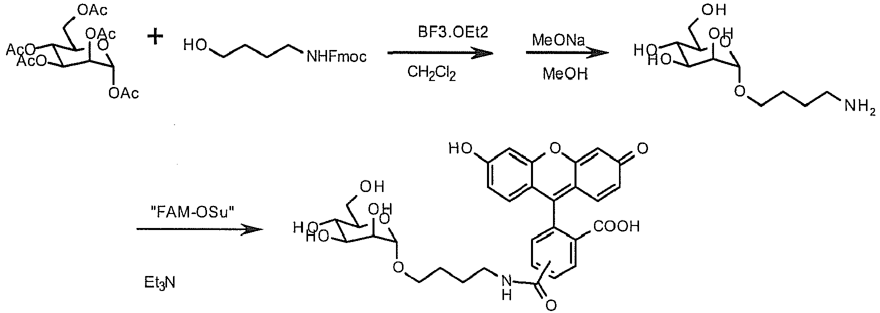

**8.1 [(2S,3S,4S,5R,6R)-4,5-diacetoxy-6-(acetoxymethyl)-2-[4-(9H-fluoren-9-ylmethoxycarbonylamino)butoxy]tetrahydropyran-3-yl] acetate:** Under nitrogen atmosphere, at 0 °C boron trifluoride diethyl etherate (0.115 g, 0.81 mmol) was added dropwise into the solution of α-D-mannose pentaacetate (0.107 g, 0.27mmol) and 4-(Fmoc-amino)-1-butanol (0.168 g, 0.54 mmol) in 5 ml of CH₂Cl₂. After a few mins the mixture was heated to reflux and kept stirring for more than 36 hrs. The reaction was then quenched with water and extracted with CH₂Cl₂. The CH₂Cl₂ layer was collected, then dried with Na₂SO₄, filtered, and concentrated. The resulting residue was purified by silica gel chromatography with hexane/ethyl acetate combinations as eluent to give [(2S,3S,4S,5R,6R)-4,5-diacetoxy-6-(acetoxymethyl)-2-[4-(9H-fluoren-9-ylmethoxycarbonylamino)butoxy]tetrahydropyran-3-yl] acetate (0.106 g) in 60% yield. ¹H NMR(300 MHz, CDCl₃) δ ppm 1.48∼1.79 (m, 4H), 2.01 (s, 3H), 2.05 (s, 3H), 2.11 (s, 3H), 2.17 (s, 3H), 3.25 (m, 2H), 3.50 (m, 1H), 3.73 (m, 1H), 3.91∼4.04 (m, 1H), 4.05∼4.18 (m, 1H), 4.18∼4.37 (m, 2H), 4.41 (d, *J* = 6.87 Hz, 2H), 4.74∼4.94 (m, 2H), 5.17∼5.41 (m, 3H), 7.29∼7.37 (m, 2H), 7.37∼7.47 (m, 2H), 7.61 (d, *J* = 7.42 Hz, 2H), 7.78 (d, *J* = 7.42 Hz, 2H). MS (ESI): found: [M+H]⁺, 642.2.

**8.2 (2S,3S,4S,5S,6R)-2-(4-aminobutoxy)-6-(hydroxymethyl)tetrahydropyran-3,4,5-triol (98):** [(2S,3S,4S,5R,6R)-4,5-diacetoxy-6-(acetoxymethyl)-2-[4-(9H-fluoren-9-ylmethoxycarbonylamino)butoxy]tetrahydropyran-3-yl] acetate (0.106 g, 0.165 mmol) was stirred in 6 mL of methanol with catalytic amount of sodium methoxide (0.02M) at rt overnight. The solvent was removed and the residue was purified by silica gel chromatography with combination of ammonia aqueous/methanol as eluent to afford **98** (0.036 g) in 88% yield. ¹H NMR(300 MHz, CD₃OD) δ ppm 1.46∼1.78 (m, 4H), 2.61∼2.88 (m, 2H), 3.41∼3.55 (m, 2H), 3.59 (t, *J* = 9.3 Hz, 1H), 3.65∼3.87 (m, 5H), 4.75 (d, *J* = 1.65 Hz, 1H). MS (ESI): found: [M+H]⁺, 252.1.

**8.3Fluorescent mannosides(99** and **100):** The mixture of **98** (0.018 g, 0.070 mmol), 5-(and -6)-carboxyfluoresceinsuccinimidyl ester (0.022 g, 0.046 mmol) and triethylamine (0.058 g, 0.57 mmol) in DMF (2 mL) was stirred overnight. After removing the solvent, the residue was purified by silica gel chromatography with dichloromethane/methanol combination as eluent, giving rise to **99** and **100** (0.023 g) in 82% yield. MS (ESI): found: [M + H]⁺, 610.6. (see Table 8)

### 9. Procedures for the preparation of (2R,3S,4S,5S,6S)-2-(hydroxymethyl)-6-(4-phenyltriazol-1-yl)tetrahydropyran-3,4,5-triol (101):

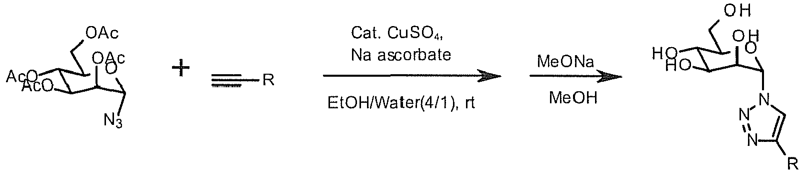

**9.1 [(2S,3S,4S,5R,6R)-4,5-diacetoxy-6-(acetoxymethyl)-2-(4-phenyltriazol-1-yl)tetrahydropyran-3-yl] acetate:** Under nitrogen atmosphere, EtOH/H₂O (4 mL/1mL) was added into the RB flask containing α-azido-D-mannose tetraacetate (0.075 g, 0.2 mmol), phenyl acetylene (0.026 g, 0.24 mmol), copper (II) sulfate pentahydrate (0.01 g, 0.04 mmol) and sodium ascorbate (0.016 g, 0.08 mmol). The mixture was stirred at room temperature overnight. The solvent was removed and the residue was purified by silica gel chromatography with hexane/ethyl acetate combination as eluent, giving rise to [(2S,3S,4S,5R,6R)-4,5-diacetoxy-6-(acetoxymethyl)-2-(4-phenyltriazol-1-yl)tetrahydropyran-3-yl] acetate (0.030 g) in 31% yield. ¹H NMR (300 MHz, CHLOROFORM-*d*) δ ppm 2.05 - 2.12 (m, 9 H) 2.20 (s, 3 H) 3.89 - 3.99 (m, 1 H) 4.08 (dd, *J*=12.50, 2.61 Hz, 1 H) 4.40 (dd, *J*=12.50, 5.36 Hz, 1 H) 5.40 (t, *J*=8.79 Hz, 1 H) 5.95 - 6.04 (m, 2 H) 6.07 (d, *J*=2.75 Hz, 1 H) 7.34 - 7.53 (m, 3 H) 7.79 - 7.92 (m, 2 H) 7.96 (s, 1 H). MS (ESI): found: [M + Na]⁺, 498.1.

**9.2 (2R,3S,4S,5S,6S)-2-(hydroxymethyl)-6-(4-phenyltriazol-1-yl)tetrahydropyran-3,4,5-triol (101):** [(2S,3S,4S,5R,6R)-4,5-diacetoxy-6-(acetoxymethyl)-2-(4-phenyltriazol-1-yl)tetrahydropyran-3-yl] acetate (0.028 g, 0.059 mmol) was stirred in 6 mL of methanol with catalytic amount of sodium methoxide (0.02 M) at room temperature overnight. H⁺ exchange resin (DOWEX 50WX4-100) was added to neutralize the mixture. The resin was filtered off and the filtrate was concentrated, then dried in vacuo giving rise to pure product **101** (0.015 g) in quantitative yield. ¹H NMR(300 MHz, CD₃OD) δ ppm 3.34 - 3.44 (m, 1 H) 3.70 - 3.92 (m, 3 H) 4.12 (dd, J=8.52, 3.57 Hz, 1 H) 4.71 - 4.80 (m, 1 H) 6.08 (d, J=2.75 Hz, 1 H) 7.30 - 7.41 (m, 1 H) 7.41 - 7.51 (m, 2 H) 7.77 - 7.90 (m, 2 H) 8.51 (s, 1 H). (ESI): found: [M + Na]⁺, 330.2. (see Table 9)

### 10. Procedures for the preparation of Methyl 3-[4-[[(2S,3S,4S,5S,6R)-3,4,5-trihydroxy-6-(hydroxymethyl)tetrahydropyran-2-yl]amino]phenyl]benzoate (102):

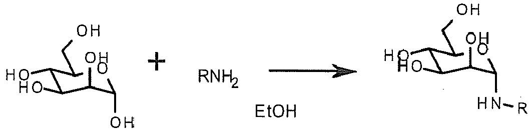

α-D-mannose (0.360 g, 2 mmol) and methyl 4'-aminobiphenyl-3-carboxylate (0.454 g, 2 mmol) in ethanol (5 mL) was heated to 55 °C for 17 h. After cooling down to rt, the white precipitate formed was collected by filtration. The precipitate was washed with ethanol (3 mL) two times, then dried in vacuo to afford pure **102** in 77% yield. ¹H NMR (300 MHz, ACETONITRILE-d3 and D20) δ ppm 3.28 - 3.38 (m, 1 H) 3.54 - 3.59 (m, 2 H) 3.64 - 3.71 (m, 2 H) 3.86 (s, 3 H) 3.88 - 3.92 (m, 1 H) 4.89 (d, J=1.10 Hz, 1 H) 6.80 - 6.91 (m, 2 H) 7.44 - 7.58 (m, 3 H) 7.75 - 7.90 (m, 2 H) 8.11 - 8.20 (m, 1 H). (ESI): found: [M + H]⁺, 390.1.

### 11. Procedures for the preparation ofmethyl 3-[4-[(2R,3S,4S,5S,6R)-3,4,5-trihydroxy-6-(hydroxymethyl)tetrahydropyran-2-yl]sulfanylphenyl]benzoate (103):

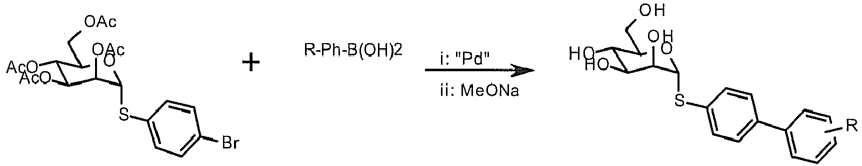

**11.1 [(2R,3S,4S,5R,6R)-4,5-Diacetoxy-6-(acetoxymethyl)-2-(4-bromophenyl)sulfanyl-tetrahydropyran-3-yl] acetate:** Under nitrogen atmosphere, at 0 °C boron trifluoride diethyl etherate (0.427 g, 3.0mmol) was added dropwise into the solution of α-D-mannose pentaacetate (0.390 g, 1.0mmol) and 4-bromobenzenethiol (0.378 g, 2.0mmol) in 6 ml of CH₂Cl₂. After a few mins the mixture was warmed to rt and kept stirring for 48 hrs. The reaction was then quenched with water and extracted with CH₂Cl₂. The CH₂Cl₂ layer was collected, dried with Na₂SO₄, filtered, and concentrated. The resulting residue was purified by silica gel chromatography with hexane/ethyl acetate combinations as eluent, giving rise to [(2R,3S,4S,5R,6R)-4,5-diacetoxy-6-(acetoxymethyl)-2-(4-bromophenyl)sulfanyl-tetrahydropyran-3-yl] acetate (0.40 g) in 77% yield. ¹H NMR (300 MHz, CHLOROFORM-*d*) δ ppm 2.01 - 2.10 (m, 9 H) 2.16 (s, 3 H) 4.10 (dd, *J*=12.09, 2.47 Hz, 1 H) 4.30 (dd, *J*=12.09, 6.04 Hz, 1 H) 4.43 - 4.61 (m, 1 H) 5.24 - 5.40 (m, 2 H) 5.43 - 5.52 (m, 2 H) 7.32 - 7.39 (m, 2 H) 7.42 - 7.49 (m, 2 H). MS (ESI): found: [2M + H]⁺, 1039.1.

### 11.2 Methyl 3-[4-[(2R,3S,4S,5S,6R)-3,4,5-trihydroxy-6-(hydroxymethyl)tetrahydropyran-2-yl]sulfanylphenyl]benzoate (103):

Under nitrogen atmosphere, the mixture of [(2R,3S,4S,5R,6R)-4,5-diacetoxy-6-(acetoxymethyl)-2-(4-bromophenyl)sulfanyl-tetrahydropyran-3-yl] acetate (0.20 g, 0.39 mmol), 3-methoxycarbonylphenylboronic acid (0.106 g, 0.59 mmol), cesium carbonate (0.381 g, 1.17 mmol) and tetrakis(triphenylphosphine)palladium (0.045 g, 0.04 mmol) in dioxane/water (5 mL/1 mL) was heated at 80 °C with stirring for 1 h. After cooling down, the mixture was filtered through silica gel column to remove the metal catalyst and salts with hexane/ethyl acetate (2/1) as eluent. The filtrate was concentrated, and then dried in vacuo. Into the residue, 6 mL of methanol with catalytic amount of sodium methoxide (0.02 M) was added and the mixture was stirred at room temperature overnight. H⁺ exchange resin (DOWEX 50WX4-100) was added to neutralize the mixture. The resin was filtered off and the filtrate was concentrated. The resulting residue was purified by HPLC (C18, 15*150 mm column; eluent: acetonitrile/water (0.1% TFA)) to give **103** (0.095 g) in 63% yield. ¹H NMR(300 MHz, CD₃OD) δ ppm 3.66 - 3.91 (m, 4 H) 3.94 (s, 3 H) 4.01 - 4.16 (m, 2 H) 5.51 (d, J=1.37 Hz, 1 H) 7.51 - 7.69 (m, 5 H) 7.81 - 7.93 (m, 1 H) 8.00 (dt, J=7.83, 1.44 Hz, 1 H) 8.24 (t, J=1.65 Hz, 1 H). MS (ESI): found: [M + H]⁺, 407.1.

### 12. Procedures for the preparation ofmethyl 3-[4-[[(2S,3S,4S,5S,6R)-3,4,5-trihydroxy-6-(hydroxymethyl)tetrahydropyran-2-carbonyl]amino]phenyl]benzoate (104):

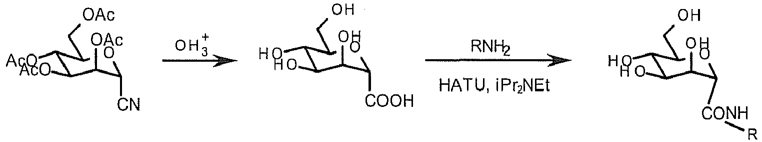

The solution of α-cyano-D-mannose tetraacetate (0.107g, 0.3 mmol) in 25% hydrochloric acid was heated at 50 °C for 48 h. The solvent was removed. Water (10 ml) and H⁺ exchange resin (DOWEX 50WX4-100) was added and kept stirring for 5 mins. The resin was filtered off and the filtrate was concentrated, and then dried in vacuo. Into the resulting residue, HATU (0.274 g, 0.72 mmol) and anhydrous DMF (6 ml) were added at 0 °C. After stirring for 10 min, methyl 4'-aminobiphenyl-3-carboxylate (0.164 g, 0.72 mmol), then *N,N*-diisopropylethylamine (0.233 g, 1.80 mmol) were added. The mixture was stirred overnight while being warmed to rt naturally. The solvent was removed and the residue was purified by silica gel chromatography with CH₂Cl₂/MeOH combinations as eluent to give **104** (0.066 g) in 52% yield. ¹H NMR(300 MHz, CD₃OD) δ ppm 3.50 (dt, J=4.67, 2.33 Hz, 1 H) 3.58 - 3.65 (m, 2 H) 3.76 (dd, J=11.81, 7.42 Hz, 1 H) 3.86 - 4.03 (m, 4 H) 4.52 (t, J=2.61 Hz, 1 H) 4.57 (d, J=2.75 Hz, 1 H) 7.55 (t, J=7.69 Hz, 1 H) 7.60 - 7.68 (m, 2 H) 7.68 - 7.76 (m, 2 H) 7.82 - 7.91 (m, 1 H) 7.97 (dt, J=7.69, 1.37 Hz, 1 H) 8.21 - 8.28 (m, 1 H). MS (ESI): found: [M + H]⁺, 418.1.

### Example 6. Structure-Based Drug Design and Optimization of Mannoside Bacterial FimH Antagonists.

As shown in **Scheme 1,** α-D-mannoside derivatives were prepared using traditional Lewis acid mediated glycosidation.³Reaction of acylated α-D-(+)-mannose **1a** with a variety of phenols and BF₃-OEt₂ resulted in exclusive formation of the α-isomer mannosides **2.** Subsequent deacylation with NaOMe in methanol gave the desired aryl mannosides **3-8** in good yield. Biological activity against FimH was evaluated using a guinea pig red blood cell-based hemagglutination (HA) assay (ref) in which the concentration of the mannoside on reducing agglutination by 50% was used as the primary endpoint (50% HA titer). This assay was preferred to a simple FimH binding assay for screening and developing SAR since it assesses the compound's ability to prevent bacterially-mediated adhesion directly in a cellular assay.

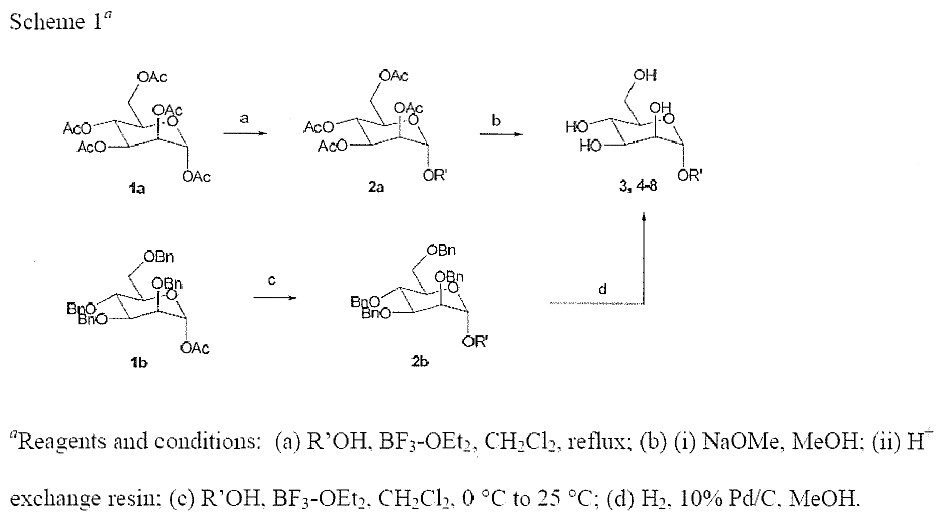

As shown in **Table 10,** it was found that the general trend that 2- and 3-substitution was optimal for potency relative to 4-substitution in most examples with the exception of the acyl anilines **3q-s** in which this trend was dramatically reversed. Ortho-substituted chlorophenyl **3g,** cyanophenyl **3m,** and meta-substituted methyl ester **3j** all showed an impressive greater than 5-fold improvement in potency relative to parent phenyl mannoside **3a.** Interestingly, carboxylic acid **3l** lost 10-fold potency relative to matched pair methyl ester **3j** showing a 50% HA titer of only 60 µM. Incorporation of an additional methyl ester in the 5-position of **3j**, as with compound **4,** resulted in a relatively large 3-fold enhancement in potency. Benzylic analogs **5a** and **5b** (pictured below) which have different conformational space and flexibility relative to direct phenyl substitution of the anomeric oxygen, as with to matched pairs **3a** and **3b,** show a 2-fold decrease (50% HA titer = 60 µM) in potency.

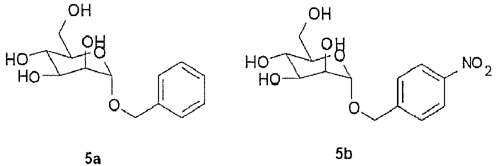

| **Table 10. SAR of simple aryl substitution mannoside library.** | | | | | |
|---|---|---|---|---|---|
| 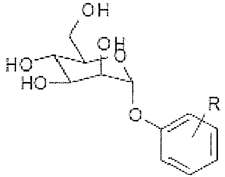 | | | | | |

| **Compound** | **R** | **HA 50% Titer (µM)** | **Compound** | **R** | **HA 50% Titer (µM)** |
|---|---|---|---|---|---|
| **3a** | H | 30 | **3m** | 2-CN | 6 |
| **3b** | 4-NO₂ | 31 | **3n** | 3-CN | 23 |
| **3c** | 3-NO₂ | 16 | **3o** | 4-CN | 30 |
| **3d** | 4-NH₂ | 32 | **3p** | 4-OMe | 8 |
| **3e** | 3-Me | 4 | **3q** | 2-NHAc | 125 |
| **3f** | 4-Me | 8 | **3r** | 3-NHAc | 12 |
| **3g** | 2-Cl | 4 | **3s** | 4-NHAc | 8 |
| **3h** | 3-Cl | 8 | **3t** | 3-CONH₂ | 16 |
| **3i** | 4-Cl | 32 | **3u** | 4-CONH₂ | 15 |
| **3j** | 3-CO₂Me | 8 | **3w** | 4-CH₂CO₂Me | 30 |
| **3l** | 3-CO₂H | 60 | **4** | 3,5-CO₂Me | 2 |

Upon examination of the X-ray structures of α-D-butylmannoside and oligomannose-3 coupled with docking studies of the monophenyl inhibitors bound to FimH, it was encouraging to observe that additional improvements in binding affinity could be achieved by addition of a second aryl or aliphatic ring system in anticipation of introducing both additional hydrophobic and π-π stacking interactions with Tyr-48 and Tyr-137 as seen from the directionality of the butyl side chain in butylmannoside and position of the second mannose residue in oligomannose-3.

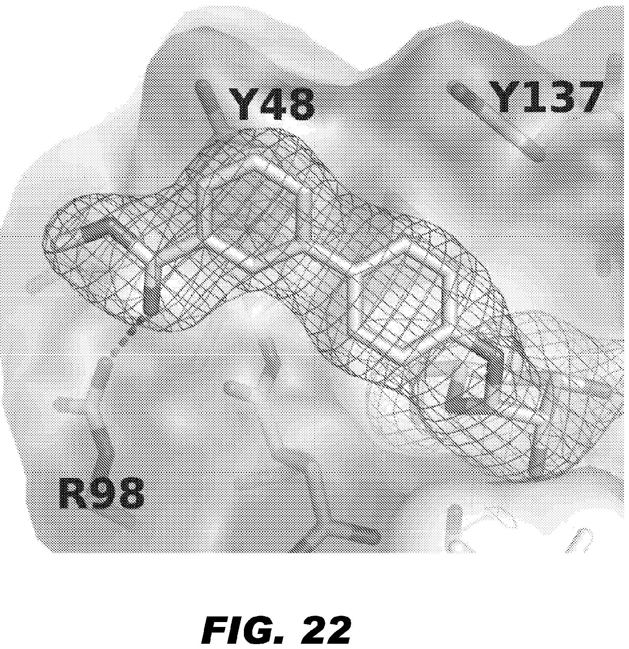

To this end, analogs with an additional ring system either directly attached or fused ring to the parent aryl mannosides of **Table 10** were explored. It was discovered that a variety of ring systems were tolerated (**Table 11**) relative to the previously described coumarin analog 4-methylumbellferyl-α-D-mannoside **6.** The most attractive inhibitor in this series of compounds was the 4'-biphenyl derivative **8e** bearing a methyl ester off the meta position of the second aryl ring and showing a 2-fold improvement relative to **6** displaying an HA titer 1 µM. In order to determine the source of this potency enhancement and aid in further improvements, a high-resolution X-ray crystal structure of **8e** bound to FimH was obtained. Shown in **Figure 22****,** inhibitor **8e** binds in a very complementary "lock and key" fashion to the FimH binding pocket. The mannose ring is making conserved interactions with the mannose-binding pocket similar to those described previously while the two aromatic rings of the biphenyl moiety exist in a non-planar conformation allowing for a π-stacking and hydrophobic interactions with Tyr-48 and other residues encompassing the exterior hydrophobic cleft of FimH. The π-stacking interaction with Tyr 48 occurs on the opposite side of the phenyl ring than that engaged by oligomannose-3, which inserts itself into the open "tyrosine gate" formed by Tyr-48 and Tyr-137. Thus, **8e** engages the "tyrosine gate" in a way that results in alteration of the extended FimH binding pocket through closure of the tyrosine gate upon rotation of Tyr-48. This binding mode places the ester of **8e** within H-bonding distance to a salt bridge formed between Arg-98 and Glu-50 (**Figure 22**). It is noteworthy that the HA 50% titer for Methylαman is >1 mM making compound **8e** greater than 1000-fold more potent from the addition of the biphenyl ester.

| **Table 11. SAR of multi-ring system analogs.** | | | | | |
|---|---|---|---|---|---|
| 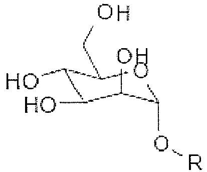 | | | | | |

| Compound | R | HA 50% titer EC₉₀ (µM) | Compound | R | HA 50% titer EC₉₀ (µM) |
|---|---|---|---|---|---|
| **6** | 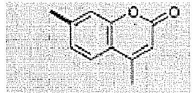 | 2 | **8b** | 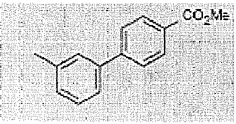 | 6 |
| **7a** | 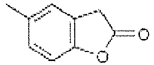 | 8 | **8c** | 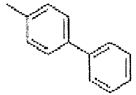 | 8 |
| **7b** | 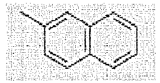 | 6 | **8d** | 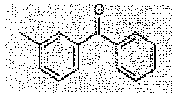 | 8 |
| **7c** | 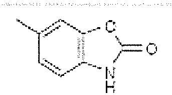 | 4 | **8e** | 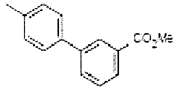 | 1 |
| **7d** | 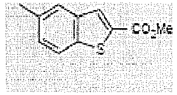 | 2 | **8f** | 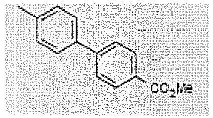 | 4 |
| **7e** | 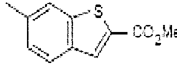 | 2 | **8g** | 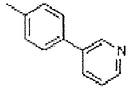 | 2 |
| **8a** | 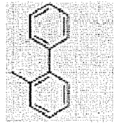 | 62 | **8h** | 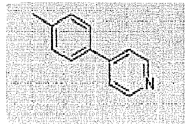 | 2 |

This observation lead to investigating replacing the mannose with alternate sugars or mimics since the biphenyl portion alone provides a significant contribution to biological activity presumably from tighter binding to FimH. It was decided to start by replacing the mannose portion of **8e** with glucose since all chiral centers are identical except for the 3-hydroxyl group adjacent to the anomeric center, being of R stereochemistry in mannose and S in glucose (**Scheme 2**). Standard Lewis acid mediated glycosidation gave isomeric mixtures at the anomeric center favoring the undesired β-isomer, conversely to the clean formation of the α-isomer seen for the mannosides. Correspondingly, reaction of benzoyl protected α-D-glucose **9** with phenol **10** using BF₃-OEt₂ followed debenzoylation yielded both the α-D glucoside **11a** and β-D-glucoside **11b** in a 3:7 ratio. Unfortunately, neither glucoside isomer showed activity in the hemagglutination assay even up to a concentration of 2.5 mM. Therefore, modification of a single chiral hydroxyl group stereocenter on the mannoside is sufficient to significantly decrease biological activity, presumably resulting from the inability to tightly bind FimH. Upon analysis of the specific interactions realized from α-D-mannose bound to FimH,⁴ this hydroxyl group is making an H-bonding interaction with the N-terminal residue of FimH and a water molecule contained inside the binding pocket. In any event, this finding demonstrates the exquisite specificity of FimH for mannose epitopes which has enabled UPEC bacteria to exclusively recognize mannose-presenting cells.

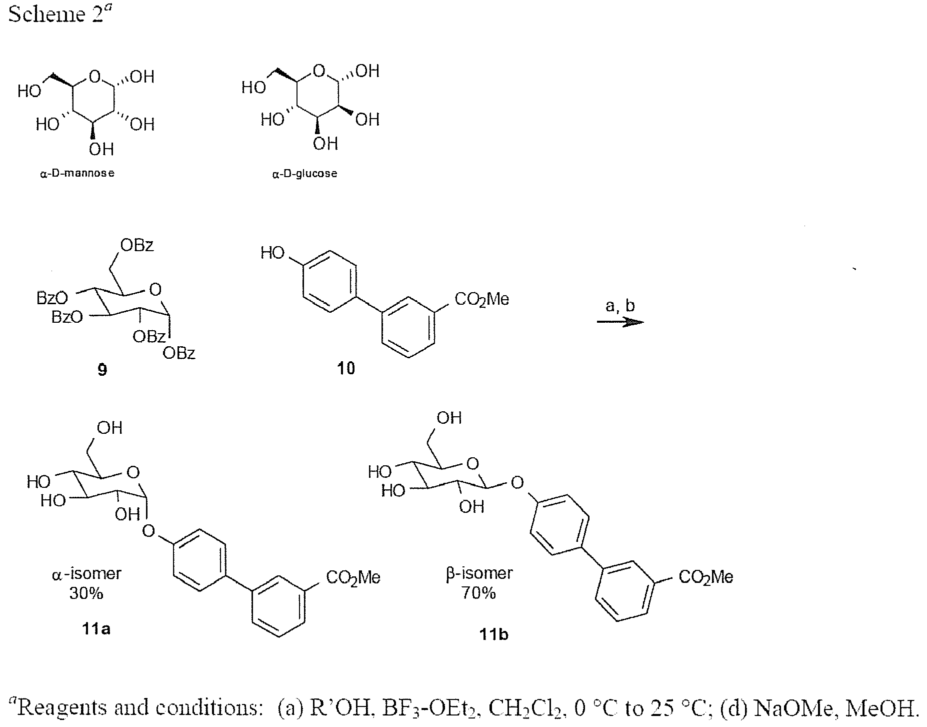

Next, the biaryl portion of the mannoside was optimized by undertaking an extensive SAR evaluation of the second ring through a modified Topliss-type evaluation of substituents. This focused set of compounds was designed with some bias toward improving interactions with Arg-98 and Glu-50 but also with interest in elucidating the importance of ring electronic properties as a means to improve the stacking interaction with Tyr-48. A few initial analogs were prepared using the synthetic route outlined previously in **Scheme 1** but a more convergent synthesis was developed based on Suzuki coupling of arylbromide intermediate **12** as shown in **Scheme 3.**

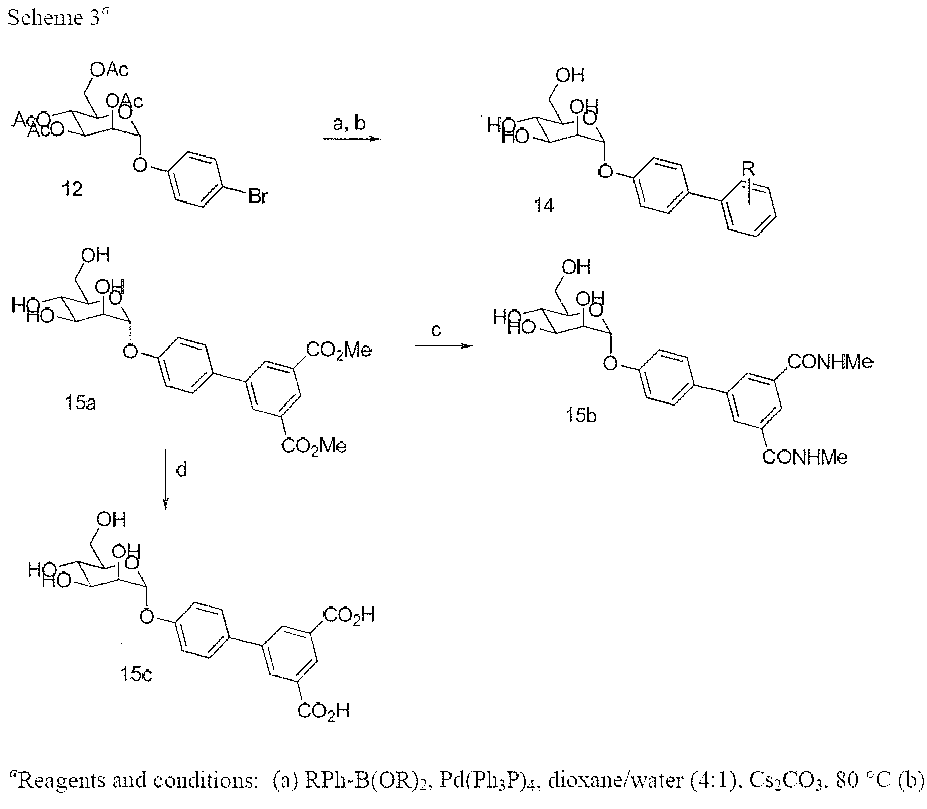

Employing standard Suzuki conditions by reaction of aryl boronic acids or esters with Pd(Ph₃P)₄ and Cs₂CO₃, **12** was converted to protected biphenyl mannosides **13** in good yield. The acylated biaryl mannosides **13** were then deprotected as before to generate the final target compounds **14** or **15a** displayed in **Table 12.** Di-ester **15a** was further functionalized to di-methyl amide **15b** by reaction with dimethylamine or converted to di-acid **15c** by basic hydrolysis in methanol. Upon evaluation of HA titers, the matched pair meta-substituted methyl amide **14a** was equipotent to ester **8e** while reverse amide **14b** and free acid **14c** displayed moderately lower potencies of 2 µM and 4 µM respectively. The latter result was surprising since these modifications were designed to introduce an electrostatic interaction with Arg-98 and disrupt the Arg-98/Glu-50 salt bridge. On the other hand, sulfonamide **14r** has equivalent potency to carboxamide **14a** providing further evidence that a hydrogen bond acceptor is required for optimal potency presumably from interaction with Arg-98. From analysis of ortho, meta, and para matched pairs, it was obvious that para-substitution was least preferred for activity while meta substitution was preferred to ortho substitution. This preference for meta substitution was most pronounced with the methyl alcohols **14g** and **14h** where the ortho analog **14g** was over 5-fold less potent with an HA titer of only 16 µM. It was surmised that this large substituent results in an increased rotation of the two rings out of the plane causing a non-productive alignment for interactions with Tyr-48. Although a majority of the analogs tested contained electron withdrawing groups, in general electron donating groups such as the methyl alcohols **14g-i** and phenols **14j-l** were less active in the HA titer assay. The most potent of the mono-substituted mannosides is meta nitro derivative **14m** with a 50% HA titer of 0.5 µM. It is possible that the partial negative charge on the nitro oxygen atoms allow for an optimized H-bond acceptor-donor interaction with Arg-98 coupled with the increased electron withdrawing ability of the nitro group possibly enhancing stacking interactions with Tyr-48.

| **Table 12. SAR of substituted biphenyl mannosides.** | | | | | |
|---|---|---|---|---|---|
| 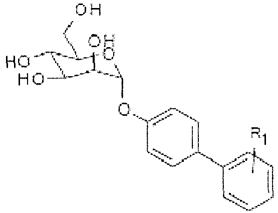 | | | | | |

| Compound | R₁ | HA 50% titer ECgo (µM) | Compound | R₁ | HA 50% titer EC₉₀ (µM) |
|---|---|---|---|---|---|
| **14a** | 3-CONHMe | 1 | **14o** | 2-NHSO₂Me | 12 |
| **14b** | 3-NHAc | 2 | **14p** | 3-NHSO₂Me | 2 |
| **14c** | 3-CO₂H | 4 | **14q** | 2-SO₂NHMe | 4 |
| **14d** | 2-CN | 2 | **14r** | 3-SO₂NHme | 1 |
| **14e** | 3-CN | 1 | **14s** | 3-NO₂ | 0.5 |
| **14f** | 4-CN | 8 | **14t** | 4-NO₂ | 2 |
| **14g** | 2-CH₂OH | 16 | **14u** | 2-CONH₂ | 6 |
| **14h** | 3-CH₂OH | 3 | **14v** | 3-CONH₂ | 2 |
| **14i** | 4-CH₂OH | 6 | **14w** | 2-CF₃ | 8 |
| **14j** | 2-OH | 8 | **14x** | 3-CF₃ | 2 |
| **14k** | 3-OH | 4 | **14y** | 3-F | 6 |
| **14l** | 4-OH | 6 | **15a** | 3,5-CO₂Me | 0.15 |
| **14m** | 2-OMe | 1 | **15b** | 3,5-CONHMe | 0.37 |
| **14n** | 3-OMe | 1.5 | **15c** | 3,5-CO₂H | 2 |

Perhaps the most unexpected finding from this study was that addition of another ester or amide substituted in the other meta position such as di-ester **15a** and di-methyl amide **15b** resulted in the two most potent mannoside inhibitors of FimH with activities of 150 nM and 370 nM, respectively. With respect to di-ester **15a** this constitutes a 7-fold improvement in activity relative to mono ester **8e.** The di-amide **15b,** while slightly less potent, has much improved solubility relative to di-ester **15a.** It was hypothesized that the addition of the second ester or amide serves a two-fold purpose for improving activity: First, the electron withdrawing group results in less electron density of the aryl ring thus improving π-π stacking interactions with Tyr48. Second, the mono-ester (or amide) analog can presumably access conformations in which the aryl ring is not in close proximity to Arg-98 whereas the meta di-ester (or di-amide) analog likely exists only in conformations where the ester or amide resides in close proximity to Arg-98 resulting in less entropic loss upon binding and a lower energy bound conformation resulting in increased binding affinity and potency.

In order to more accurately determine the relative contributions of both binding affinity to FimH versus other compound properties to the potency seen in the cellular HA titer assay, a high-throughput fluorescence polarization (FP) (or anisotropy) competitive binding assay was developed using a fluorescent-labeled mannoside ligand shown in **Scheme 4.** Synthesis was achieved as before using Lewis acid mediated glycosylation with protected aminoalcohol **17** followed by dual Fmoc and acyl deprotection of intermediate **18** to give free amine **19.** Subsequent reaction of 5(6)FAM-OSu with **19** using triethylamine in DMF gave the desired fluorescently tagged FAM-mannoside **20.**

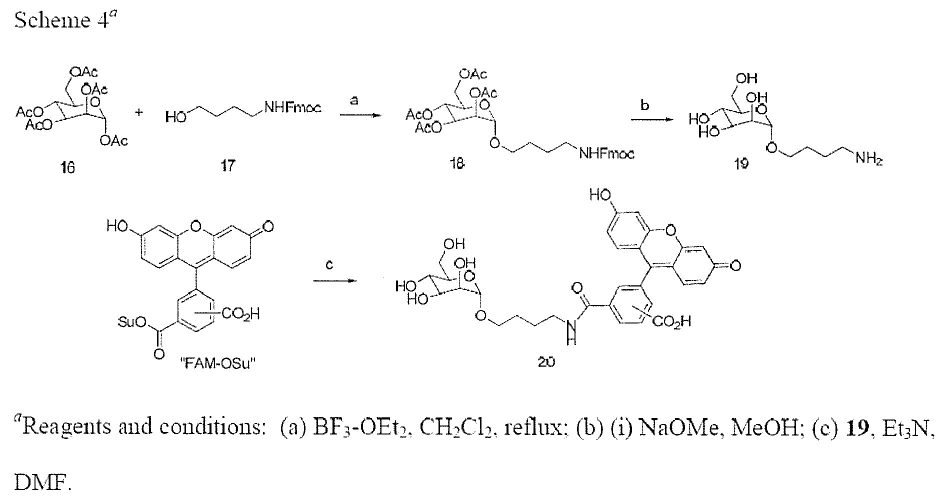

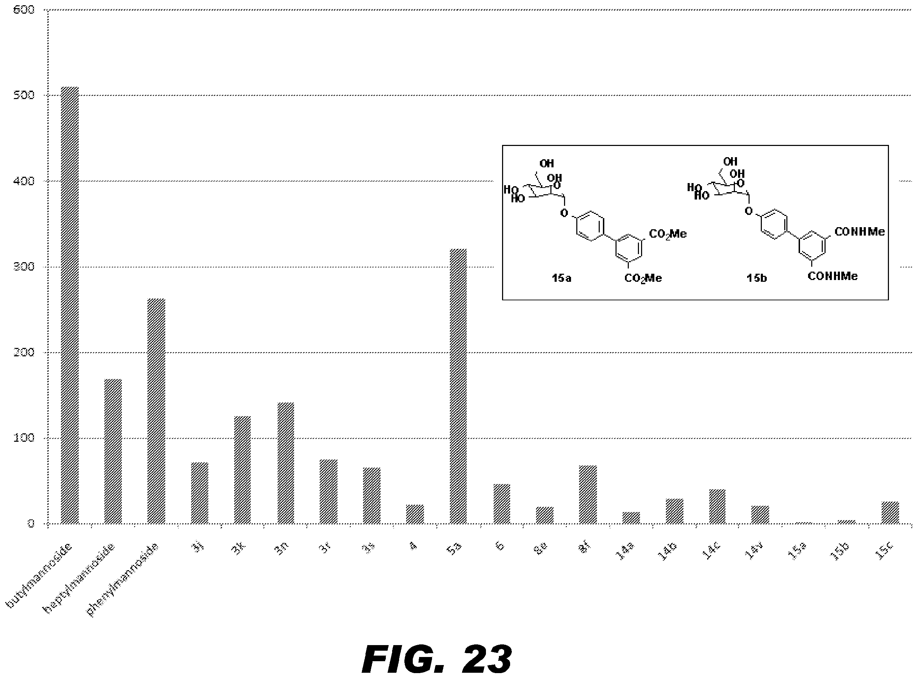

The K_{D} for FAM mannoside **20** was measured to be 0.17 µM while the 50% HA titer was only 125 µM. Twenty mannoside ligands with structural diversity and activities were evaluated in the HA titer cell assay ranging from 150 nM to 125 µM for their ability to competitively inhibit binding of FAM **20.** Interestingly, it was found that all potently competed for FimH binding having EC₅₀'s less than 0.25 µM but having no clear correlation of EC₅₀ with the activity found in the HA cell assay (**Table 13**). Although there was over a 60-fold range in cellular potency, there was only about a 5-fold range in binding affinity (**Fig. 23**). In addition, there was also no linear correlation of data with the two assays. Simple alkyl mannosides have the highest drop in cell activity as demonstrated with butylmannoside having the most dramatic difference with a 510-fold drop in cell activity relative to binding.

| **Table 13. Binding affinity of selected mannosides in fluorescence polarization assay.** | | | | | |
|---|---|---|---|---|---|
| Compound | FP Binding EC₅₀ (µM) | HA 50% titer (µM) | Compound | FP Binding EC₅₀ (µM) | HA 50% titer (µM) |
| **6 (MeUmbαman)** | 0.044 | 2 | **Heptylαman** | 0.089 | 15 |
| **8e** | 0.052 | 1 | **4** | 0.091 | 2 |
| **8f** | 0.059 | 4 | **14p** | 0.097 | 2 |
| **3k** | 0.064 | 8 | **14c** | 0.099 | 4 |
| **14b** | 0.07 | 2 | **3a** | 0.114 | 30 |
| **14a** | 0.075 | 1 | **3s** | 0.122 | 8 |
| **15a** | 0.077 | 0.15 | **3r** | 0.160 | 12 |
| **14v** | 0.078 | 2 | **3n** | 0.162 | 23 |
| **3j** | 0.084 | 6 | **5a** | 0.187 | 60 |
| **15b** | 0.087 | 0.37 | **Butylαman** | 0.245 | 125 |

Interestingly, the most potent biphenyl mannoside series shows a modest correlation of binding affinity having a much smaller drop in cell activity relative to binding. The di-ester **15a** and di-amide **15b** show the best correlation with only a 2-fold and 4-fold difference in cell potency vs. binding. It is important to note that even though these two analogs are the most potent in the HA titer cell assay, they have about 2-fold less binding affinity in the FP assay than the tightest binding mannosides. While it is unclear why there is no correlation of binding to activity in the cell, there is a moderate correlation when compounds with binned affinities are compared. However, the latter observation cannot explain the non-linear increase in cell activity of **15a** and **15b** relative to the mono-substituted biphenyl mannosides. Perhaps differential binding kinetics including varied on rates and off rates of mannoside analogs to FimH can provide a plausible explanation for the latter. It is worth mentioning that the previously reported K_{D} values obtained through surface plasmon resonance (SPR) for **6** (MeUmbaman), butylmannoside, and Heptylαman are 20 nM, 151 nM, and 5 nM respectively. While the result for butylmannoside is similar, the FP assay suggests MeUmbαman is the most potent (44 nM) being 2-fold more potent than Heptylαman (89 nM). Considering both the lack of correlation between binding affinity and biological activity in the cell plus the variability seen comparing two separate binding assays, the HA titer assay coupled with the use of structural information is a preferred route for medicinal chemistry optimization of mannoside FimH ligands. Nonetheless, based on the HA titer cellular data, mannoside **15a** is the most potent FimH antagonist reported to date and represents an excellent lead candidate for further optimization of the monovalent mannosides toward a novel preclinical candidate with tremendous therapeutic potential for treating urinary tract infections.

The development of multivalent and dendrimeric mannosides has been the major focus of previous work on FimH antagonists since simple mannosides, while having respectable binding affinity to FimH, show poor cellular activity in the HA assay. It has been demonstrated that multivalent mannosides are effective at increasing avidity through a phenomenon termed "cluster effect" resulting in an overall increased binding affinity when calculated per mannoside monomer. Therefore, it was of interest to determine if the improved monovalent mannosides **15a** and **15b** would produce a similar increase in avidity or cluster effect previously reported for dendrimeric mannosides. For synthetic simplicity, a model system based on monoamide **14a** was chosen. A symmetrical divalent mannoside was designed by connecting two monomers via an amide based linker to the two biphenyl rings (**Scheme 5**). Accordingly, dimeric inhibitors based on **14a** were synthesized by coupling carboxylic acid **13c** to either diamine 2-(2-aminoethoxy)ethanamine or 2-[2-(2-aminoethoxy)ethoxy]ethanamine yielding diamides **21** and **22** respectively using standard HATU coupling conditions with Hunig's base in DMF. The shorter chain analog **21** showed a 50% HA titer of 0.75 µM thus showing no noticeable improvement relative to **14a,** while the longer ethylene glycol linked diamide **22** displayed an almost 8-fold increase in activity with an impressive HA titer of 130 nM. This constitutes a 4-fold increase in avidity relative to the expected potency of 500 nM based on two monomeric units of **14a.** In addition to increased potency, **22** has much improved solubility relative to **21** making it an ideal starting point for further optimization. Although these analogs have less potential to be effective as oral agents relative to the monovalent mannosides, the divalent mannosides are very useful chemical research tools and can potentially be developed into topical or intravenously dosed antibacterial agents.

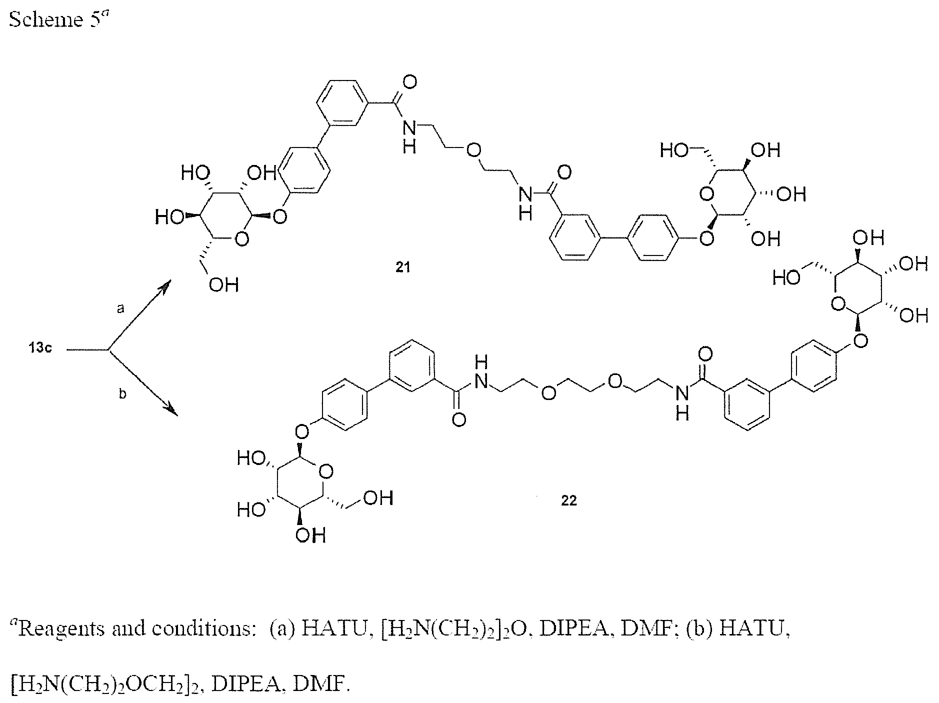

### Example 7. In vitro analysis of mannoside inhibitors.

Three FimH function assays were used to screen mannosides for inhibitory function, and are described below. Selected results for the SAR and testing of these compounds are presented in **Examples 5 and 6,** and show that some synthesized and tested compounds are more potent inhibitors of HA titer and biofilm formation than anything commercially available and previously tested, some by as much as 2 logs.
i. Hemagglutination of guinea pig red blood cells by type1 piliated UPEC is dependent upon FimH mannose binding ability. Serial dilutions allow a quantitative analysis using this assay.
ii. Compounds that decrease hemagglutination by 75% were then tested in a type 1 dependent biofilm assay. First, the ability of mannoside derivatives to prevent UPEC type 1 dependent biofilm formation in the published Kolter assay was measured as optimized for the prototypic UTI89 strain used in these assays. Mannoside derivatives that allow growth but inhibit biofilm formation at 50% or greater were verified by repeating the biofilm assay and performing titration curves to determine the IC₅₀. A more rigorous assay tested the ability of the strongest inhibitory mannoside derivatives to reverse hemagglutination of guinea pig red blood cells and to disrupt preformed type 1 dependent biofilms.
iii. A fluorescent polarization assay was developed and miniaturized to monitor the direct binding of a synthesized fluorescently labeled butyl mannose derivative to the FimH receptor as well as competitive displacement by other synthetic mannosides. This fluorescent polarization assay provides a rapid method for monitoring improvements to receptor affinity during the synthetic process, as well as providing Kd binding affinity measurements.

### Example 8. Crystallography.

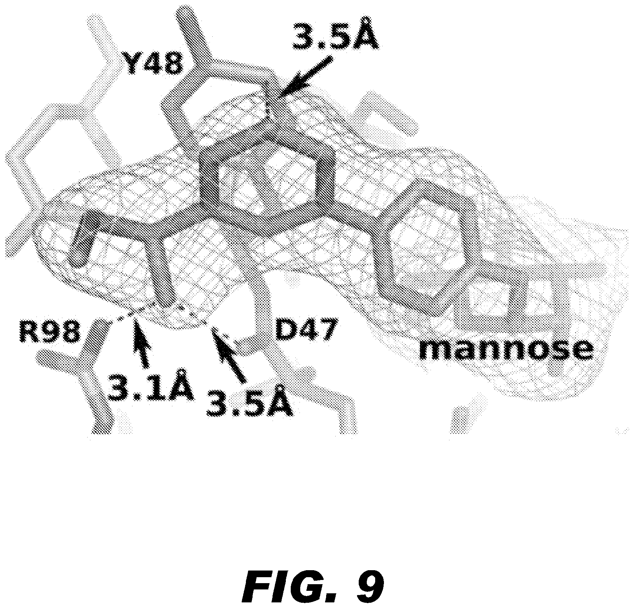

In order to further rationally design better mannoside derivatives to inhibit FimH binding the most efficient inhibitors, such as ZFH253, with the FimH adhesin domain (FimH_{A}) were crystallized to detail their interactions with FimH (**Fig 9**).

The adhesin domain of FimH (residues 1-175 with a C-terminal 6-histidine tag, hereafter FimHA) was cloned into a pTRC99 plasmid and expressed in E. coli. FimH_{A} was purified from bacterial periplasm by passage over cobalt affinity (Talon; Clontech) and Q Sepharose (GE Healthcare) columns. Protein was subsequently dialyzed against 10mM MES at pH 6.5 and concentrated to 15mg/ml for crystallization trials. FimH_{A} crystallized in 20% ethanol, 100mM imidazole pH 8.0, and 200mM MgCl2. Tetragonal bipyramidal crystals measuring 150X150µm formed in two days and did not diffract unless slowly dehydrated over the course of at least 12 hours. The FimH_{A} - ZFH253 complex was therefore formed by soaking individual crystals in a stabilizing solution initially containing 15% ethanol, 100mM imidazole pH 8.0, 200mM MgCl2, 10% PEG200, 5% glycerol and 1mM ZFH253. Individual crystals were placed in 150µl stabilizing solution and allowed to dehydrate to one-third their original volume over the course of 24 hours. Crystals were then harvested without further cryopretection and plunged into liquid nitrogen. Crystals diffracted to 2.55Å at beamline 4.2.2 at the Advanced Light Source. The structure of FimH_{A}-ZFH253 was solved by molecular replacement with the program PHASER using a previously-solved model of FimH_{A} with its methylmannose ligand removed as the search model (PDB ID 1UWF).

An initial map calculated with Fo-Fc coefficients showed unambiguous difference density for ZFH253 occupying the mannose binding pocket of all four copies of FimH_{A} present in the asymmetric unit. These studies showed that the mannose portion of ZFH253 engages in conserved interactions with FimH_{A}. ZFH253 buries 293Å² (calculated with PISA). This places ZFH253 between monomannose, which buries approximately 185Å², and oligomannose-3, which buries 388Å². Relative to monomannose, most of the additional surface area is buried by the two phenyl groups of ZFH253, which do not engage the "tyrosine gate" of Tyr48 and Tyr137 but orient toward Tyr48 such that the phenyl group most distal to the mannose moiety forms a close pi-stacking interaction with Tyr48 (**Fig 9**). This interaction mimics the aromatic-to-saccharide stacking between Tyr48 and Man3 of oligomannose-3 that contributes to the high affinity of high-mannose epitopes to FimH *in vivo.* Additionally, the terminal carboxyl group of ZFH253 forms two hydrogen bonds with FimH (**Fig 9**), offering a structural basis for its 16-fold increase in potency in the HA titer assay over ZFH296, which contains only two unsubstituted phenyl groups. Further, the FimH_{A} /ZFH253 crystal structure suggested several avenues for rational improvement of mannoside derivatives. First, there is an ordered water at 2.7Å from the carboxyl group of the first carbon of the mannose ring that forms close hydrogen bonds with the N-terminus, the main-chain carbonyl of Phe1, and the epsilon oxygen of Gln133. Extension of this carboxyl group by two bond lengths would allow the mannoside to engage these interactions. There is a second ordered water 4.7Å from an ortho carbon of the first phenyl group of the mannoside that could be coordinated by a hydrogen bond donor. Importantly, the FimH_{A} /ZFH253 crystal structure serves as proof of concept that computational docking may serve as a useful tool in rational design of related mannosides. The conformation of ZFH253 seen in the crystal structure is essentially identical to that of a lowest-energy conformer generated by OMEGA2 and docked into a previous crystal structure of FimH by FRED (OpenEye, Inc.). This approach will speed the design of further compounds, with additional crystallography for the exploration of scaffolds other than the biphenyl group of ZFH253.

### Example 9. Inhibition of in vivo functions of FimH.

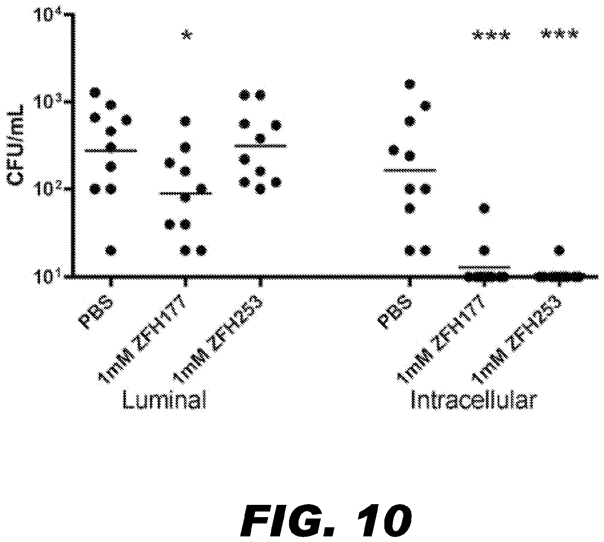

Based on the extensive elucidation of the UPEC pathogenic cascade, critical nodes in pathogenesis that mannosides would target and thereby have powerful and potent therapeutic ramifications were identified. The first step in the UPEC pathway involves FimH-mediated colonization of the bladder epithelium via the recognition of mannose receptors. Bacterial invasion into superficial bladder umbrella cells can then ensue. By preventing bacterial attachment, mannosides will also inhibit the subsequent invasion of bacteria into the bladder epithelial cells. To test this, a well-established gentamicin protection assay that allows the determination of the luminally bound fraction versus the intracellular fraction was utilized (**Fig 10**). In the presence of mannosides ZFH177 and ZFH253 there was a significant reduction in intracellular bacteria at 1 hour post-infection arguing that the mannosides are disrupting specific type 1-mediated binding required for subsequent invasion. It is not clear why a reduction was not observed in the luminal UPEC population. This may be due to contamination from bacteria in the urine or bacteria that are non-specifically associated with the epithelium. In any case, blocking invasion blocks the ability of the bacteria to rapidly expand in numbers. This is due to the ability of the mannosides to block the IBC mechanism.

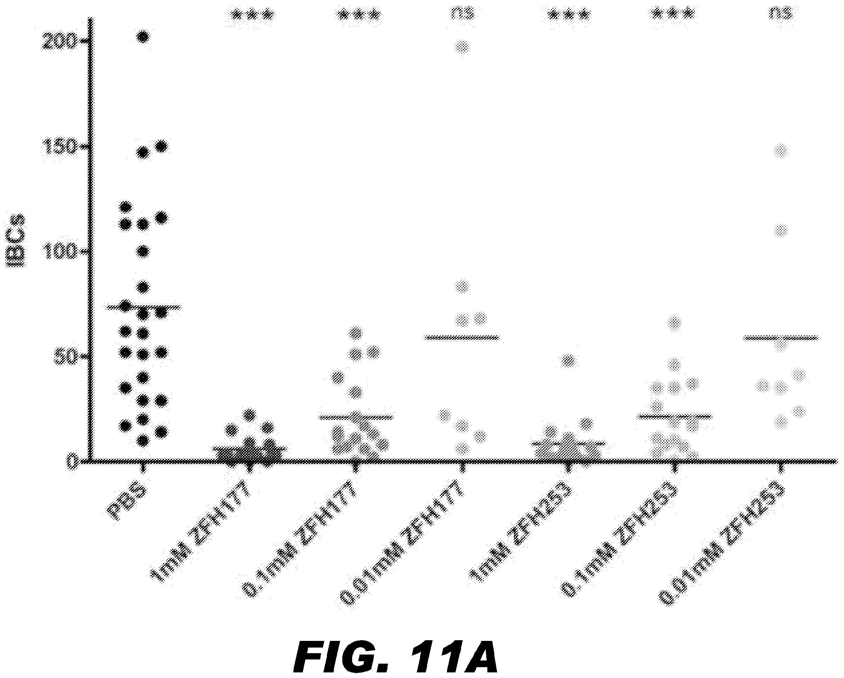

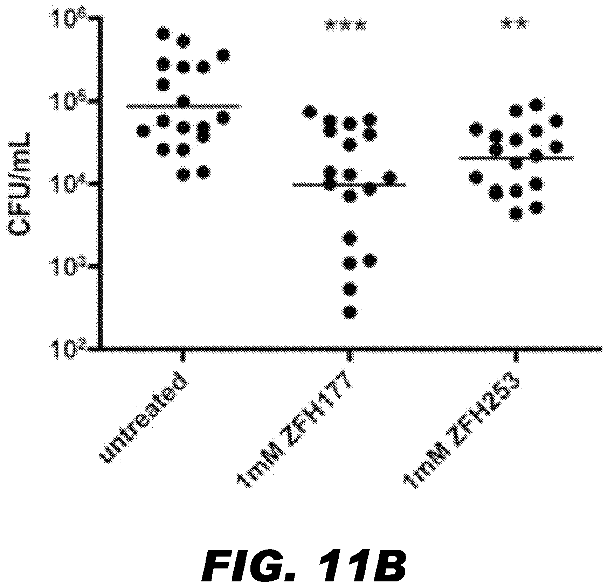

After invasion, UPEC are able to rapidly replicate to form IBCs, thus dramatically expanding in numbers. After maturation of the IBC, bacteria are able to disperse from the biomass and spread to neighboring cells to expand the buildup in bacterial numbers via next generation IBC. The ability of mannosides to block IBC formation was evaluated based on LacZ staining of whole mount bladders, a protocol developed to enumerate IBCs. Whole bladder titers were also determined by quantifying colony forming units (CFUs) at 6 hours post-infection and later time points as test compounds were further developed. In the presence of mannosides ZFH177 and ZFH253 there was a significant decrease in IBCs observed 6 hours post-infection at concentrations as low as 0.1 mM and almost no IBCs observed at 1 mM mannoside (**Fig 11A**). A significant decrease was also seen in CFUs at 6 hours post-infection with 1 mM ZFH177 and ZFH253 (**Fig 11B**). These results strongly argue that the mannosides are able to inhibit first round IBC formation by preventing FimH-mediated binding and invasion into the bladder epithelial cells. The success of these experiments suggested that these compounds would have dramatic potential as potent therapeutics for treating UTI, the most common bacterial infection in highly industrialized countries.

### Example 10. Pharmokinetics/Bioavailability/Toxicity

Pharmokinetic, bioavailability and evaluation of toxicity studies will be performed to elucidate the effectiveness of synthesized mannosides in treating UTI. Using these studies we will identify: i. the kinetics by which the mannoside is excreted in the urine, ii. the dosage administered to the mouse versus the amount that reaches the urine to obtain 1 mM and 0.1 mM concentrations (determined effective through above results), iii. the length of time the mannoside remains in the urine after one dose and iv. the presence of mannoside within the tissue, namely the bladder. The most effective way of delivering the mannoside (oral, IP or tail vein) with no toxicity to the animal will be determined. Toxicity will be evaluated based on animal survival and weight loss/gain of treated animals relative to non-treated animals. Urine was evaluated for mannoside presence and concentration using LC-MS. The delivery method, dose and timing, which gave the highest levels in the urine were then used in treatment trials.

### Example 11. Mannosides as inhibitors of UTI.

Once the proper delivery method, dose and timing will be determined, female mice will be pre-treated with mannoside (two doses) prior to infection (using our well-developed mouse model of UTI) and treated with mannoside throughout the course of infection to maintain efficacious levels within the urine. The potency of the mannoside in preventing UTI based on CFUs in the bladder will be assessed at 6 hours, 48 hours and 2 weeks post-infection and IBC formation at 6 hours post-infection compared to untreated mice.

The ability of mannosides to treat established infections will be also tested. Mice will be treated with mannoside at 24 hours post-infection and the effect on bacterial clearance will be assessed by determining CFUs in the bladder 24 and 48 hours and two weeks post-mannoside treatment. The last set of experiments mimic a potential situation within the clinic.

These experiments will show that inhibition of binding of the FimH adhesin to the bladder epithelium can eliminate infection. The above experiments also establish that the mannosides block the formation of QIRs, which proves their efficacy in reducing the predisposition of the host to suffer a recurrent infection. Due to increasing antibiotic resistance and high rates of recurrence, these compounds introduce an entirely new means of treating microbial infections, using an anti-virulence agent instead of an antimicrobial.

### Example 12. Investigation of dual therapy with adhesion and pilus assembly inhibitors and/or antibiotics.

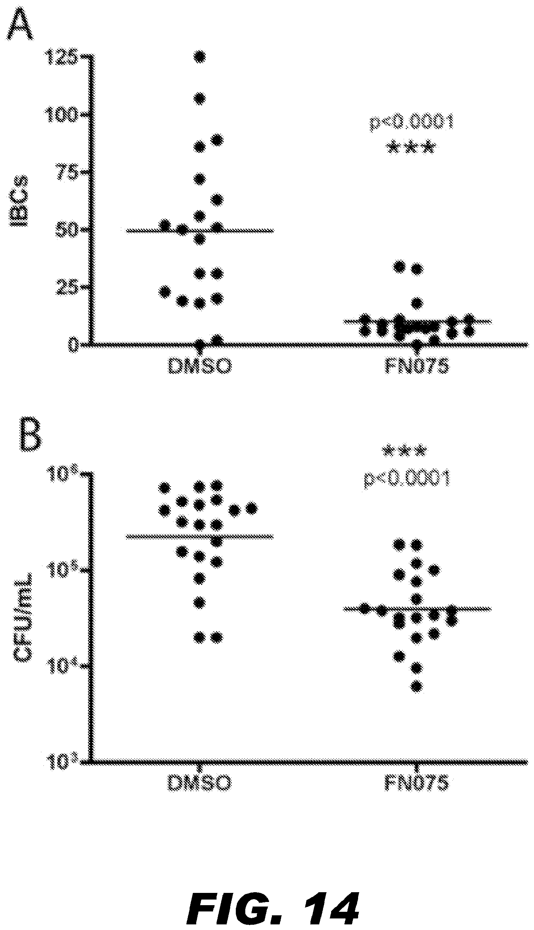

Due to the critical nature of type 1 pili in UPEC pathogenesis, in addition to the work to develop high affinity inhibitors of FimH binding described above, the extensive structural and functional knowledge of the mechanism of pili assembly by the chaperone/usher pathway (see biosketch) was used to develop efficient inhibitors of pilus biogenesis ("pilicides") (**Fig 12**). The most efficient pilicides show greater than 90% inhibition of hemagglutination and type 1 dependent biofilm at concentrations in the low micromolar range (**Fig. 13**). Further, the structural basis by which pilicide block pilus assembly were identified. These compounds were rationally designed based on a 2-pyridone scaffold that has affinity for beta-sheet structures. One of the best pilicides to date, FN075, was able to achieve significant attenuation of virulence as measured by CFUs and IBC formation (**Fig. 14**).

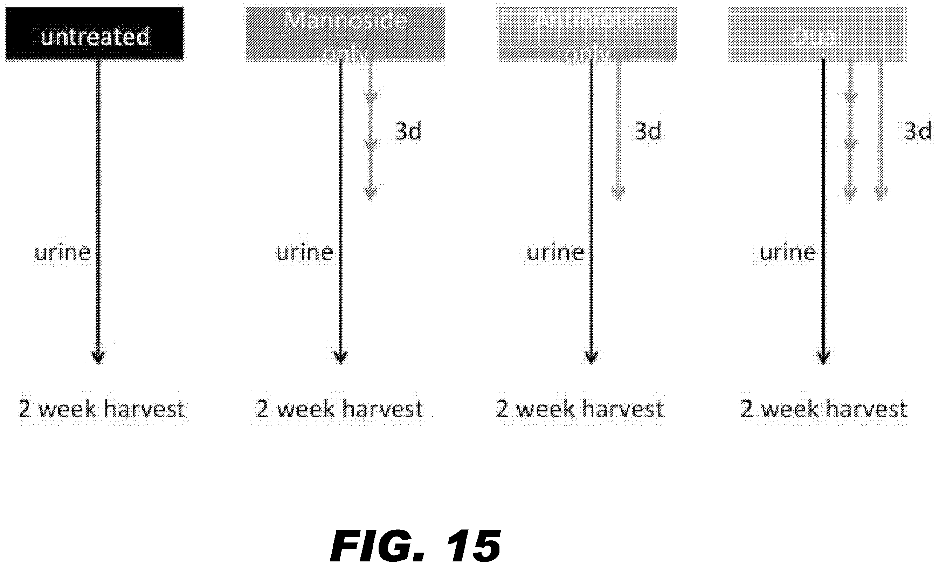

Using pilicides and mannosides in combination would potentially give a "double hit" to this critical type 1 pilus system, potentially resulting in synergy by inhibiting pre-existing type 1 pilus function while eliminating the ability to produce new pili. Combination therapy using the most effective mannoside inhibitors and pilicides with antibiotics were tested. In one experiment, groups of mice were treated with ZFH56 mannoside treatment only, with antibiotic treatment only, or with a dual treatment (mannoside + antibiotic). One group of untreated mice was used as a control (**Fig. 15**).

For mannoside treatment, bacteria were incubated with 1mM ZFH56 prior to inoculation. Every 24h for 3 days, mice were transurethrally inoculated with 1mM ZFH56. For antibiotic treatment, mice were inoculated with UTI89 and immediately given antibiotics in the drinking water for 3 days. Antibiotics used were trimethoprim-sulfamethoxazole (SXT) at 54 and 270 mg/ml, respectively. For dual treatment, bacteria were incubated with 1mM ZFH56 prior to inoculation. Every 24h for 3 days, mice were transurethrally inoculated with 1mM ZFH56. Additionally, mice were immediately given antibiotics in the drinking water for 3 days as in the antibiotic only treatment. Urine was collected every day for 13 days. At 2 weeks, mice were sacrificed and bladders were titered.

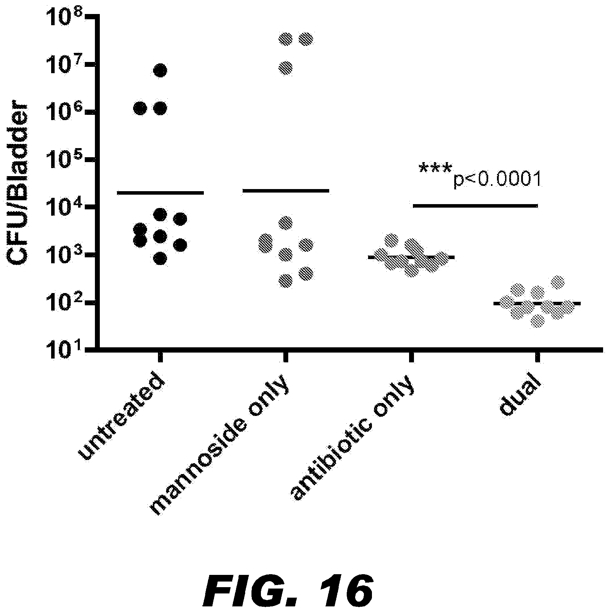

Dual treatment showed significantly lower levels of bacteria in the bladder than antibiotics alone (**Fig. 16**).

### Example 13. Bacterial Adhesion - A Source of Alternate Antibiotic Targets

### Introduction

More than a century ago, the discovery of Penicillin marked a significant, albeit not immediately recognized, advance in the field of medicine. By the middle of the 20^{th} century this naturally occurring fungal antibiotic had single-handedly vanquished the biggest wartime killer -- infected wounds. Just four years after the mass production of penicillin began in 1943, resistant microbes started to appear beginning with *Staphylococcus aureus,* followed shortly thereafter by *Streptococcus pneumoniae* and *Neiserria gonorrheae.* Today this list includes antibiotic resistant *Enterococcus, Salmonella, Mycobacterium tuberculosis* and *Escherichia coli,* to name just a few. The bacterial infections which contribute most to human disease are also those in which emerging and microbial resistance is most evident: diarrheal diseases, respiratory tract infections, urinary tract infections, meningitis, sexually transmitted infections, and hospital acquired infections. Thus, there is dire need for new therapeutics to save the lives and ease the suffering of millions of people.

### Rising antibiotic resistance

The antibiotics that are available today are primarily variations on a single theme-bacterial eradication based on inhibition of essential molecular processes required for bacterial growth (bacteriostatic) or cellular maintenance (bactericidal). Some target cell-wall biosynthesis, whereas others inhibit protein synthesis or DNA replication. These life or death treatments put selective pressure on the bacteria to adapt or die, selecting for antibiotic resistance. Second generation drugs were developed to combat the resistances that arose, however, they employed the same general mechanism of action. In addition, while some last resort drugs have minimal resistance thus far, they are much more expensive, often are more toxic and typically unavailable to many countries around the world. Consequences of treatment failure due to antibiotic resistance are high with greater morbidity, mortality and transmission of the resistant organism. Even if pharmaceutical companies increased efforts to develop next generation replacement drugs, bacterial evolution is outpacing drug development for these classes of antibiotics. We are nearing an end to the seemingly endless flow of antimicrobial drugs. We are now faced with a long list of microbes that have found ways to circumvent different structural classes of drugs and are no longer susceptible to most, if not all, therapeutic regimens. New tactics and weapons are needed to combat bacteria that are, owing to evolution and selection, moving targets.

### Alternative antibiotic targets

Targeting bacterial virulence is an alternative approach to the development of new classes of antimicrobials that can be used to specifically target and disarm pathogens in the host, while leaving commensal bacteria unperturbed. Prevention of the activity of virulence factors will render the bacteria less able to colonize and give beneficial symbionts and the host immune system time to eradicate the disease causing pathogen prior to colonization or facilitate clearing of an established infection. Furthermore, stripping microorganisms of their virulence properties without threatening their existence may offer a reduced selective pressure for drug-resistant mutations and provide increased potency. Upon exposure to the host, bacteria respond by the production of an arsenal of virulence factors to combat host immune responses and facilitate persistence in their desired niche. There are many examples of virulence factors that represent potential therapeutic targets. Polysaccharide capsules prevent phagocytosis. Siderophores facilitate iron acquistion. Flagella promote motility and chemotaxis. Some bacterial species secrete toxins that alter and disrupt the eukaryotic host cell resulting in disease. Yet another strategy employed by bacteria is the production of a molecular syringe, known as a type three secretion system, that injects effectors into the host cell causing disease. Another fundamental virulence property of nearly all microbes is their ability to adhere to host cells. This step is typically crucial in pathogenesis and without it bacteria are more likely to be eradicated by the host. Bacterial adhesins typically mediate attachment to a specific receptor with stereochemical specificity, a process that is thought to determine the host and tissue tropisms of a pathogen. While many virulence factors may represent attractive therapeutic targets, this review will focus on two strategies to develop anti-virulence therapeutics based on targeting adhesive pili using uropathogenic *E. coli* (UPEC) as the model system. The first strategy depends on competitively inhibiting bacterial binding to host cells through addition of exogenous carbohydrates that mimic host ligands. The second strategy targets the mechanism by which adhesive pili are assembled thus inhibiting bacterial adhesion to host cells. Thus, both strategies could be used in synergy: the first compound prevents bacterial attachment and colonization by occupying the binding pocket of the bacterial adhesin while the second compound enters the cell and prevents future pilus production. Such a two-pronged approach could eliminate adherence to host cells and promote clearance of the bacteria.

### Pili in bacteria

Pathogens are capable of presenting multiple adhesins that can be expressed differentially to permit binding in specific sites and at particular times over the course of a complex infectious cycle. To achieve this, many bacterial species possess long filamentous structures known as pili or fimbriae extending from their surfaces. Pili are extracellular polymers that also have been shown to play a role in invasion, biofilm formation, cell motility, and protein and DNA transport across membranes. Pilus formation is common to many pathogenic bacteria, both Gram-negative and Gram-positive pathogens. Despite the diversity in pilus structure and biogenesis, assembly mechanisms among Gram-negative and Gram-positive bacteria are conserved within each group. Gram-positive pili are formed by covalent polymerization of pilin subunits in a process that requires a dedicated sortase enzyme. In contrast, Gram-negative pili are typically formed by non-covalent homopolymerization of major pilus subunit proteins. This review will focus on a major family of adhesive pili in Gram-negative bacteria that are classified by their mechanism of assembly; the so-called chaperone/usher (CU) pili (**Table 14**).

**Table 14**

| **Chaperone-usher assembled pili** | | | | | |
|---|---|---|---|---|---|
| **Adhesive fiber** | **Major pilin** | **Chaperone/ usher** | **Adhesion** | **Organisms** | **Disease** |
| Type 1 | FimA | FimC/FimD | FimH | *Escherichia coli, Klebsiella pneumoniae, Salmonella* species | cystitis, sepsis, meningitis |
| P | PapA | PapD/PapC | PapG | Uropathogenic *E. coli* (UPEC) | pyelonephritis |
| Prs | PrsA | PrsD/PrsC | PrsG | *E. coli* | cystitis |
| S | SfaA | SfaE/SfaF | SfaS | *E. coli* | UTI, meningitis, sepsis |
| Hif | HifA | HifB/HifC | HifE | *Haemophilus influenzae* | otitis media, meningitis |
| Type 2 and 3 | Fim2 & Fim3 | FimB/FimC | FimD | *Bordetella pertussis* | whooping cough |
| Pef | PefA | PefD/PefC | unknown | *Salmonella typhimurium* | gastroenteritis |
| Long polar fimbriae | LpfA | LpfB/LpfC | unknown | *S. typhimurium* | gastroenteritis |
| MR/K | MrkA | MrkB/MrkC | MrkD | *K. pneumoniae* | pneumonia |
| Myf fimbriae | MyfA | MyfB/MyfC | unknown | *Yersinia enterocolitica* | enterocolitis |
| PMF | PmfA | PmfC/PmFD | PmfF | *Proteus mirabilis* | UTI |
| Dr | DraA | DraB/DraC | DraE | UPEC | pyelonephritis |
| | | | | Diffuse adhereing *E. coli* (DAEC) | cystitis |
| Afa | AfaA | AfaB/AfaC | AfeE | UPEC | cystitis, diarrhea |
| F1 | Caf1 | CHf1M/Caf1 A | | *Yersinia pestis* | plague |

| | | | | | |
|---|---|---|---|---|---|
| **Alternarive chaperone pathway** | | | | | |
| **Adhesive fiber** | **Major pilin** | **Assembly proteins** | **Adhesion** | **Organism** | **Disease** |
| CS1 pili | CooA | CooB/CooC | CooD | *E. coli* | diarrhea |

| | | | | | |
|---|---|---|---|---|---|
| **Extracellular nucleation-precipitation pathway** | | | | | |
| **Adhesive fiber** | **Assembly proteins** | **Adhesin** | **Organism** | **Disease** | |
| Curli | CsgB (nulceator), CsgE and CsgF (assembly) and CsgG (secretion) | CsgA (major subunit) | *E. coli* | sepsis | |
| | | | *Salmonella* spp. | gastroenteritis, sepsis | |

In Gram-negative bacteria, one of the best-characterized pilus assembly systems is the CU pathway. Hundreds of operons encoding CU systems have been reported in a plethora of pathogenic organisms with as many as twelve encoded by a single organism. Examples of CU-assembled adhesive virulence fibers include: Type 1 and P pili predominately on UPEC that cause urinary tract infection (UTI), S pili of *E*. *coli* strains that cause sepsis, meningitis and UTI and Hif pili of *Haemophilus influenza* which causes otitis media. The causative agents of whooping cough (*Bordetella pertussis*), gastroenteritis (*Salmonella typhimurium*), and pneumonia (*Klebsiella pneumoniae*) also assemble fibers through the CU pathway. Additionally there are 'non-pilus adhesins' assembled by the CU pathway that are generally homopolymers composed of a single protein subunit, like the Afa/Dr family of adhesins of *E. coli* and the polymeric F1 capsular antigen of *Yersinia pestis.* The ubiquitous nature of this pathway paves the way for a potentially broad-spectrum anti-virulence therapeutic that disrupts the assembly of the pilus fibers thus eliminating bacterial colonization.

### Pili in UTI

Of the CU pathway assembled pili, P and type 1 pili are the most extensively characterized. Type 1 pili are expressed throughout the *Enterobacteriaceae* family but have been shown to be essential for UPEC in colonization of the urinary epithelium. UPEC is the leading causative agent for greater than 80% of UTIs which is one of the most common bacterial infections in industrialized countries. UTIs are a significant burden, resulting in more than 8 million outpatient visits per year in the United States and expended costs of US $3.5 billion annually for evaluation and treatment. Women are the most frequent sufferers - a female has a 60% chance of suffering from at least one UTI in her lifetime. Additionally, up to 44% of women who present with an initial episode of UTI will experience recurrence. While the increased likelihood of recurrence it not entirely understood, data suggests that bacteria can persist within bladder tissue despite antibiotic treatments, and may serve as bacterial reservoirs for recurrent infections.

Type 1 pili are expressed by virtually all UPEC isolates. The FimH adhesin at the tip of type 1 pili achieves specific binding to host cells within the urinary tract. FimH specifically binds mannose groups that abundantly decorate uroplakins on the luminal surface of the bladder. A deep, negatively charged pocket in the N-terminal receptor-binding domain of FimH accommodates the mannose with stereochemical specificity. Once bound, a pathogenic cascade is initiated that involves several distinct phases as examined in the murine cystitis model and human UTIs. Numerous innate defenses are activated early in an attempt to stem off the infection. These defenses include, cytokine induction followed by inflammation, iron sequestering, exfoliation of the colonized epithelial cells and sheer forces elicited by urine flow. Even if UPEC successfully invade into a bladder epithelial cell, the bladder cell has been shown to be able to expel UPEC, potentially serving as an additional innate defense. However, if UPEC are able to escape into the cytoplasm of superficial umbrella cells, they are able to rapidly replicate to form tightly packed intracellular bacterial communties (IBCs) with biofilm-like qualities. Within the urothelial cells, the bacteria are protected from the flow of urine and host defenses. Eventually UPEC detach from the IBC, disperse and initiate new rounds of IBC formation. This represents a mechanism by which a single invasion event dependent on type 1 pili adhesion can lead to rapid bacterial expansion and colonization of the urinary tract. IBC formation occurs in the acute states of infection. One long-term outcome of infection is that UPEC are also able to form a quiescent intracellular reservoir (QIR) within the bladder. Bacteria in the QIR do not rapidly replicate, but remain dormant, hidden from the immune system and antibiotics. They are then able to reestablish an infection at later timepoints post-treatment resulting in a recurrence. Due to this alternative pathway of continuous colonization, UTIs are more frequently being identified as chronic infections. Detailed analysis of the UPEC pathogenic cascade has identified several potential targets to inhibit virulence, such as siderophores, capsule and flagella. However, type 1 pili represent a particularly attractive drug target because they are ubiquitous among UPEC, and are required to initiate a pathogenic cascade that evades the host immune system and can lead to acute, chronic, persistent or asymptomatic infection. Disruption of type 1 pili function could break the cycle of chronic infection.

### New drug development strategies to inhibit pilus mediated function

Carbohydrates of various forms are abundantly expressed on the animal cell surface. Microbes have taken advantage of this property and evolved with the ability to adhere to sugar receptors in an organ-specific manner for colonization and infection. Sugars are commonly involved in cell recognition and communication playing important roles in microbial adherence and uptake, colonization and biofilm formation, and in virulence. Since bacterial adhesion to host cells is extremely specific, exogenous sugars can block binding to carbohydrate receptors and competitively displace or inhibit bacteria from attachment to cells. The ability to impair bacterial adhesion represents an ideal strategy to combat bacterial pathogenesis because it targets an important early step in the infectious process, and would also be suitable for use as a prophylactic to prevent infection. In the case of UPEC, knowledge of the structural basis of how FimH recognizes the mannose receptor, has lead to the development of a ligand-based antagonist, termed mannoside, that mimics the natural receptor for FimH but with increased affinity and avidity. Mannosides effectively block the adhesive properties of type 1 pili by inhibiting bacterial colonization, invasion, IBC formation and disease. Furthermore, the sequence of approximately 300 *fimH* genes of clinical UPEC isolates showed that the mannose-binding pocket is invariant. Thus mannosides are a powerful new class of therapeutics that could have a potent therapeutic effect by preventing UPEC pathogenesis and disease. This approach can be readily extended to other adherent organisms by tailoring the anti-adhesive compounds to antagonize their specific receptor-ligand interactions (**Fig. 17**). Indeed, sugars designed to target adhesive structures of organisms such a *Pseudomonas aeruginosa, Stapyhlococcus* and *Streptococcus* have already proved efficacious at inhibiting adherence *in vitro.*

The goal of the second strategy is to interrupt pilus assembly, which, like the first strategy, blocks pilus-mediated adhesion and subsequent disease. To disrupt pilus assembly, one must first understand the mechanisms employed by bacteria to form fibers. Chaperone-usher mediated assembly prevents premature subunit aggregation and facilitates the ordered secretion, folding and assembly of hundreds of thousands of subunits into fibers on the surface of a bacterium. Components of the pilus are secreted through the general secretory pathway into the periplasm. Once in the periplasmic space, a specific chaperone binds each pilus subunit to facilitate proper folding and prevent premature assembly of subunits. Each subunit consists of six of the seven strands of a standard immunoglobulin (Ig)-fold and an N-terminal extension (Nte) (**Fig. 18A**). In order to form a stable structure prior to incorporation into the growing fiber, the chaperone donates structural information to each subunit, its G1 β strand, to compensate for the missing seventh strand of the subunit in a process called donor strand complementation (DSC) (**Fig. 18B**). DSC prevents aggregation of the subunits prior to fiber formation. The pilin/chaperone complex is then delivered to the usher, which is a pore in the outer membrane and serves as a platform for pilus assembly. During pilus assembly, the free Nte of one subunit displaces the chaperone bound to another subunit and serves as the seventh strand of the Ig-like fold in a process called donor strand exchange (DSE) (**Fig. 18C**). DSE leads to the polymerization of the fiber extending from the bacterium. Since chaperone-usher systems are necessary for the assembly of extracellular adhesive organelles in a wide range of pathogens, inhibitors may serve as broad-range anti-virulence therapeutics, an attractive feature that would enhance the marketability of an effective drug.

Pilicides are a class of pilus inhibitors that target chaperone function and inhibit pilus biogenesis. A recent study (Pinkner JS et al: PNAS (2006) 103(47):17897-17902) identified a new class of pilicides based on a bi-cyclic 2-pyridone chemical scaffold that inhibit pilus assembly by binding to the chaperone and disrupting the chaperone-usher interaction. As a result, pilicides inhibit pilus dependent activities such as colonization, invasion and biofilm formation. Biofilms are structured communities of microorganisms encapsulated within a self-developed polymeric matrix that defends them against antibiotics and the host immune system. They are of great medical importance, accounting for over 80% of microbial infections in the body. Pilicides target regions on the chaperone that are highly conserved among chaperone-usher pathways and are thus able to inhibit assembly of multiple pilus systems (type 1 and P pili of *E*. *coli*) (**Fig. 17**). Pyridinones also avoid the severe drawbacks observed for peptide-based drugs; poor absorption after oral administration, rapid enzymatic degradation, and quick excretion.

Another distinct mechanism of adhesive fiber assembly among Gram-negative bacteria is fibers assembled by the extracellular nucleation/precipitation pathway (curli). Curli are proteinaceous fibers found on enteric bacteria such as *E*. *coli* and *Salmonella* spp. Despite curli's unclear role in pathogenesis, their biochemical and structural properties resemble eurkaryotic amyloid fibers found in neurodegenerative diseases, such as Alzheimer's, Parkinson's, and prion diseases making them an ideal model system to study amyloid biogenesis. Curli are heteropolymers that consist of a major subunit, CsgA, and minor subunit, CsgB. CsgA and CsgB are secreted into the extracellular milieu via the outer membrane pore, CsgG. With the help of two chaperone-like proteins, CsgE and CsgF, CsgA is nucleated into a fiber by CsgB. Curli fibers have been implicated in biofilm formation, environmental survival and resistance to sanitizing agents. A recent study (Cegelski L *et al:* submitted) showed that derivatives of the 2-pyridone compounds mentioned above were able to inhibit curli assembly giving them the name curlicides. The lack of curli fibers prevented curli-dependent biofilm formation and reduced infection load *in vivo* as measured by the murine model of UTI. Pilicides and curlicides selectively disrupt a protein-protein interaction required for the biogenesis of a bacterial virulence factor instead of targeting a process essential for survival.

Employing both anti-virulence strategies, inhibition of receptor binding and disruption of pilus assembly, could result in very potent anti-adhesive therapeutics. For example, synergistic effects could be obtained by inhibiting pre-existing type 1 pilus function through mannosides while also eliminating the ability to produce new pili through pilicides. Furthermore, if infection is not entirely controlled by the two-pronged approach, antibiotics can be added, however, at much lower concentration to reduce the opportunity to develop resistance. Type 1 pili are an essential virulence factor for colonization of the urinary tract and are the most extensively studied chaperone-usher fimbrial system. Thus, they have been described in this review as an excellent target resulting in the development of new prototypic therapeutics to treat UTIs. This new drug class could translate into a multitude of other therapies for Gram-negative infections.

### Conclusion

While this review focuses on the development of therapeutics to inhibit adhesion, there are many other virulence factors employed by bacteria to colonize and persist within the host. The general strategy of inhibiting virulence factors rather than essential growth factors changes the paradigm of drug development and reduces the evolutionary pressure for bacteria to develop resistance. Bacteria can survive in the presence anti-virulence drugs, but will be eliminated by the host immune system prior to establishing a foothold. Furthermore, since a complex virulence mechanism is targeted, if mutants arise, they will most likely be avirulent. If the host immune system cannot resolve any residual infection, it is possible to couple anti-virulence therapeutics with traditional antibiotics although at lower doses thus reducing resistance potential. A commitment to developing therapeutics that target virulence requires a serious change in our perspective for treating infectious diseases and increased efforts should be focused on bringing these new therapeutics from bench to bedside.

### Example 14. Orally active mannosides subvert antibiotic resistance of E. coli in bladder infection

In this example, therapeutic utility of mannosides that are competitive, high-affinity inhibitors of FimH mannose binding activity is analyzed, as measured by biofilm formation, type 1 pilus expression and bladder colonization, invasion and IBC formation. The mannose binding site of FimH consists of a deep acidic pocket surrounded by a ridge of hydrophobic residues. The acidic amino acid residues of the binding pocket are invariant in all UPEC and mutational studies showed that altering any of these residues abolishes binding to mannose and attenuates virulence. From the rational design and optimization of mannosides, several potent inhibitors of FimH-dependent hemagglutination were identified, including ZFH-1101, ZFH-1177, ZFH-1253, ZFH-2050 and ZFH-2056 (**Fig. 19a**). These mannosides were evaluated first for their ability to inhibit biofilm formation or disrupt preformed biofilms. Biofilm inhibition was evaluated on FimH-dependent UTI89 biofilms grown on PVC in LB at room temperature. The median inhibitory concentration (IC₅₀) values for each of the compounds were found to be in the low micromolar range. Compounds ZFH-1253 and ZFH-2056 were the most potent biofilm inhibitors with IC₅₀ values of 0.94 and 0.74 µM, respectively (**Fig. 19b****).** Biofilm disruption was evaluated by the addition of mannosides to preformed 24 h biofilms followed by growth for an additional 16 h. All five mannosides were shown to facilitate dispersal of the preformed biofilm. The IC₅₀ values for dispersal were higher than those concentrations needed to prevent biofilm formation, however the efficacy trend remained the same (**Fig. 19c**). Visualization of mannoside-treated PVC biofilms by confocal microscopy showed that ZFH-2056 treated biofilm lacked continuity as demonstrated by holes in the biofilm, and it lacked the tall mushroom-like structures observed in untreated biofilm (**Fig. 19 d****,****e**). Biofilm causes many antibiotic treatment failures since antibiotics are unable to penetrate its dense matrix. These results demonstrate that mannoside can prevent biofilm formation and dissolve preformed biofilms arguing that mannosides may augment antibiotic efficacy by altering bacterial community behavior.

To test the efficacy of the mannosides *in vivo,* UTI89 expressing type 1 pili were pre-incubated with mannosides, inoculated into mice and IBCs were quantitated at 6 hours post-infection (hpi). The efficacy of compounds ZFH-1177, ZFH-1253, ZFH-2050 and ZFH-2056 in inhibiting IBC formation correlated with their potency observed in the *in vitro* assays. The most significant reduction in IBCs occurred with as little as 10 µM ZFH-2050 or ZFH-2056 (**Fig. 20a**). These studies identified ZFH-2056 as the most potent compound tested in preventing colonization.

Pharmacokinetic (PK) studies with ZFH-2056 evaluated *in vivo* dosing conditions after intraperitoneal (IP) injection or oral (PO) gavage. ZFH-2056 was dosed IP and compound levels in the urine were evaluated at several timepoints by HPLC using mass spectrometry (MS) detection. Doses of 5 mg/kg and 10 mg/kg resulted in concentrations of nearly 5 mM in the urine 30 min after treatment (**Fig. 20b**). Eight hours after dosing, ZFH-2056 remained at levels equal to or slightly below the IC50 (0.74 µM) as determined by biofilm inhibition. Since oral dosing is the desired route of delivery, we next evaluated the concentration of drug after 100 mg/kg PO of ZFH-2056. The latter resulted in a 10 fold lower initial concentration of ZFH-2056 versus IP but, significantly, urine levels were 10-fold higher at 8 hours post-dosing. It is noteworthy that >95% of drug cleared to the urine is intact with the remaining <5% being the hydrolysis products, D-mannose and phenol.

The efficacy of *in vivo* mannoside treatment was evaluated after dosing animals with mannoside either IP or PO 30 min prior to infecting with UTI89. At 6 hpi the bladders were removed and total bacterial CFUs were quantitated. In both the IP and PO treated cohort, there was a significant drop in bacterial counts demonstrating the efficacy of mannoside in reducing overall colonization of the bladder (**Fig. 20e**). Furthermore, IBC quantification showed a significant reduction in IBCs in the mice that were pretreated with mannoside by either the IP or oral route (**Fig. 20f**). To test whether mannoside reduced IBC formation by blocking UPEC invasion into the bladder tissue, gentamicin treatment assays were performed. Gentamicin efficiently kills extracellular UPEC but is unable to penetrate tissue and thus intracellular bacteria survive gentamicin treatment18. In the ZFH-2056-treated mice, gentamicin treatment of the bladders eliminated all CFUs (**Fig. 20g**). In bladders from untreated mice, 10³ - 10⁴ CFUs remained after gentamicin treatment. These results argued that mannoside effectively prevented UPEC penetration into the bladder tissue. Confocal microscopy confirmed these results. Bladders of the untreated cohort showed normal, robust IBC formation (**Fig. 20c**) whereas IBCs were rarely seen in the mannoside treated mouse bladders but bacteria were observed on the bladder epithelial surface (**Fig. 20d**). These results demonstrate that mannosides prevent bacterial invasion into the bladder tissue and significantly reduce infection in the bladder.

The first-line treatment of choice for UTI has long been a 3-day course of TMP-SMZ. However, resistance to TMP-SMZ is on the rise. Furthermore, humans have a 38% carriage rate of TMP-SMZ resistant (TMP-SMZR) strains, and these numbers are even higher in low resource countries with a 94% carriage rate. TMP-SMZ is known to concentrate in the urine. Therefore, it was reasoned that by preventing bacterial invasion into the bladder tissue, mannoside may have an anti-virulence synergism with TMP-SMZ and may circumvent the TMP-SMZ resistance problem. This was tested in mice given TMP-SMZ for 3 days prior to inoculation with either UTI89 or TMP-SMZR strain, PBC-1. Mice were either untreated or IP treated with mannoside ZFH-2056 30 min prior to inoculation with bacteria. After inoculation with UTI89 or PBC-1, bacterial CFUs were quantitated 6 hpi. Upon treatment with only ZFH-2056 there was a significant drop in bacterial load of both strains in the bladder. As expected, treatment with TMP-SMZ alone resulted in a significant drop in bacterial load in the UTI89-infected mice but had no effect on PBC-1, since it is resistant to TMP-SMZ. In the dual treatment group there was a significant drop in bacterial CFUs compared to mannoside alone or TMP-SMZ alone for both strains which was most pronounced for PBC-1 (**Fig. 21a**). The observation that TMP-SMZR strain PBC-1 was able to succumb to antibiotic treatment in the presence of mannoside suggested that either mannoside increases the efficacy of TMP-SMZ killing or that it exposes the bacteria to concentrations at or above the MIC necessary for killing. Mannoside was not found to increase the efficacy of TMP-SMZ killing when bacteria were grown in varying concentrations of TMP-SMZ with and without 100 µM of ZFH-2056 (**Fig. 21b**). This analysis revealed that the TMP-SMZ resistant strain had a MIC of 256 and 1280 µg/ml, respectively. Furthermore the HA titer of PBC-1 after growth in varying concentrations of TMP-SMZ showed that type 1 pili function was not affected by antibiotic (data not shown). It has long been known that TMP concentrates in the urine, and this serendipitous feature is one reason TMP-SMZ has been the antibiotic of choice for UTI over the last several decades. Using MS, the concentration of TMP-SMZ was measured in the urine of mice after 3 days of treatment with 54 µg/ml and 270 µg/ml TMP and SMZ, respectively. TMP concentrations were determined to be 9.95 +/- 4.36 mg/ml. SMZ was present at 67.17 +/- 32.51 µg/ml. These results show that by preventing bacterial invasion, mannoside keeps UPEC extracellular thus exposing them to TMP concentrations in the urine that are well above the MIC of the UPEC strain. This enables the killing of bacteria that clinically are resistant to TMP-SMZ. Thus, the invasion of the TMP-SMZR UPEC strain PBC-1 into bladder tissue protected it from the exposure of TMP-SMZ concentrations of antibiotic necessary for killing. However, mannoside prevented access of the bacteria to the intracellular compartment and thus kept the bacteria extracellular where TMP-SMZ concentrates to high levels in the urine, resulting in their succumbing to killing by the antibiotic. These results highlight the importance of the intracellular pathway in bacterial persistence. Not only do the bacteria escape the immune system in their intracellular niche, they are also able to escape exposure to effective antibiotics.

### Methods for Example 13

UTI89 biofilm was grown in LB +/- mannoside for 24 h at 22°C in PVC plates and quantitated using crystal violet. UTI89 biofilm for confocal microscopy was grown in LB for 24 h at 22°C on PVC coverslips. Mannoside was then added and biofilm was grown for an additional 16 h. Coverslips were then washed in PBS, fixed in 2% paraformaldehyde and stained with SYTO9 prior to confocal microscopy. For all animal experiments UTI89 or PBC-1 was grown 2x24 h statically in LB at 37°C and inoculated at a dose of 1 x 10⁷ bacteria in 50 µl. All mice used were female C3H/HeN (Jackson). For the pretreatment studies, bacteria were incubated with mannoside for 20 min prior to inoculation into the mouse. For IP dosing, 50 µl of 2 mg/ml (5 mg/kg) or 4 mg/ml (10 mg/kg) ZFH-2056 in PBS was injected into the mouse 30 min prior to inoculation of bacteria. For oral dosing, 100 µl of 20 mg/ml (100 mg/kg) ZFH-2056 in 8% DMSO was inoculated with a gavage needle 30 min prior to inoculation of bacteria. Mass spectrometry was used to quantify urinary mannoside or TMP-SMZ concentrations. For CFU counts, bladders were harvested at 6 hpi and placed in 1 mL PBS. Bladders were then homogenized, diluted and plated on LB. After growth at 37°C overnight, bacterial counts were determined. LacZ staining and gentamicin protection assays were performed at 6 hpi. For antibiotic experiments, mice were given TMP-SMZ in the drinking water at a concentration of 54 µg/ml and 270 µg/ml, respectively. Water was changed daily with fresh antibiotics. Standard growth curve assays and hemagglutination assays were performed with TMP-SMZ. All statistical analysis performed was a two-tailed Mann-Whitney U test.

*Bacterial strains.* UTI89 is a prototypical cystitis isolate of serotype O18:K1:H7. PBC-1 is a TMP-SMZR strain of serotype OX13:K1:H10 isolated from a 59 year old asymptomatic female with a history of recurrent UTI and diagnosis of primary biliary cirrhosis.

*Synthesis of mannosides.* Following the procedure outlined in Han et. al. 2010 (J Med Chem 53 (12), 4779-4792), ZFH-2056 was synthesized on a multi-gram scale with >98% purity by HPLC and NMR.

*Biofilm assay.* UTI89 was grown in LB broth in wells of PVC microtiter plates at 23°C in the presence of individual mannosides at varying concentrations. After 48 h of growth, wells were rinsed with water and stained with crystal violet for quantification as described. For biofilm disruption activity in PVC plates, UTI89 was grown in LB broth in wells of PVC microtiter plates at 23°C. After 24 h of growth, mannoside was added and biofilms were grown for an additional 16h. Wells were then rinsed, stained with crystal violet and quantified. For biofilm disruption activity on PVC coverslips, UTI89 was grown in LB broth in 50 mL conicals containing PBC coverslips at 23°C. After 24 h of growth, 0.3 µM ZFH-2056 was added and biofilm was grown for an additional 16 h. Coverslips were then rinsed, fixed with 2% paraformaldehyde (v/v), stained with SYTO9 (1:1000 in PBS; Molecular Probes) and observed with a Zeiss LSM410 confocal laser scanning microscope under a 63X objective.

*Animal infections.* Bacteria were grown under type 1 pili-inducing conditions (2x24 h at 37°C statically in LB). The bacteria were harvested and resuspended to an OD₆₀₀ of 0.5 in PBS. Eight-week-old C3H/HeN (Harlan) female mice were anesthetized by inhalation of isoflurane and infected via transurethral catheterization with 50 µl of the bacterial suspension, resulting in 1-2 x 10⁷ inoculum. At 6 hpi, mice were sacrificed by cervical dislocation under anesthesia and the bladders were immediately harvested and processed as described below. All animal studies using mice were approved by the Animal Studies Committee of Washington University (Animal Protocol Number 20100002).

*Enumeration of bladder IBCs.* For bacterial pretreatment experiments, mannoside was added to the bacterial suspension and incubated for 20 min. For animal pretreatment experiments, mannoside ZFH-2056 was administered either IP (5 mg/kg) or orally (100 mg/kg) 30 min prior to inoculation with UTI89. To accurately count the number of IBCs, mice were sacrificed 6 hpi and bladders were aseptically removed, bisected, splayed on silicone plates and fixed in 2% paraformaldehyde (v/v). IBCs, readily discernable as punctate violet spots, were quantified by LacZ staining of whole bladders.

*Pharamacokinetic analysis.* For intraperitoneal dosing, 50 µl of a 2 mg/ml (5 mg/kg) or 4 mg/ml (10 mg/kg) solution of ZFH-2056 in PBS was injected into the peritoneal cavity of the mouse. For oral dosing, 100 µl of a 20 mg/ml (100 mg/kg) solution of ZFH-2056 in 8% DMSO was inoculated with a gavage needle into the mouse stomach. Urine was collected at 30 min, 1, 2, 3, 4, 6, and 8 h post-treatment. An equal volume of 10 µM internal standard (ZFH-2050) was added to the urine. Mannosides were extracted from the urine by loading on C18 columns (100 mg, Waters), washing with 30% methanol, and eluting with 60% methanol. Vacuum-concentrated eluates were analyzed using liquid chromatography-mass spectrometry system30 with a lower heated capillary temperature of 190°C and a gradient as follows: Solvent B (80% acetonitrile in 0.1% formic acid) was held constant at 5% for 5 minutes, increased to 44% B by 45 minutes, and then to a 95% B by 65 minutes. SRM mode quantification was performed with collision gas energy of 30% for the following MS/MS transitions (precursor m/z/product m/z): compound ZFH-2056, 447/285; compound ZFH-2050, 390/228. Absolute quantification was achieved by comparison to a calibration curve.

*Bladder tissue bacterial titer determination.* Mannoside ZFH-2056 was administered either IP (5 mg/kg) or orally (100 mg/kg) 30 min prior to inoculation with UTI89. To enumerate the bacteria present, mice were sacrificed at 6 hpi and bladders were aseptically removed and homogenized in 1 ml PBS, serially diluted and plated onto LB agar plates. CFU was enumerated after 16 h of growth at 37oC.

*Confocal microscopy.* Mannoside ZFH-2056 was administered IP (5 mg/kg) 30 min prior to inoculation with UTI89. To visualize bacterial behavior within the bladder, mice were sacrificed at 6 hpi and bladders were aseptically removed, bisected, splayed on silicone plates revealing the luminal surface and fixed in 2% paraformaldehyde (v/v). The splayed bladders were then incubated for 20 min at room temperature with (i) SYTO9 (1:1000 in PBS; Molecular Probes) to stain bacteria and (ii) Alexa Fluor 594-conjugated wheat germ agglutinin (WGA; 1:1000 in PBS; Molecular Probes) to stain the bladder luminal surface. Bladders were rinsed with PBS, mounted using Prolong Gold antifade reagent (Invitrogen) and examined with a Zeiss LSM510 confocal laser scanning microscope under a 63X objective. SYTO9 and WGA were excited at 488 and 594 nm, respectively.

*Gentamicin protection assay.* To enumerate bacteria present in the intracellular versus extracellular compartments, bladders were aseptically harvested at 6 hpi. The bladders were then bisected twice and washed three times in 500 µl of PBS each. The wash fractions were pooled, lightly spun at 500 rpm for 5 min to pellet exfoliated bladder cells, serially diluted, and plated onto LB agar to obtain the luminal fraction. The bladders were treated with 100 µg of gentamicin/ml for 90 min at 37°C. After treatment, the bladders were washed twice with PBS to eliminate residual gentamicin, homogenized in 1 ml of PBS, serially diluted, and plated onto LB agar to enumberate the CFUs in the intracellular fraction.

*Antibiotic treatment.* Mice were given TMP-SMZ in the drinking water at a concentration of 54 □g/ml and 270 µg/ml, respectively. Water was changed daily for 3 days prior to inoculation with UTI89. Mice remained on TMP-SMZ during the infection. To determine TMP-SMZ concentration in the urine, urine was collected after 3 days of TMP-SMZ treatment and quantified by LC-MS following addition of sulfisoxazole as an internal standard.

*Growth curve.* An overnight culture of PBC-1 was diluted 1:1000 in LB in the absence or presence of TMP-SMZ and/or mannoside ZFH-2056. The highest concentration of TMP-SMZ used was 512 µg/ml and 2560 □g/ml, respectively. Two-fold dilutions of TMP-SMZ were performed. Mannoside ZFH-2056 was added at 100 µM. Growth curves were performed in a 96-well plate at 37°C with A600 readings taken every 30 min for 8 h.

*Hemagglutination assay.* PBC-1 was grown statically in LB in the absence or presence of TMP-SMZ for 2x24 h at 37°C. The highest concentration of TMP-SMZ used was 256 µg/ml and 1280 µg/ml, respectively. Two-fold dilutions of TMP-SMZ were performed. Hemagglutination assays for mannose-sensitive agglutination of guinea pig red blood cells were performed as previously described31.

*Statistical analysis.* Observed differences in bacterial titers and IBC numbers were analyzed for significance using the nonparametric Mann-Whitney U test (Prizm; GraphPad Software).

### Example 15. Improving solubility and potency of mannosides.

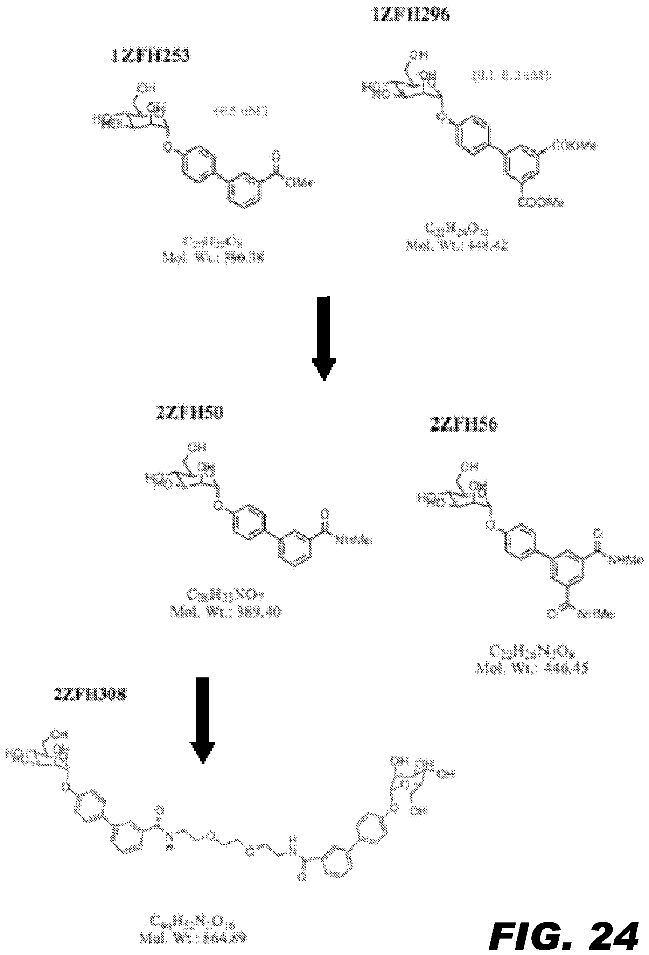

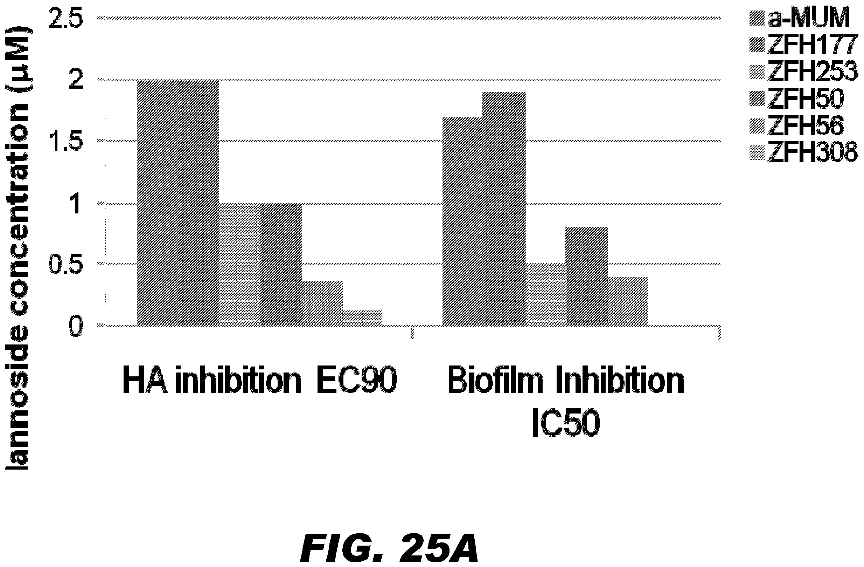

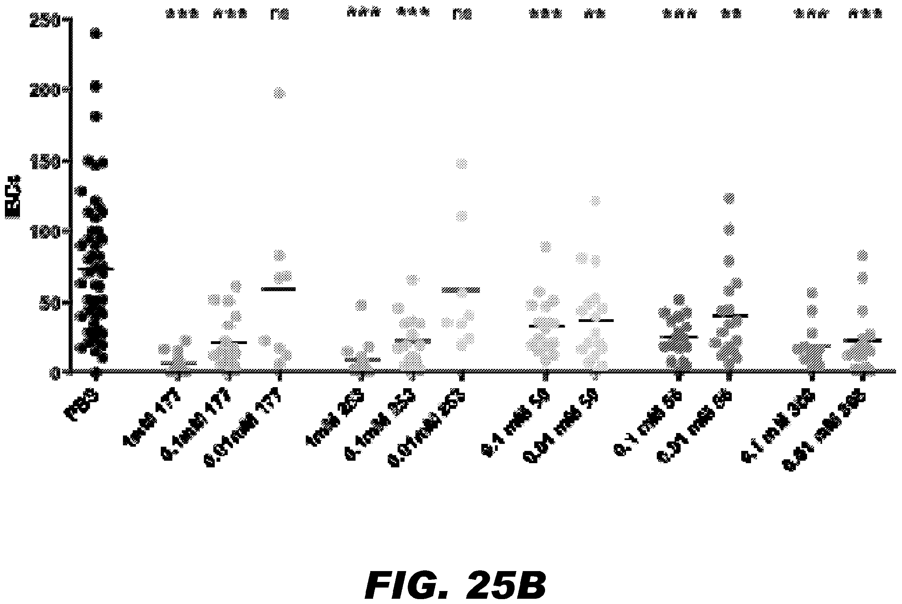

Amide groups were added to the insoluble 1ZFH253 and 1ZFH296 mannosides to produce mannoside compounds with increased solubility as shown in the **Fig. 24****.** A divalent compound was also produced using 2ZFH50. The efficacy of these compounds along with 1ZFH177 was tested in an *in vitro* inhibition of hemagglutination and biofilm assays (**Fig. 25**). These results demonstrate improved in vitro potency of mannosides comprising enhanced solubility.

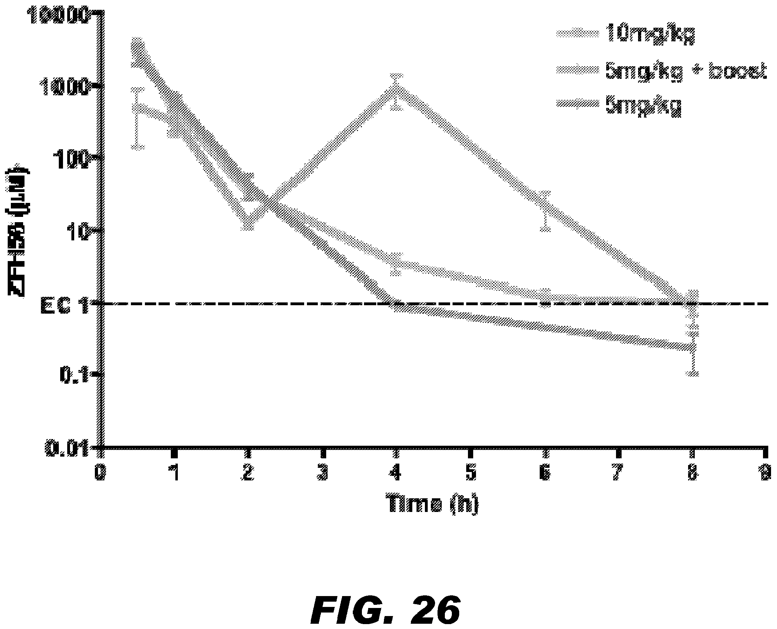

ZFH56 was injected IP into mice at various doses and levels of the compound were measured in the animal (**Fig. 26**). 10 mg/kg, 5 mg/kg or 5 mg/kg followed by a 4 hour boost were injected into animals.

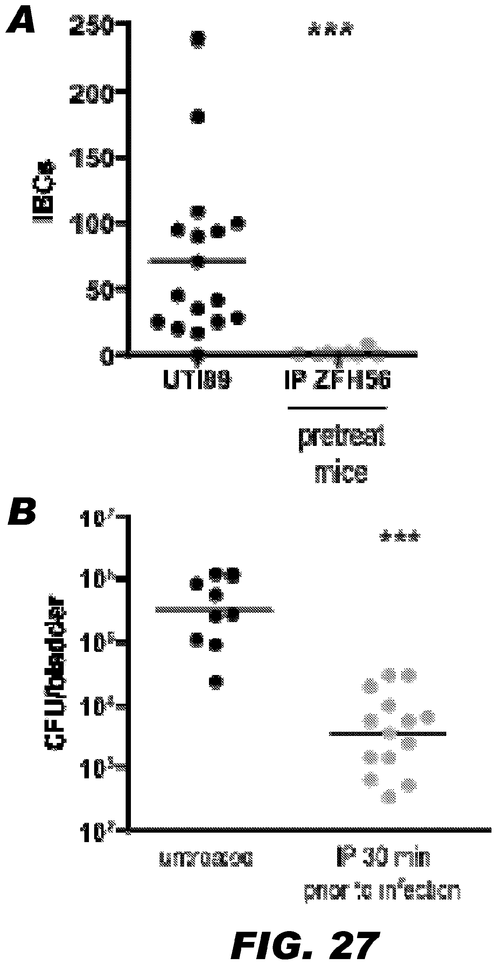

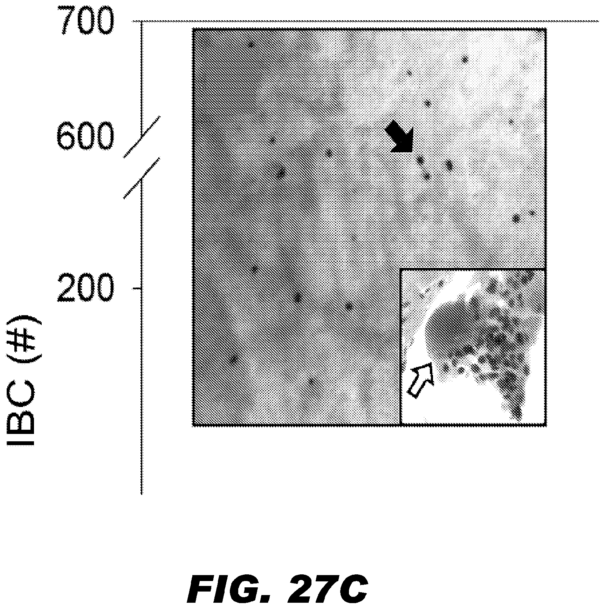

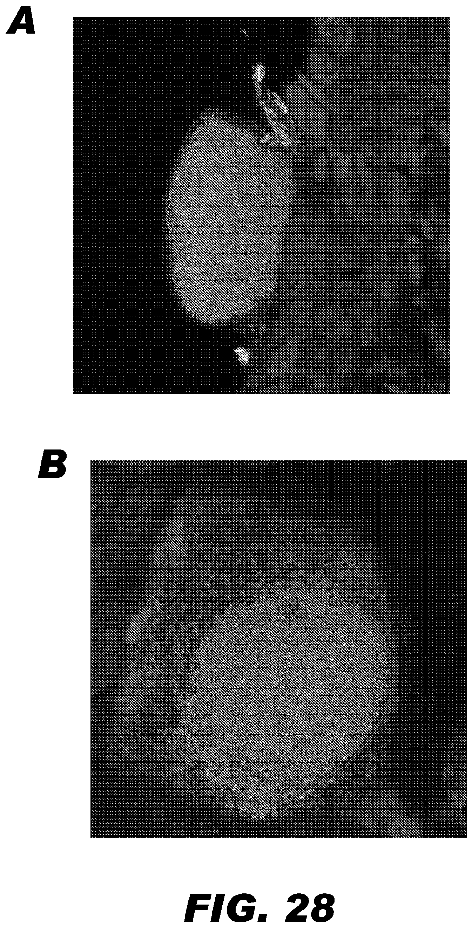

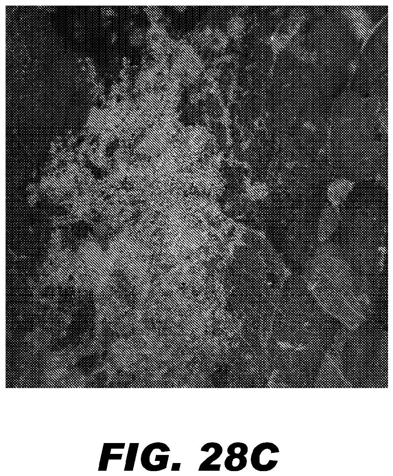

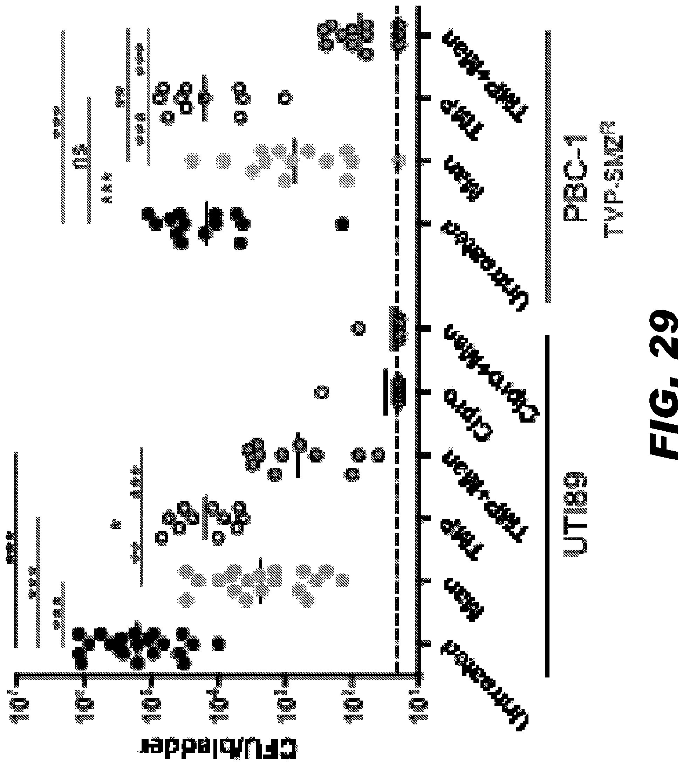

IBC numbers were also determined in animals pretreated with ZFH56 (**Fig. 27**). ZFH56 also altered the pathogenic pathway as measured by confocal microscopy (**Fig. 28**). ZFH56 also showed increased efficacy against TMP-SMZ resistant strains (**Fig. 29**).

### Example 16. Testing against various inoculum levels.

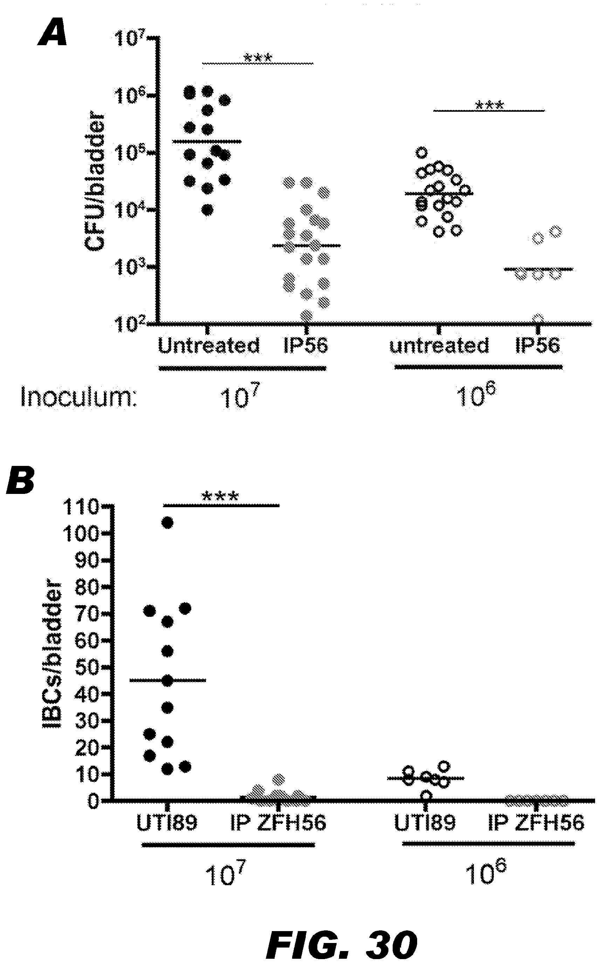

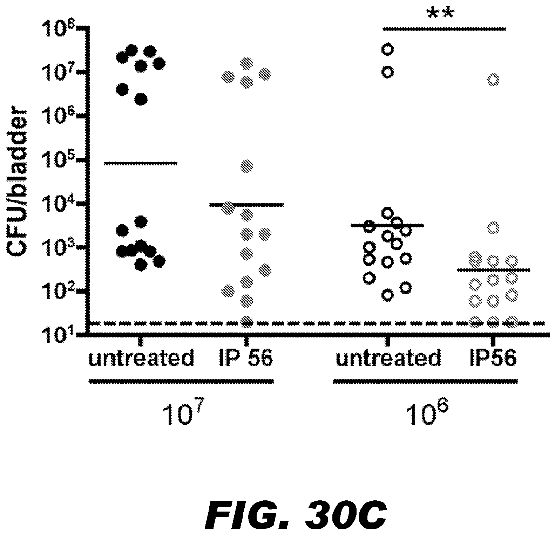

An inoculum of 10⁷ is likely well above the physiological dose typically found in a human. It is likely that only a few bacteria are inoculated into the urinary tract for a successful infection. Thus, a lower dose of bacteria was introduced to the mouse and determined whether this increased the efficacy of mannoside ZFH56 (**Fig. 30**). In order to obtain a consistent infection in the mouse model of UTI the dose could only be lowered by 10 fold. With an inoculum of UTI89 of 10⁶, there was a significant decrease (p<0.0001) in bacterial titers in the ZFH56 treated mice compared to the untreated mice. However, the level of infection in the mannoside treated mice at both the 10⁷ and 10⁶ inoculum was approximately equivalent suggesting that the lower dose did not further enhance efficacy of the mannoside. Using IBC counts in the bladder at the lower dose there were no IBCs observed in the ZFH56 treated mice at the 10⁶ inoculum. However the overall number of IBCs in the untreated mice were also much lower. Since there were no IBCs at 6h in the 10⁶ inoculum, it was hypothesized that the level of infection at 2 weeks post-infection would be significantly lower. Evaluation of 2 week titers revealed a significant decrease in bacterial load in the mannoside treated bladders with the 10⁶ inoculum. This data suggests that upon lowering the inoculum, mannoside is able to significantly reduce colonization at 2 wpi with a single dose of 5mg/kg ZFH56 30 min prior to inoculation of UTI89.

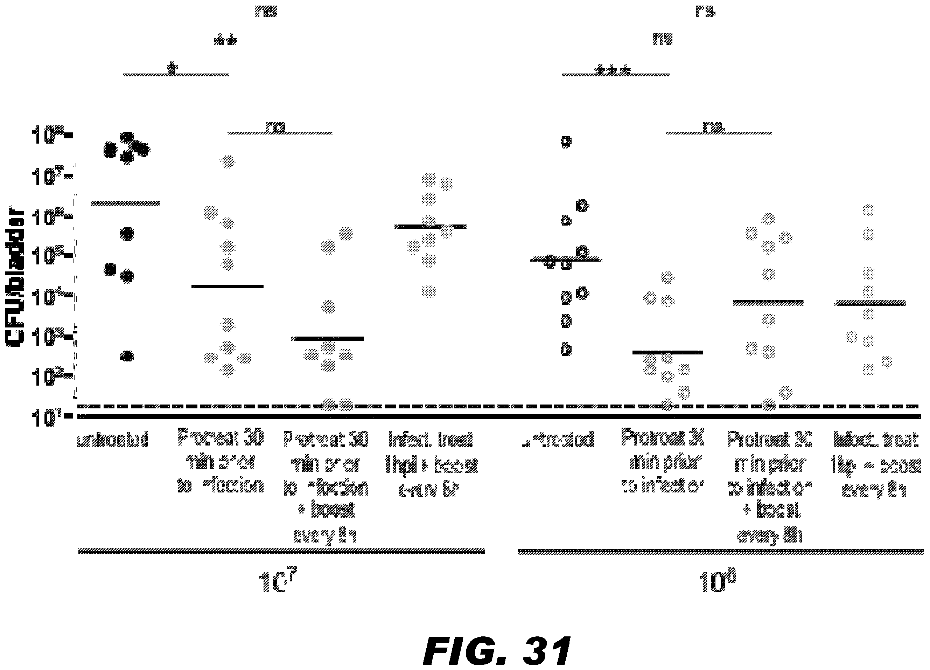

PK studies with IP dosing revealed that after injection, mannoside ZFH56 is rapidly eliminated in the urine and is mostly gone by 6-8h post injection. Thus the mice were boosted with 5mg/kg mannoside ZFH56 every 8h to maintain a constant presence of mannoside in the mouse. This experiment comprised 4 groups: (1) untreated, (2) pretreat by IP injection of 5mg/kg ZFH56 30 min prior to inoculation with UTI89, (3) pretreat by IP injection of 5mg/kg ZFH56 30 min prior to inoculation plus 2 additional treatments every 8h, and (4) inoculation with UTI89 followed by IP treatment with 5mg/kg ZFH56 1h post-infection and 2 additional treatments every 8h. Mice were harvested at 24hpi. This was done with the 10⁷ and 10⁶ inoculum of UTI89. There was a significant drop in bacterial titers with the single pretreatment of ZFH56 with both inoculum doses. In the mice inoculated with 10⁷ UTI89 there was further efficacy of ZFH56 upon boosting. However, this same effect was not observed with the 10⁶ inoculum. With both inoculums there was no efficacy observed upon post-infection treatment with ZFH56. These data suggest that mannoside is currently most efficacious when used prophylactically (**Fig. 31**).

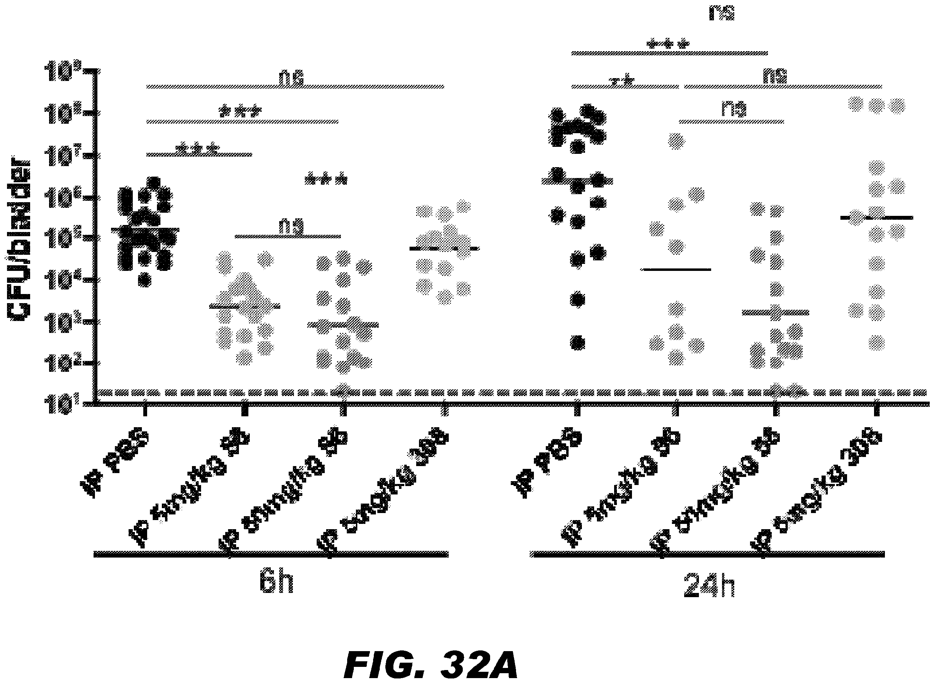

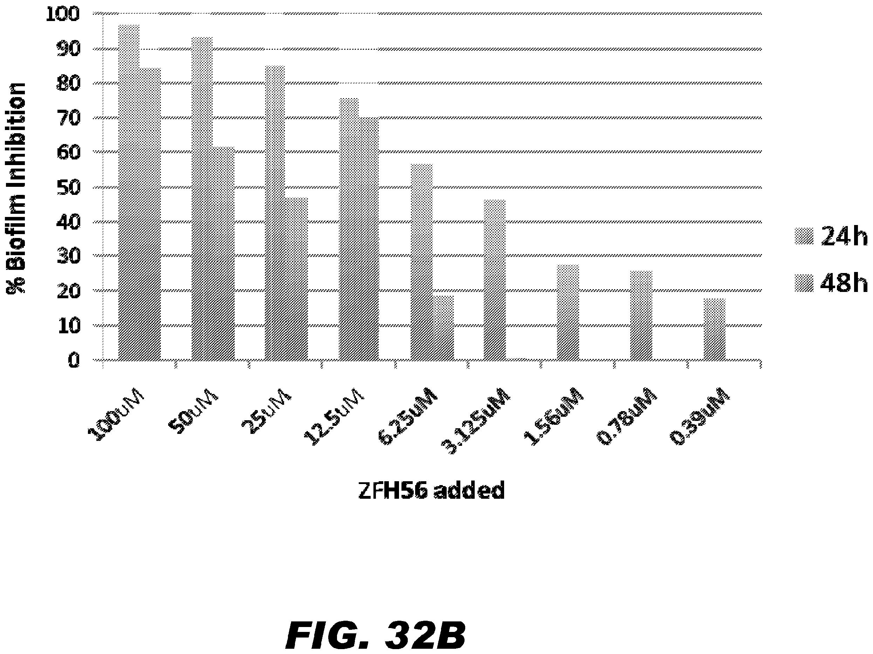

Next, it was determined if a 10 fold increase in ZFH56 would increase efficacy and if the divalent ZFH308 was as efficacious as ZFH56 at 5mg/kg (**Fig. 32**). Mice were treated with 5mg/kg ZFH56, 50mg/kg ZFH56 or 5mg/kg ZFH308 30 min prior to infection with 10⁷ UTI89. At 6 and 24 hpi bladders were harvested and titered. At 6 hpi, 5mg/kg ZFH56 and 50mg/kg ZFH56 both significantly inhibited bacterial infection. However, 50mg/kg ZFH56 did not significantly enhance efficacy over the 5mg/kg ZFH56 dose. 5mg/kg ZFH308 did not significantly reduce bacterial infection at 6 hpi. At 24 hpi, both 5mg/kg and 50mg/kg ZFH56 significantly reduced bacterial titers. 50mg/kg ZFH56 showed slight enhancement over 5mg/kg ZFH56 relative to untreated mice. Again ZFH308 did not show efficacy in reducing infection. Thus, 5mg/kg ZFH56 seems to be the optimal dose for inhibiting bacterial infection and the divalent ZFH308 does not show enhanced efficacy over the monovalent compound.

### Example 17. ZFH56 disrupts preformed biofilm.

UTI89 was grown in LB in PVC plates and allowed to form biofilm at RT for 24 or 48h. At 24 or 48h, varying concentrations of mannoside ZFH56 was added. Biofilm was then allowed to grow for another 24h at RT. Biofilm formation was then quantitated using the standard crystal violet assay. Results showed that ZFH56 was able to disrupt preformed biofilm (**Fig. 32**). At 24h the IC50 for biofilm disruption was ∼6uM and at 48h the IC50 was ∼25uM. This shows that not only can mannoside inhibit biofilm formation it can dissolve biofilm that has already formed.

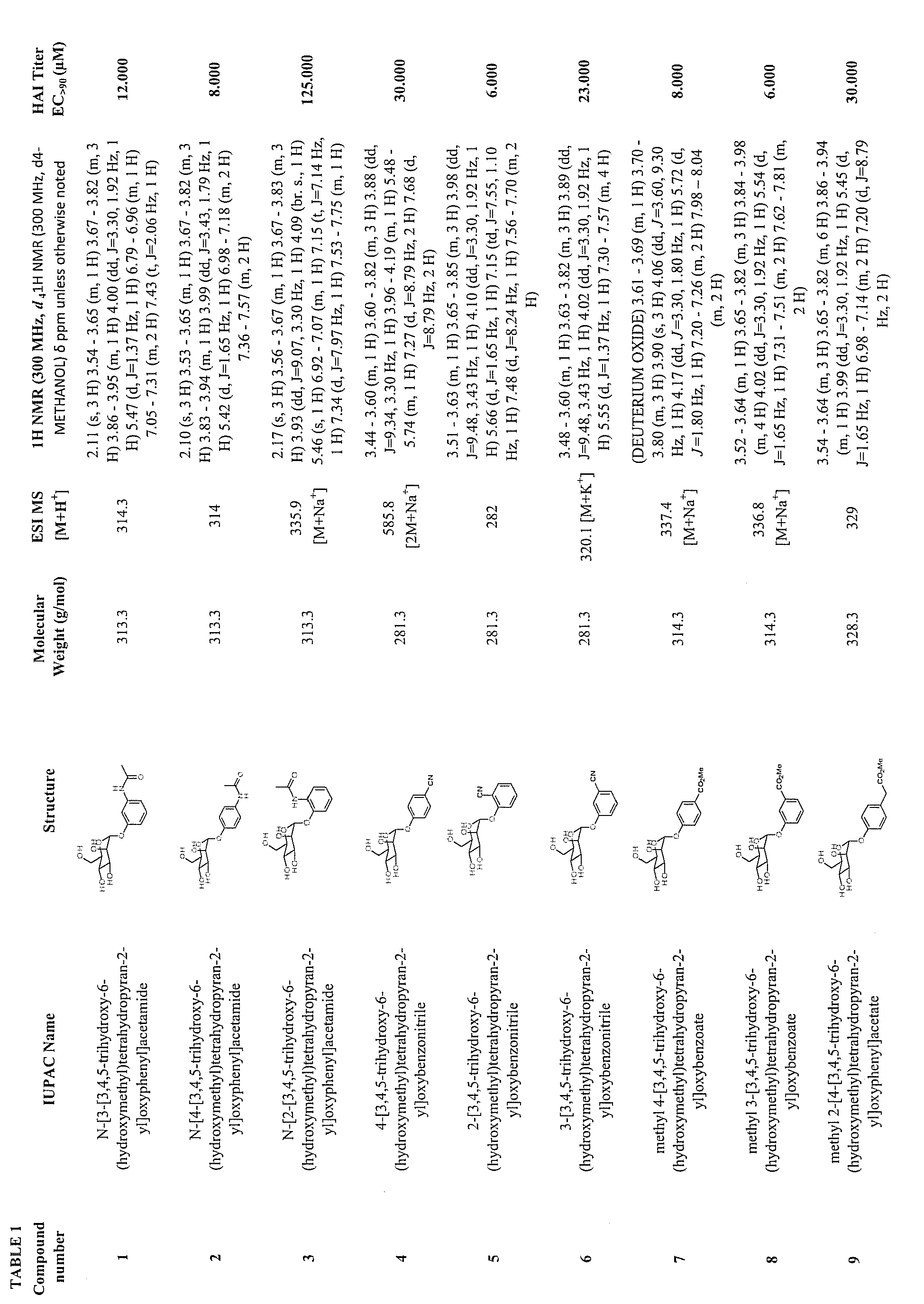

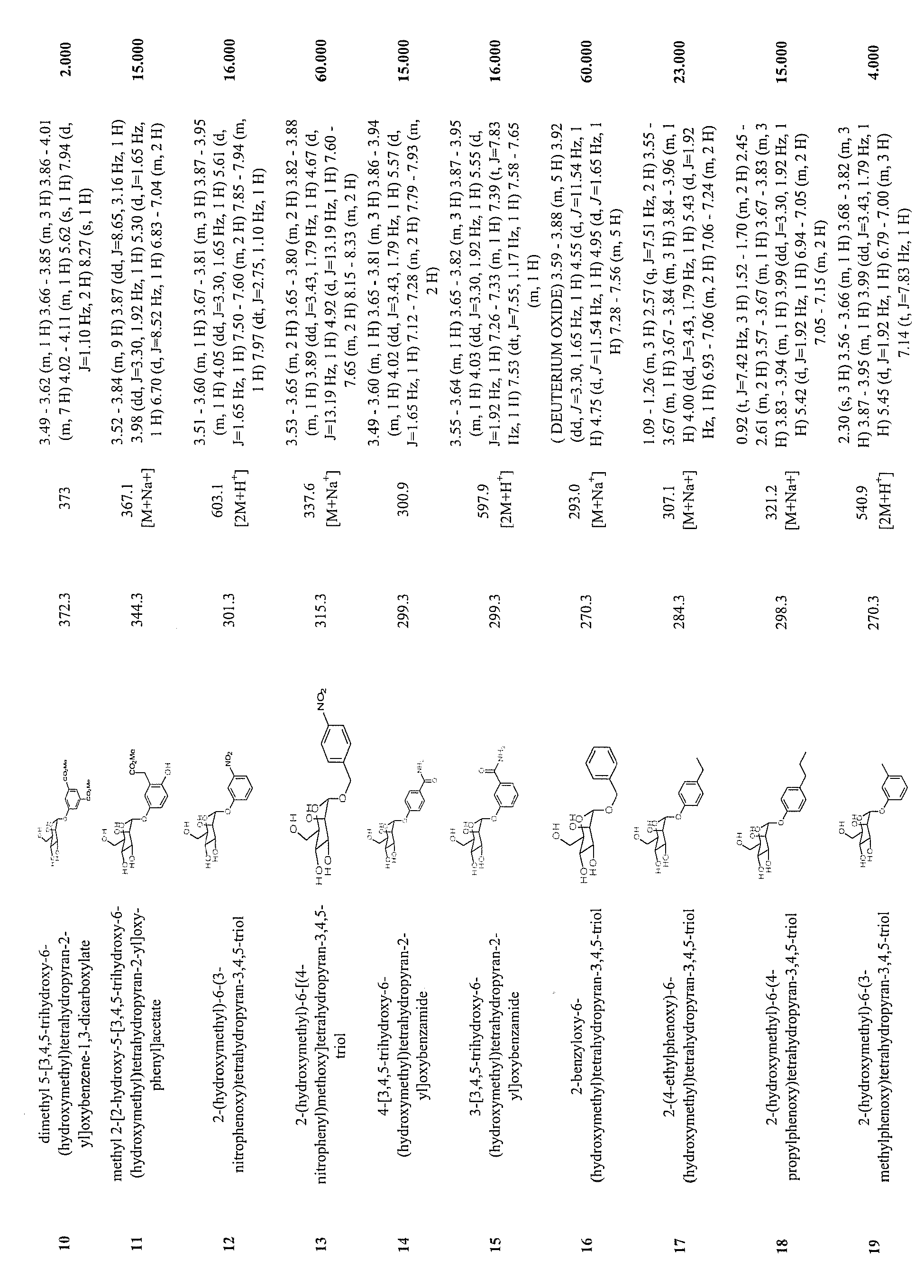

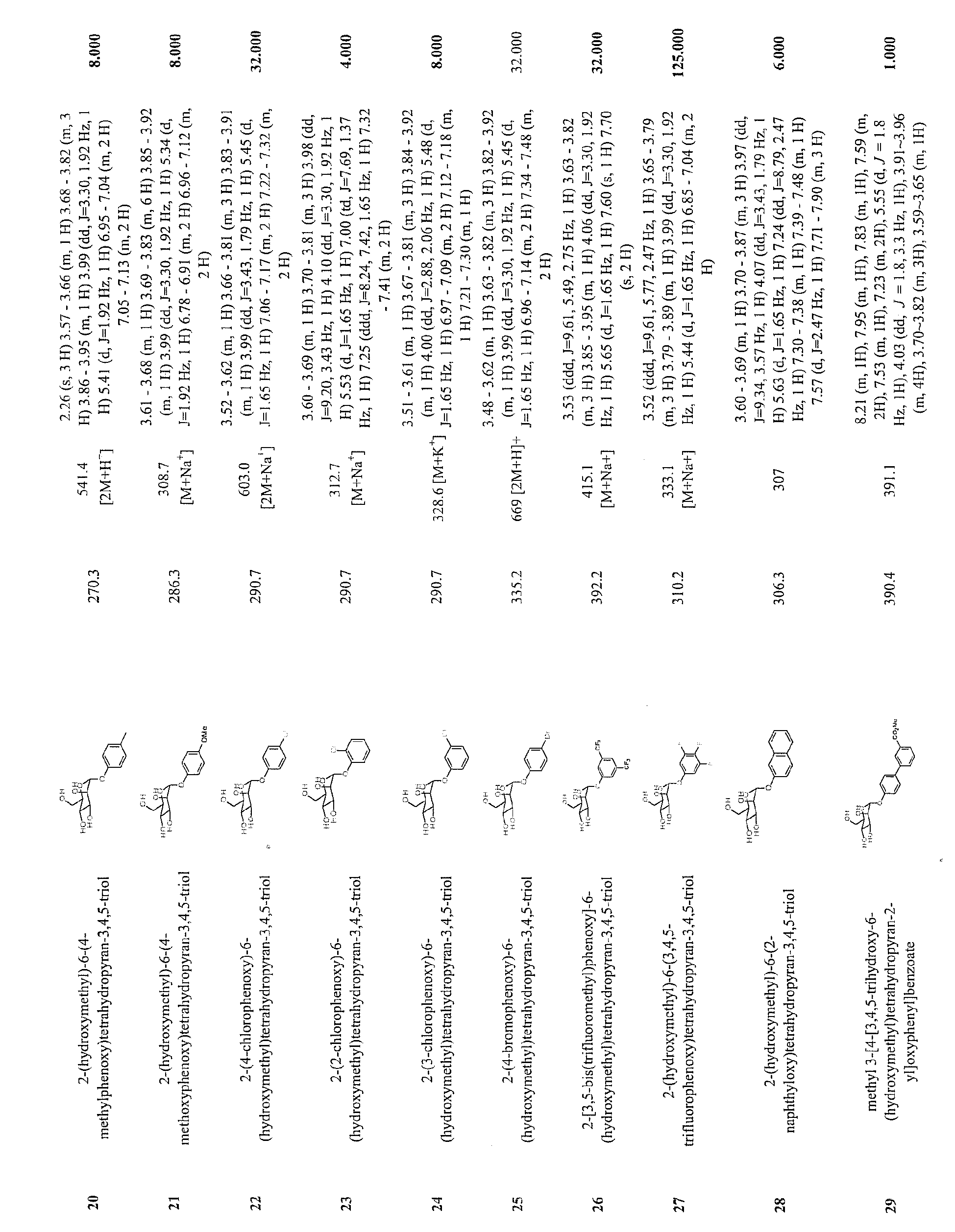

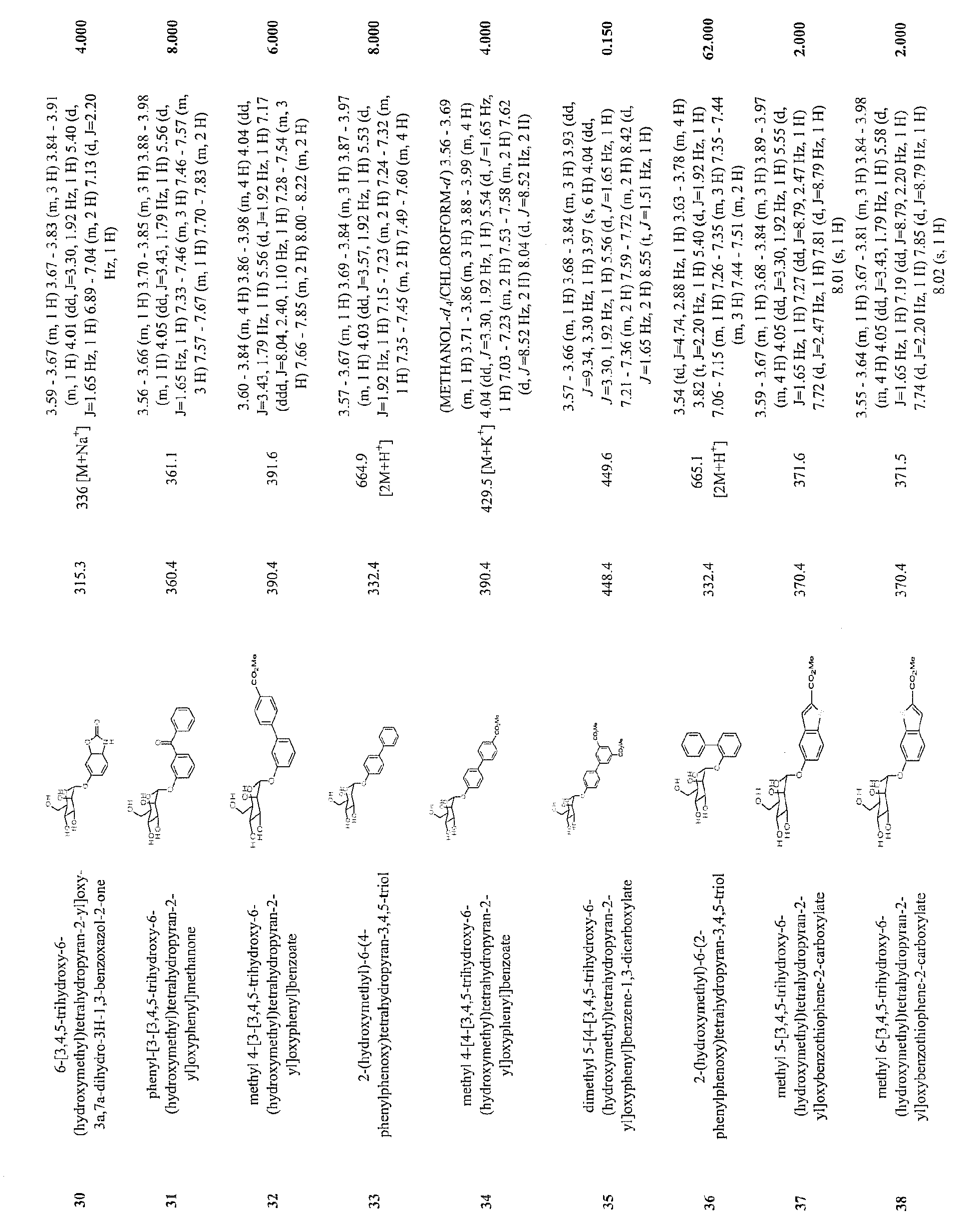

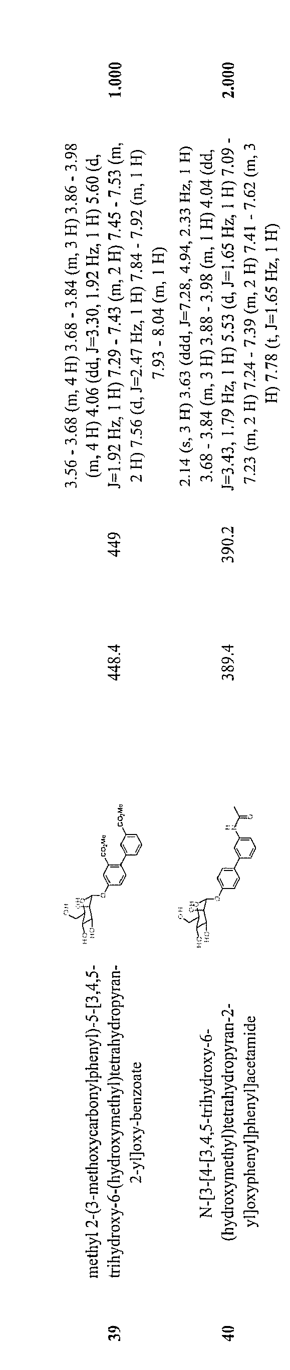

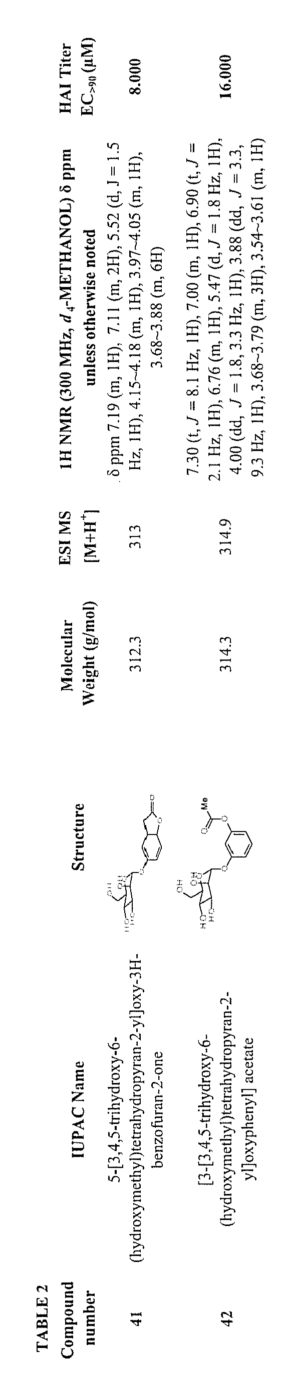

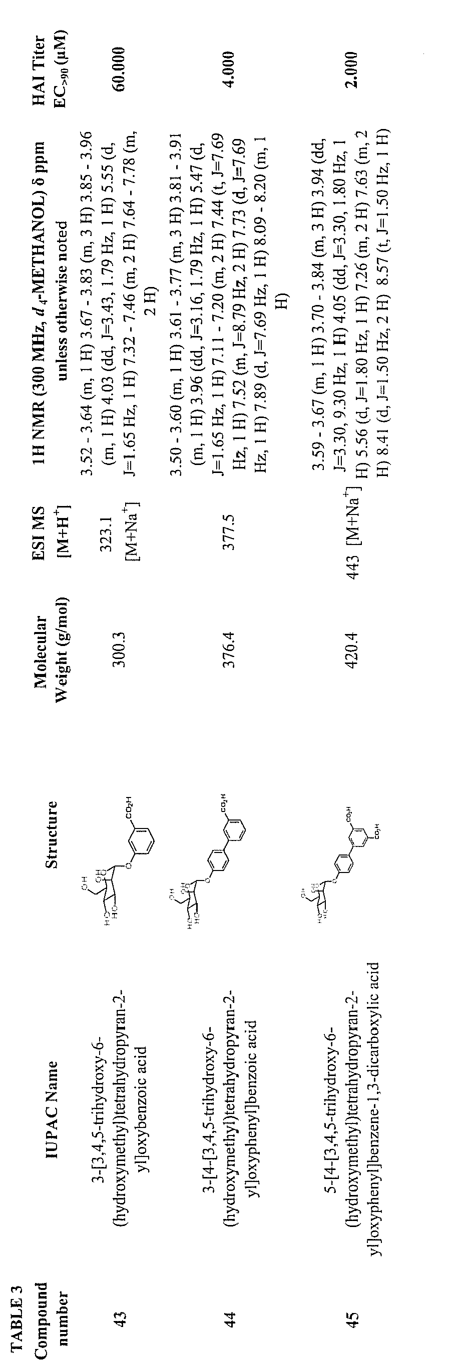

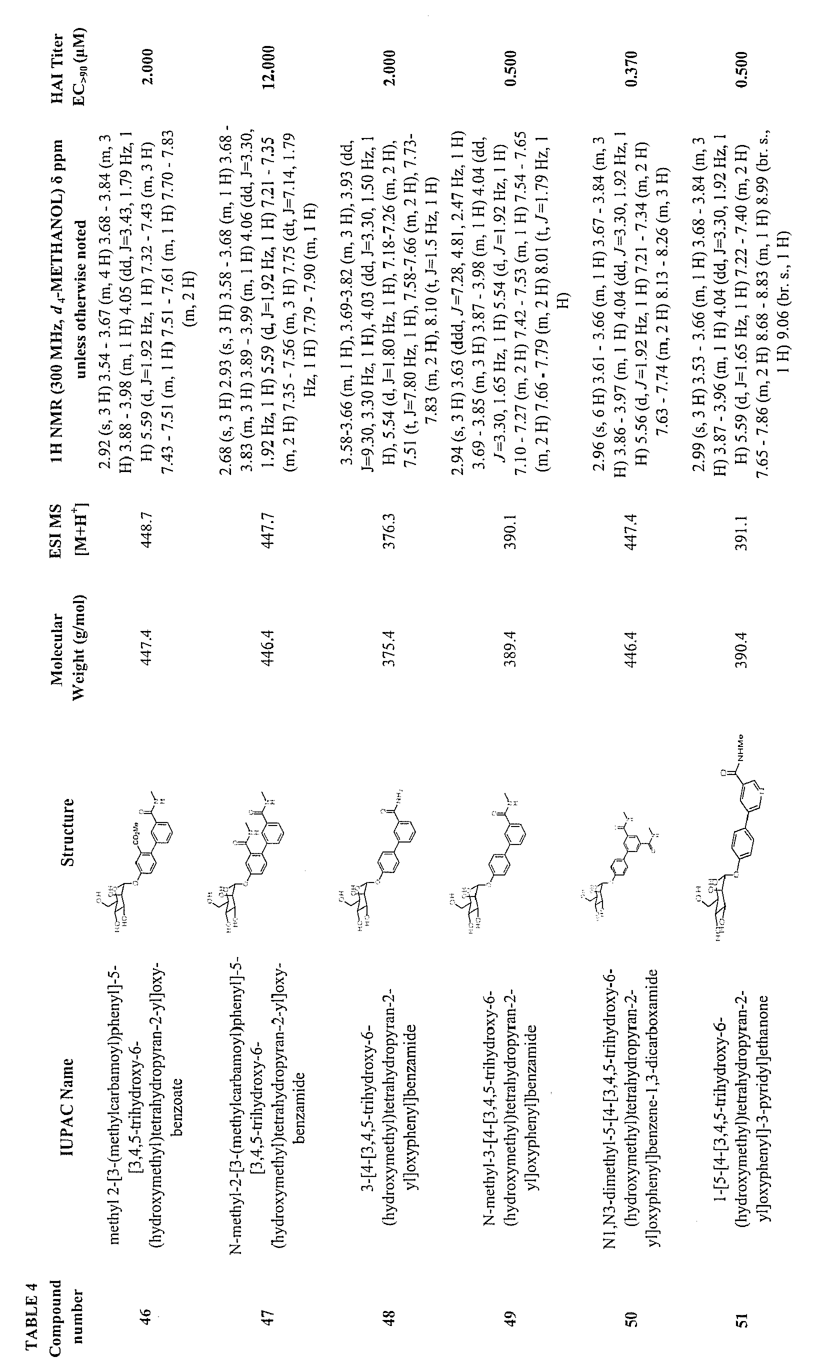

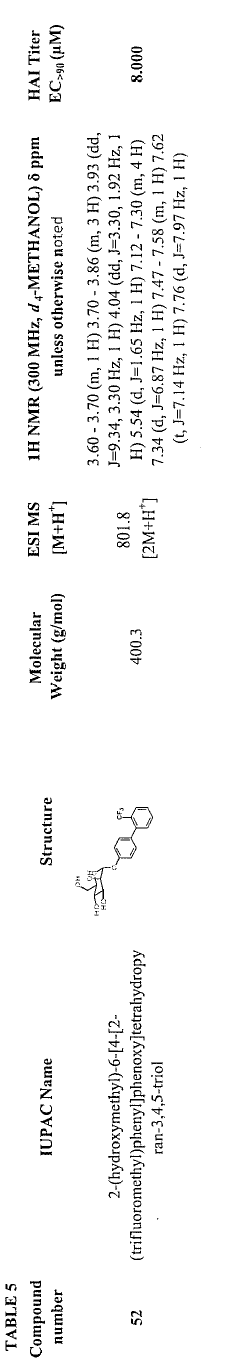

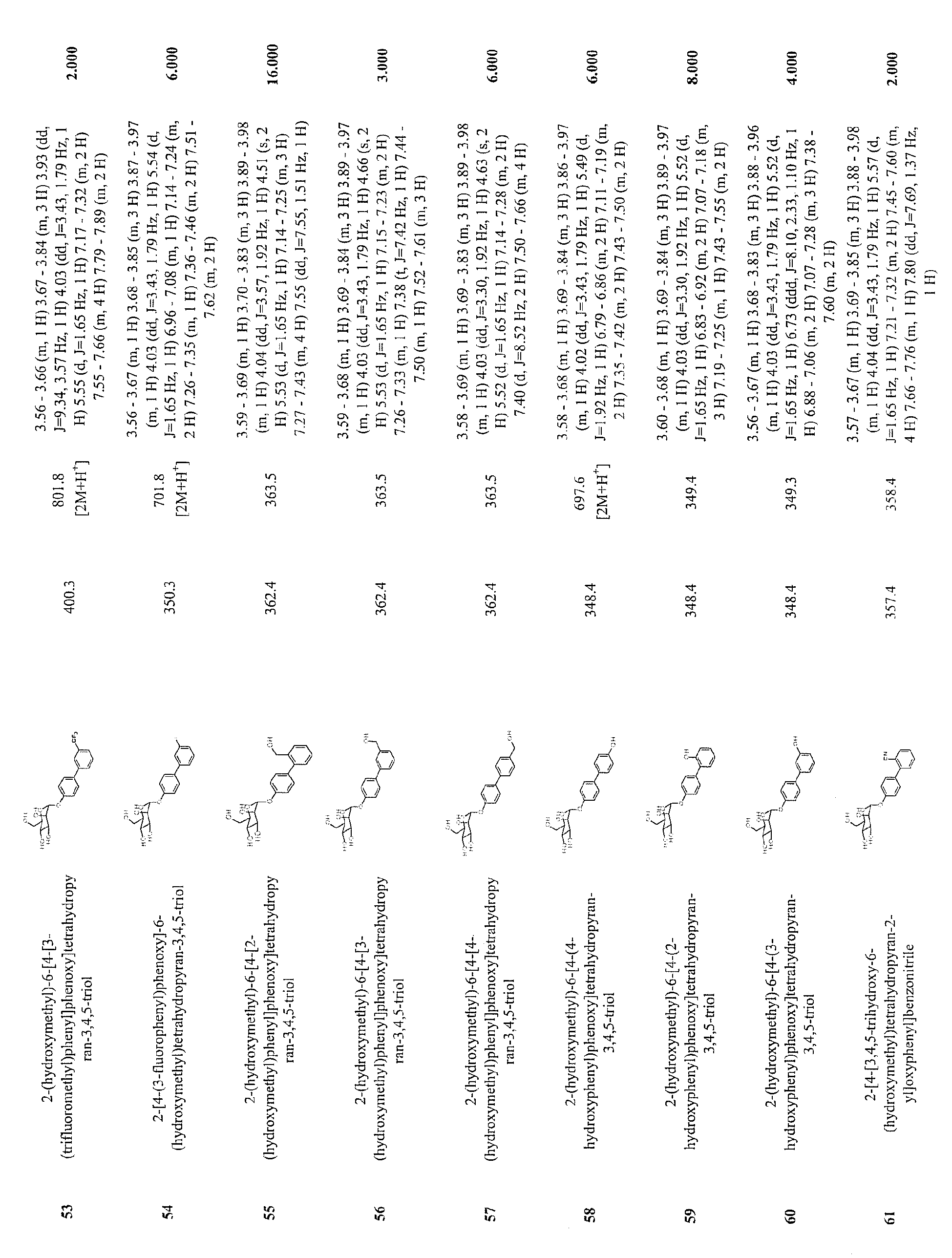

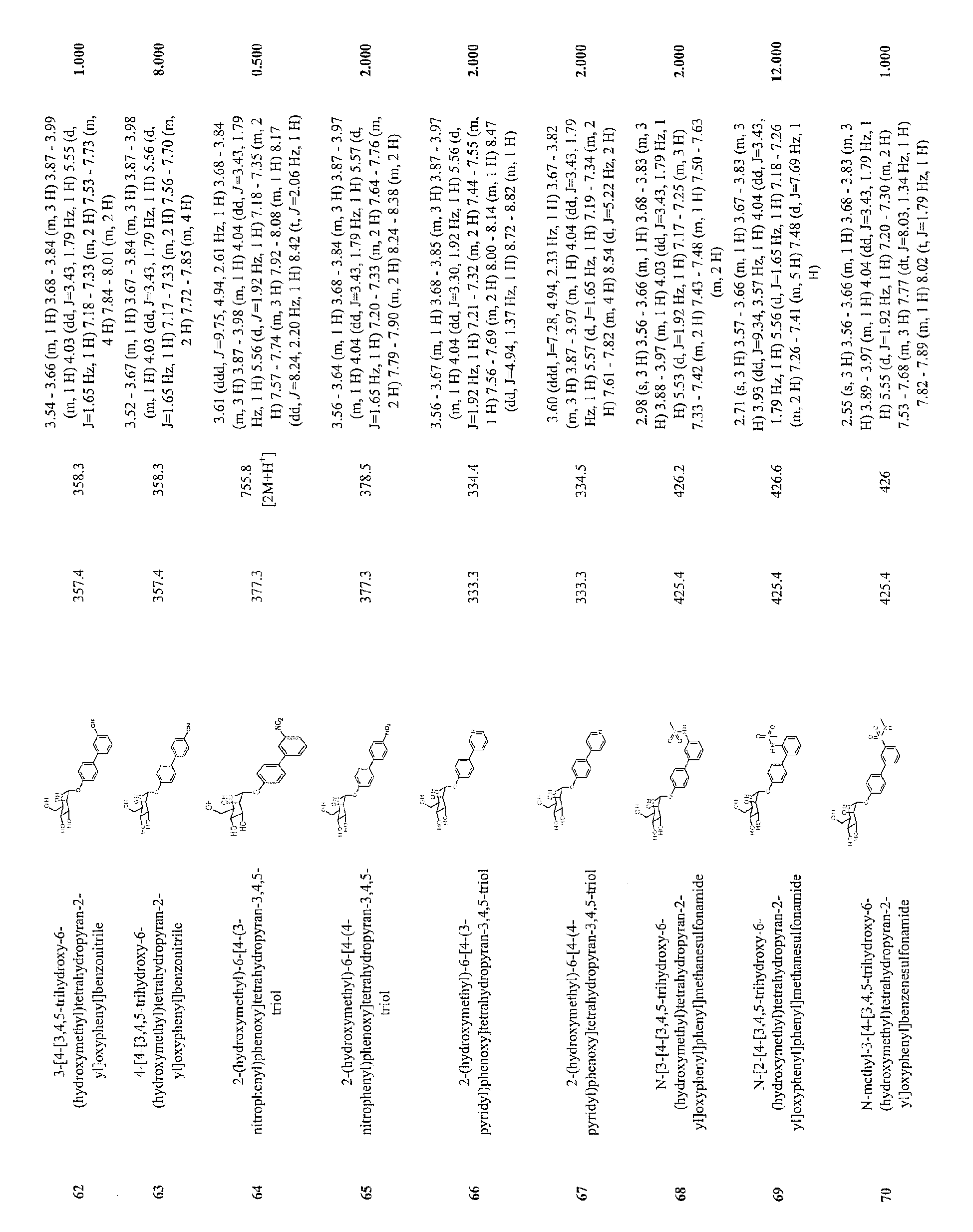

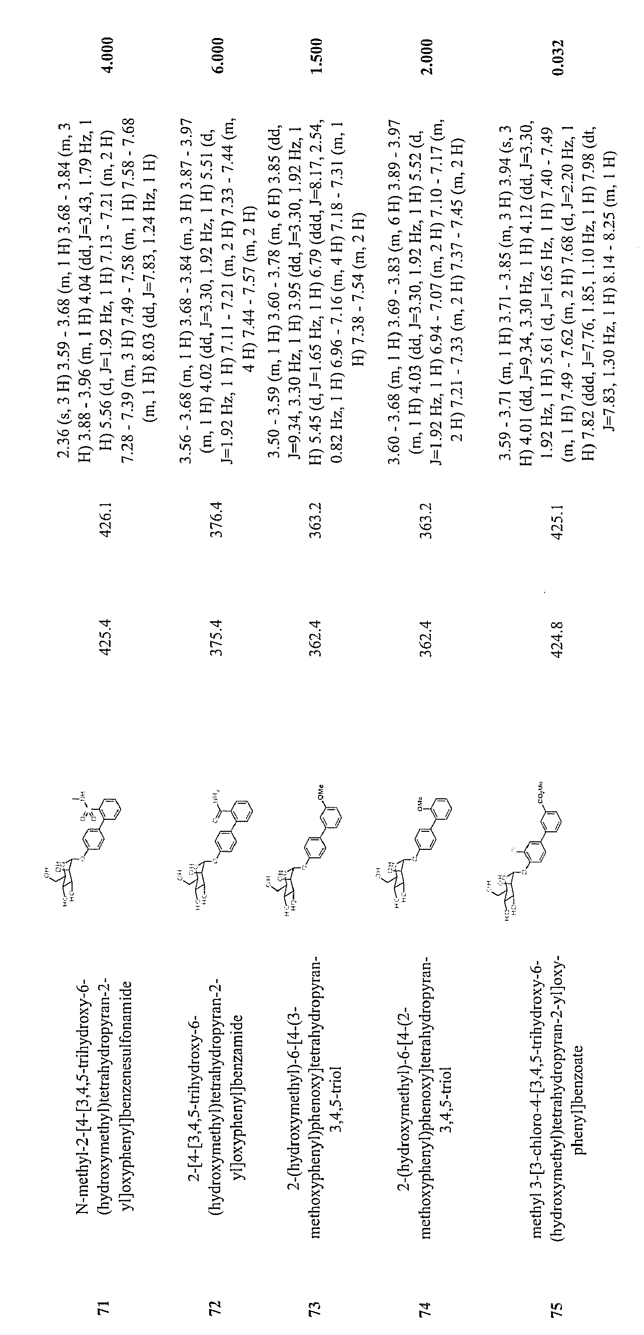

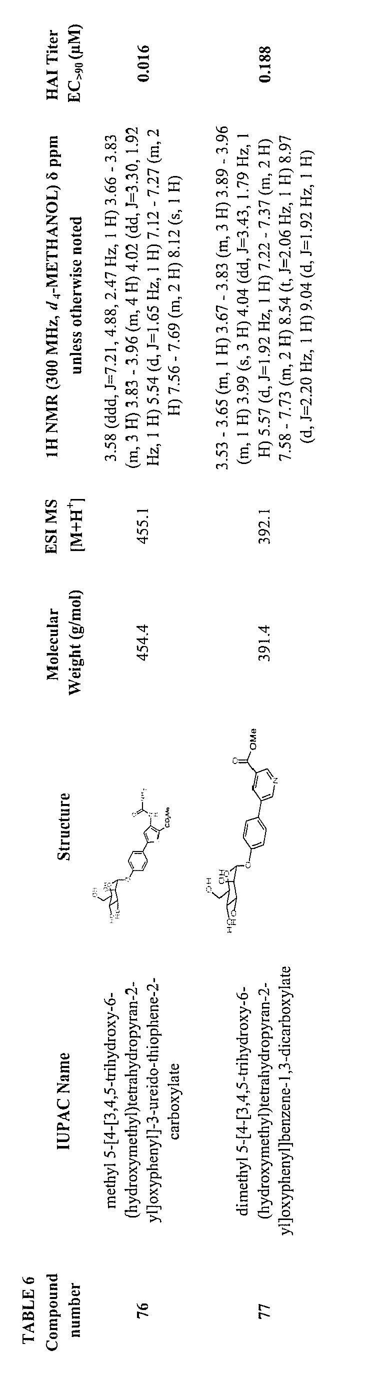

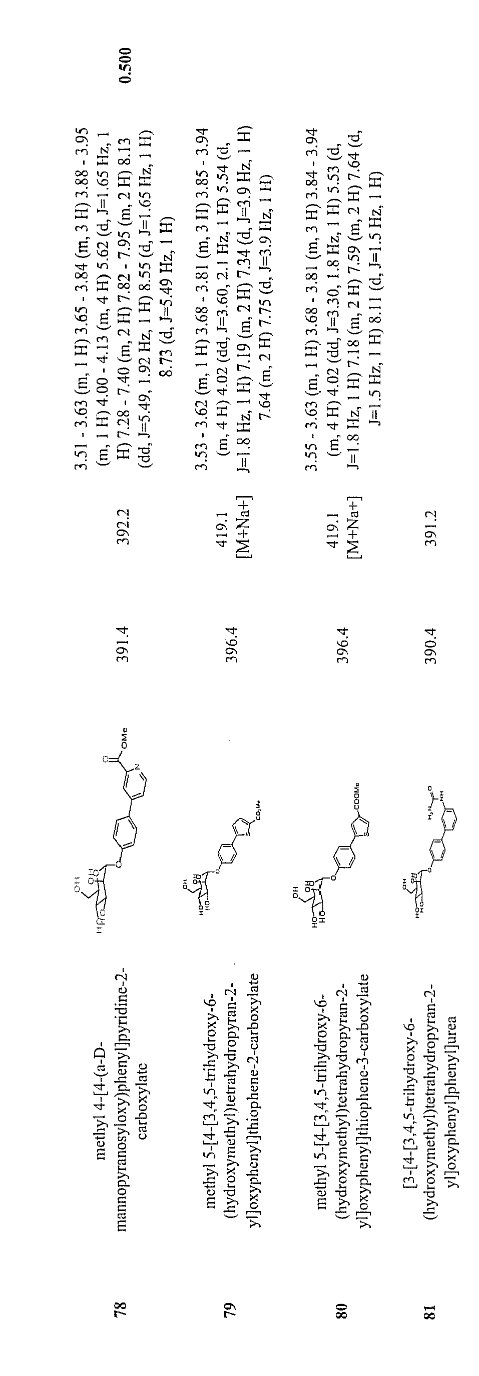

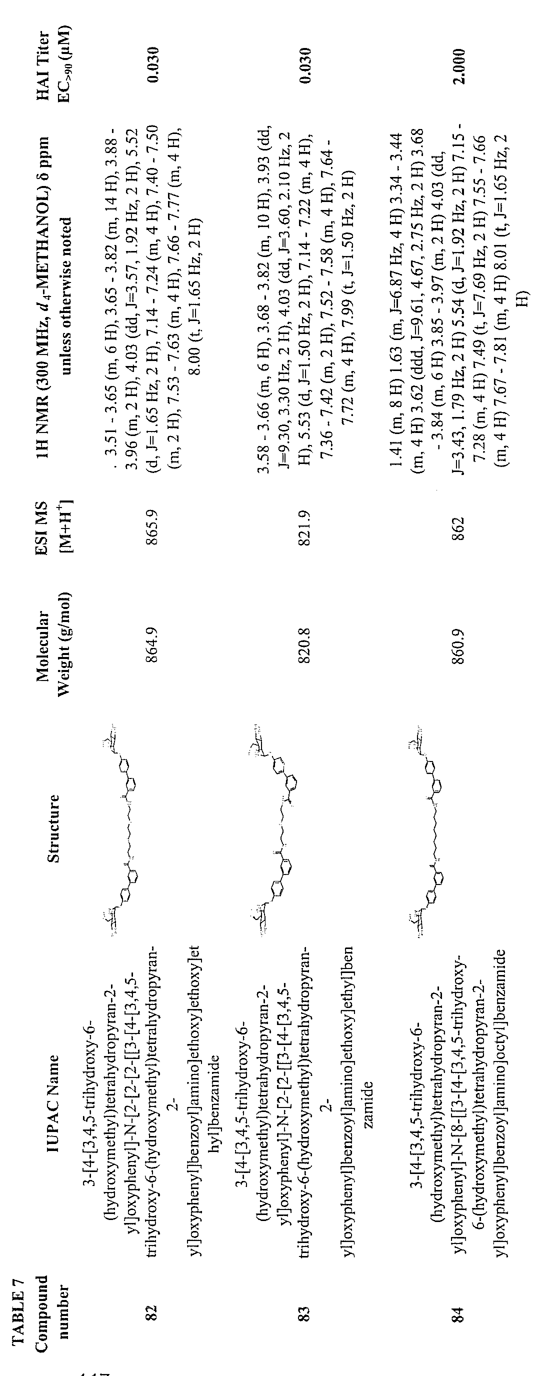

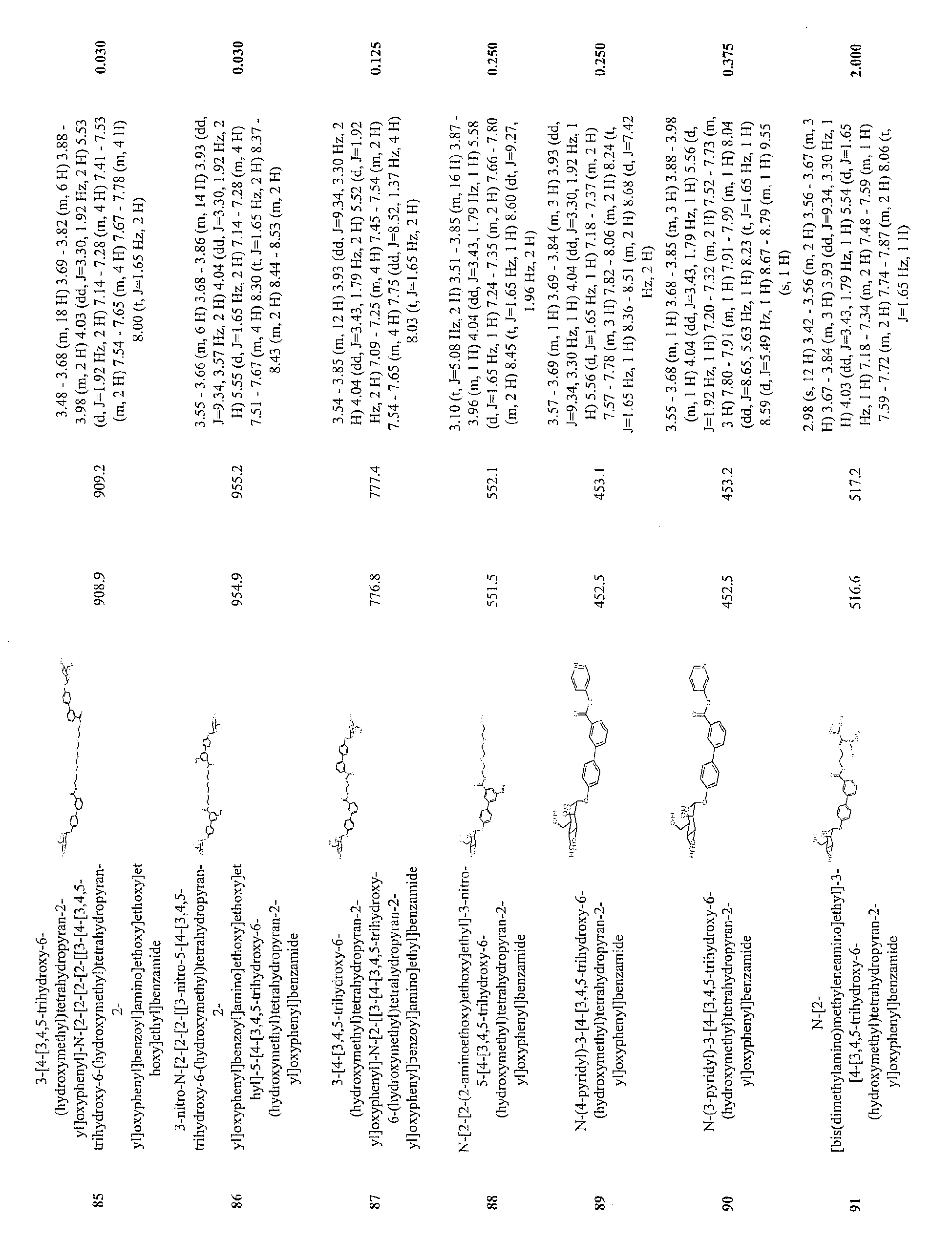

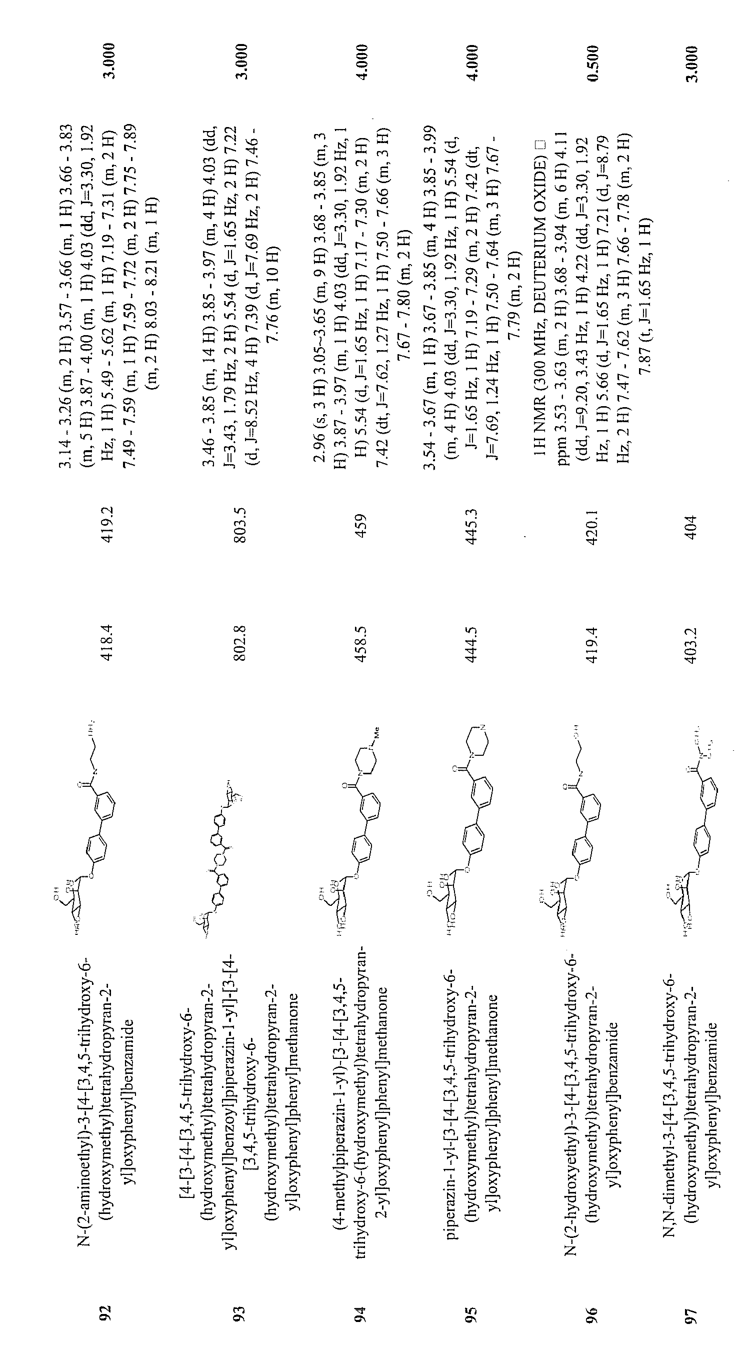

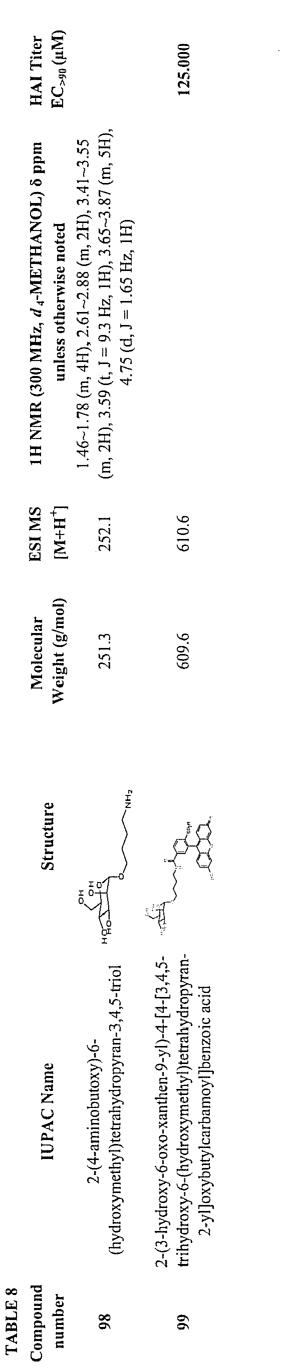

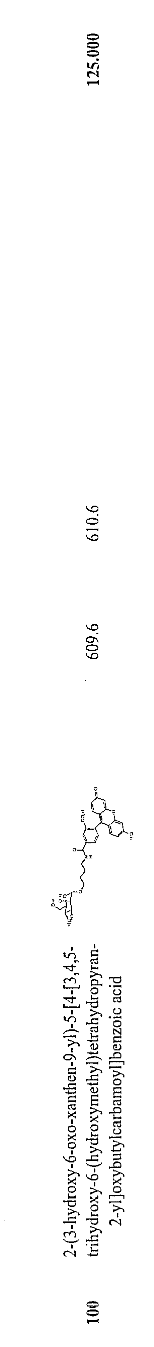

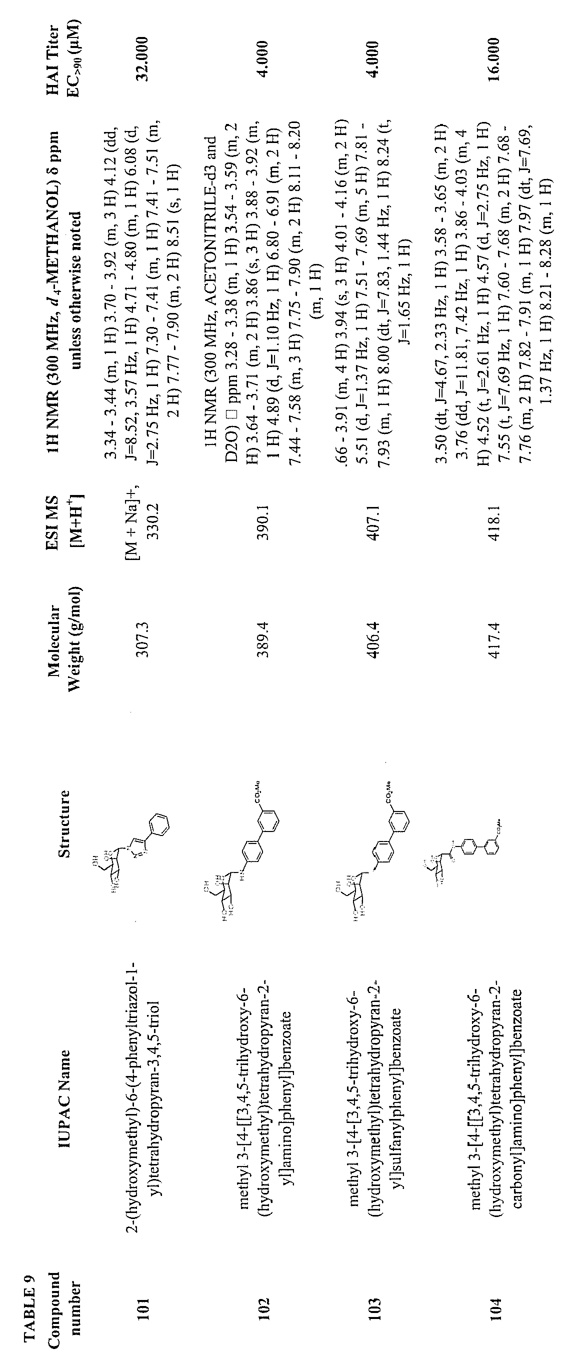

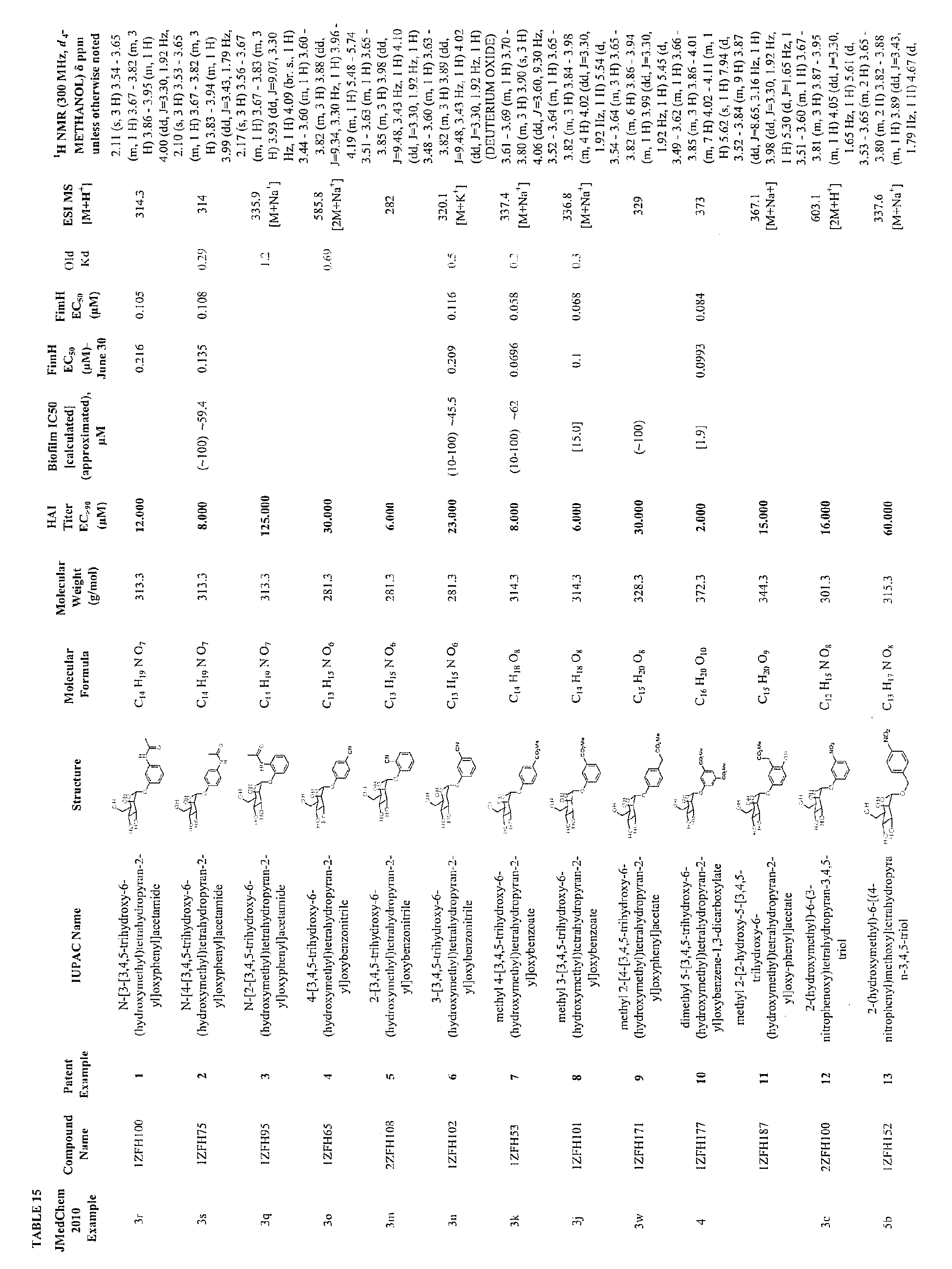

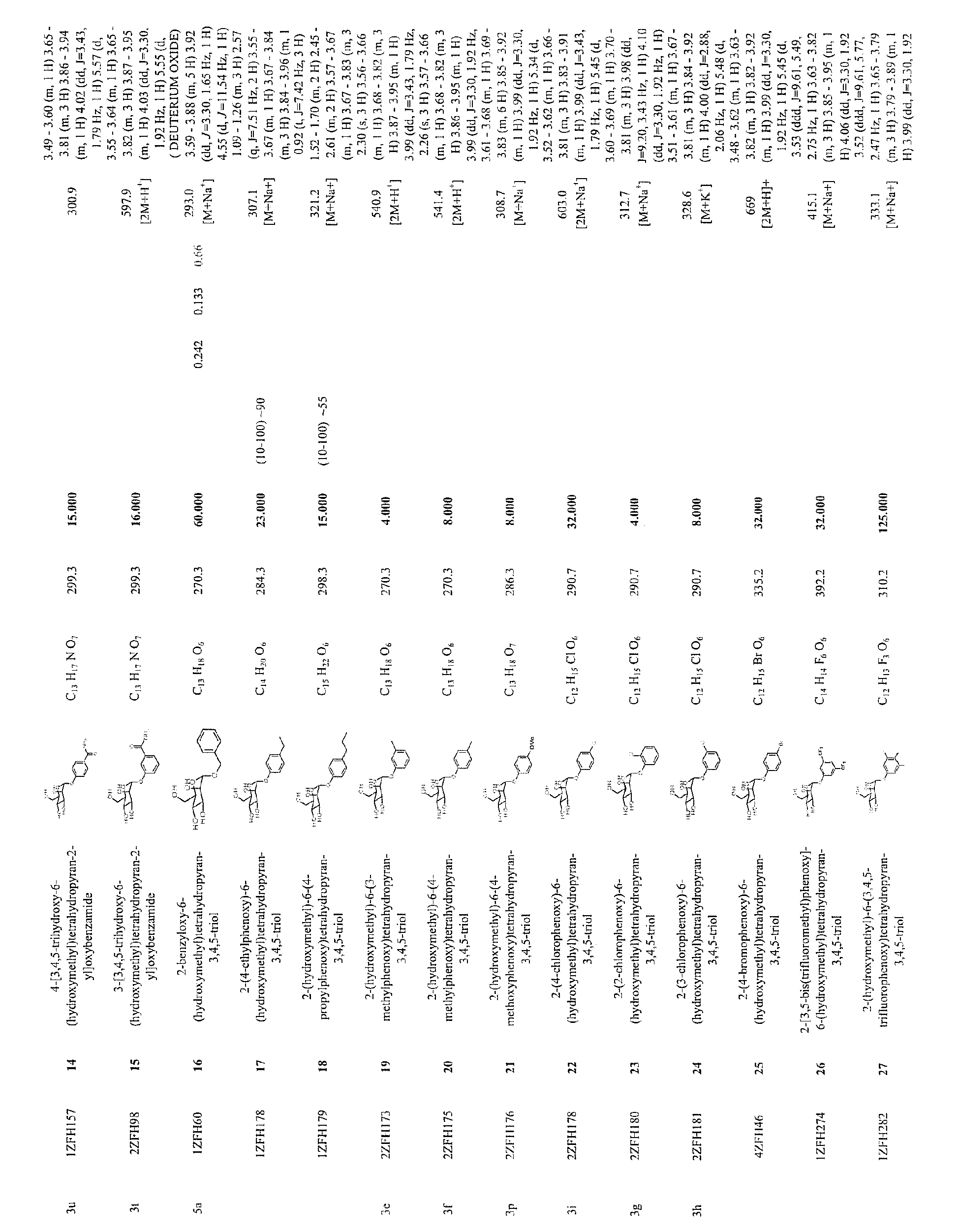

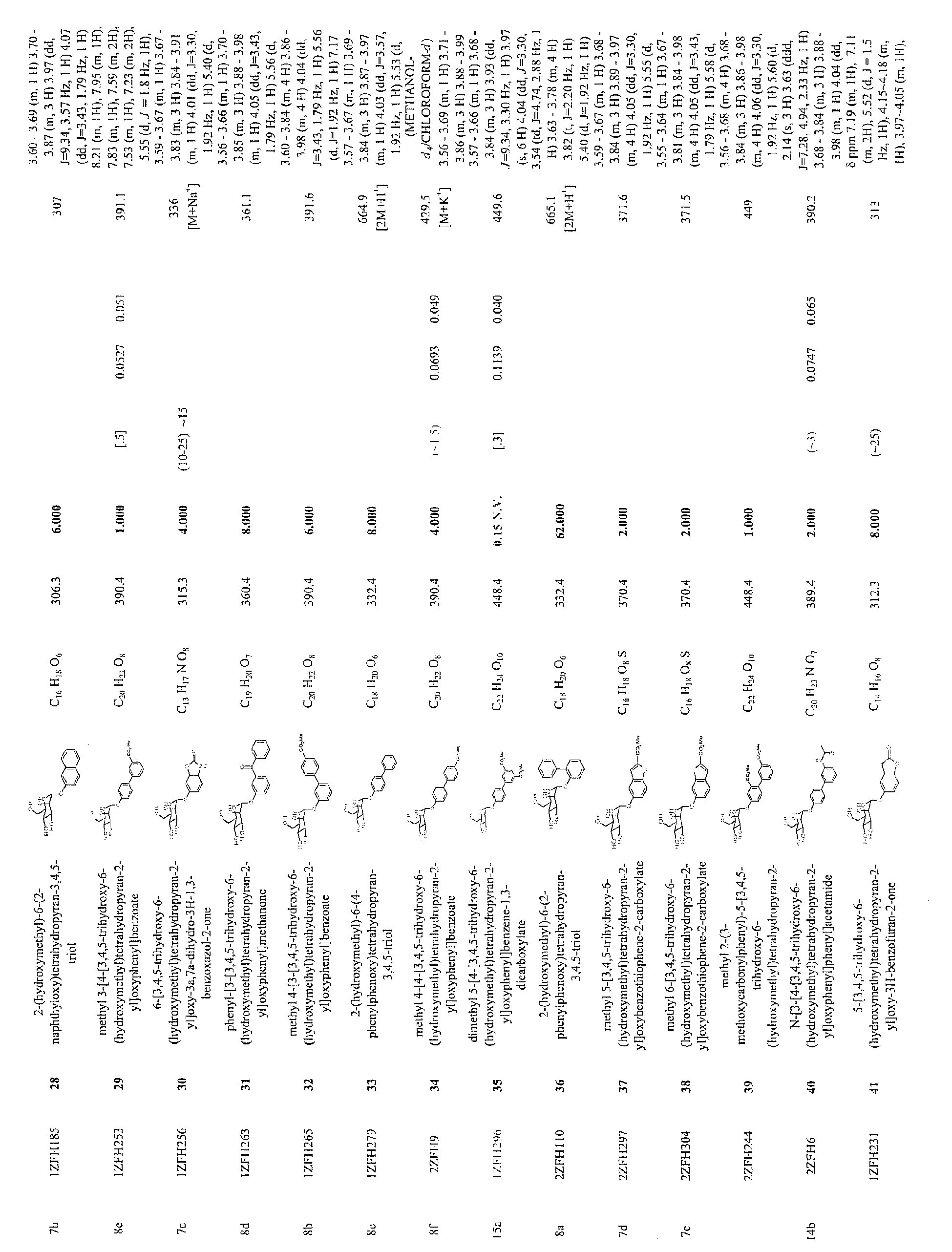

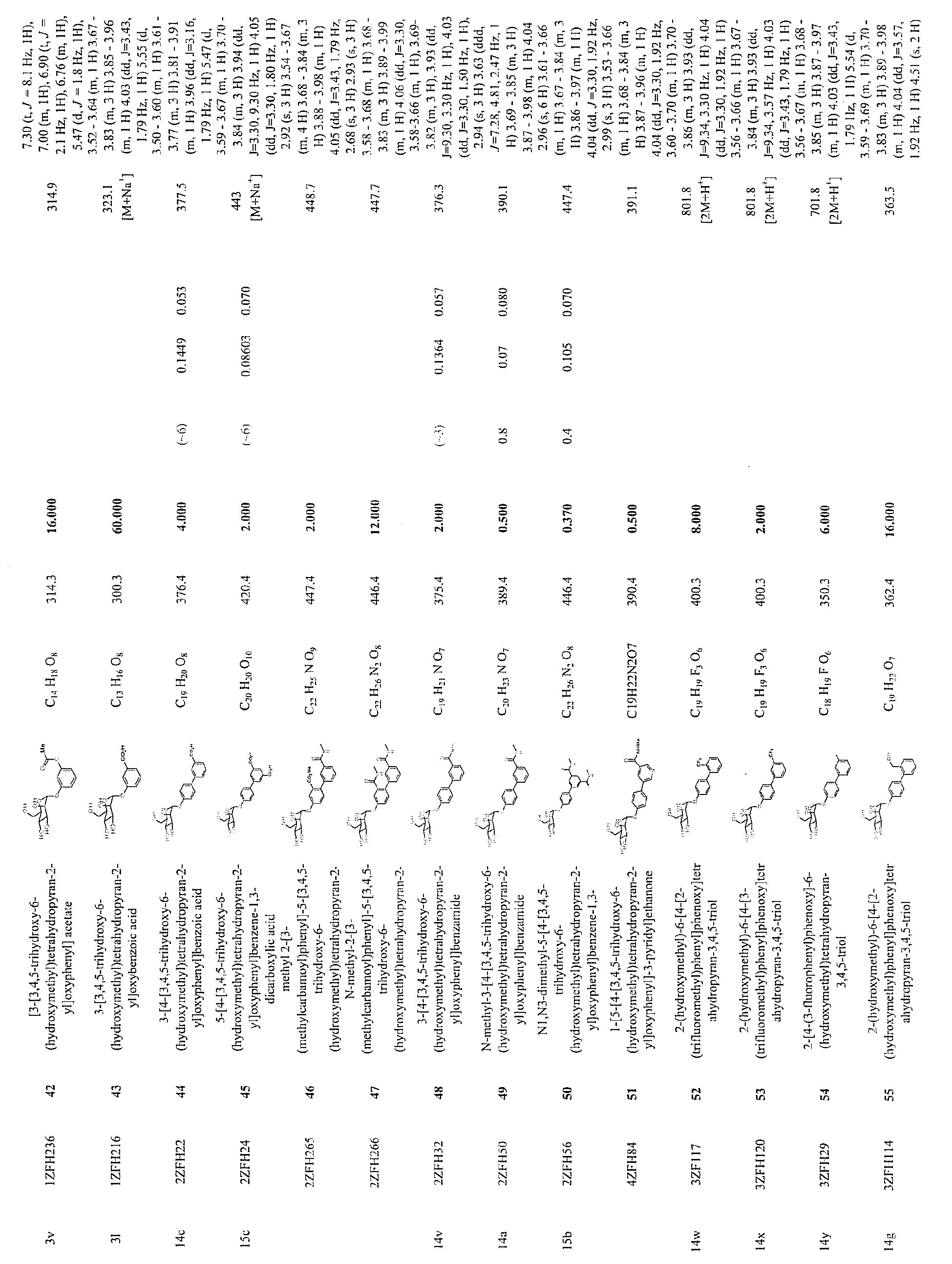

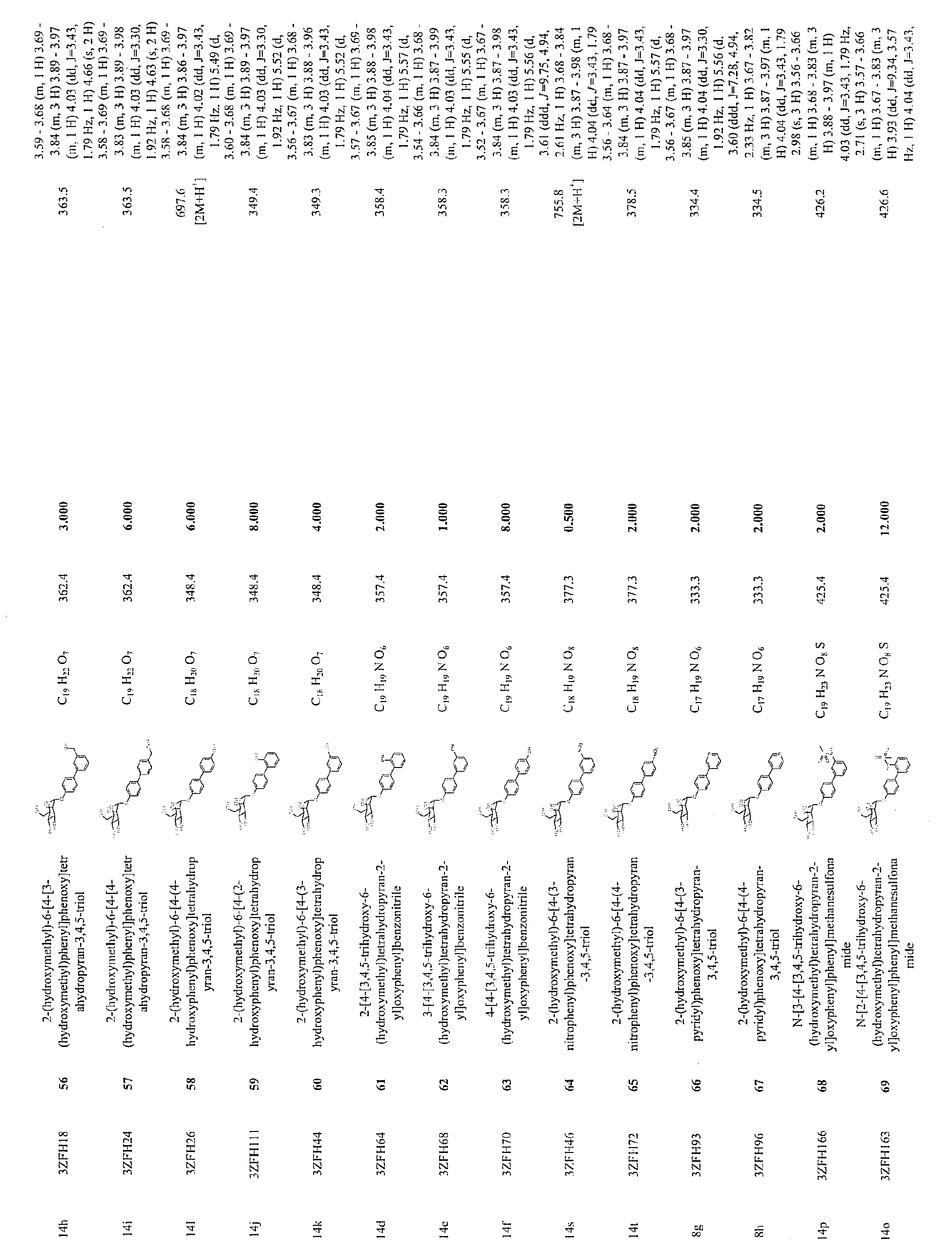

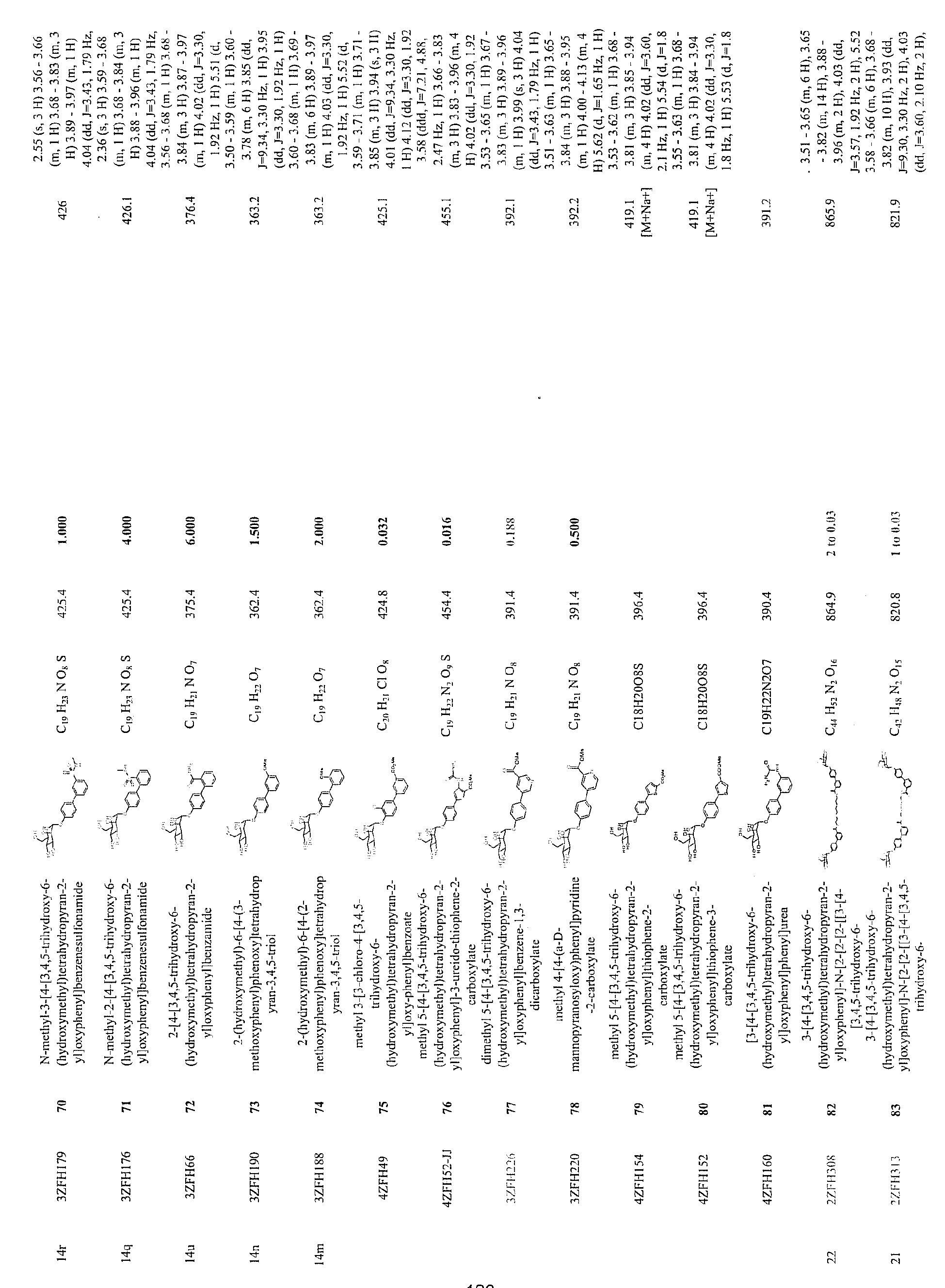

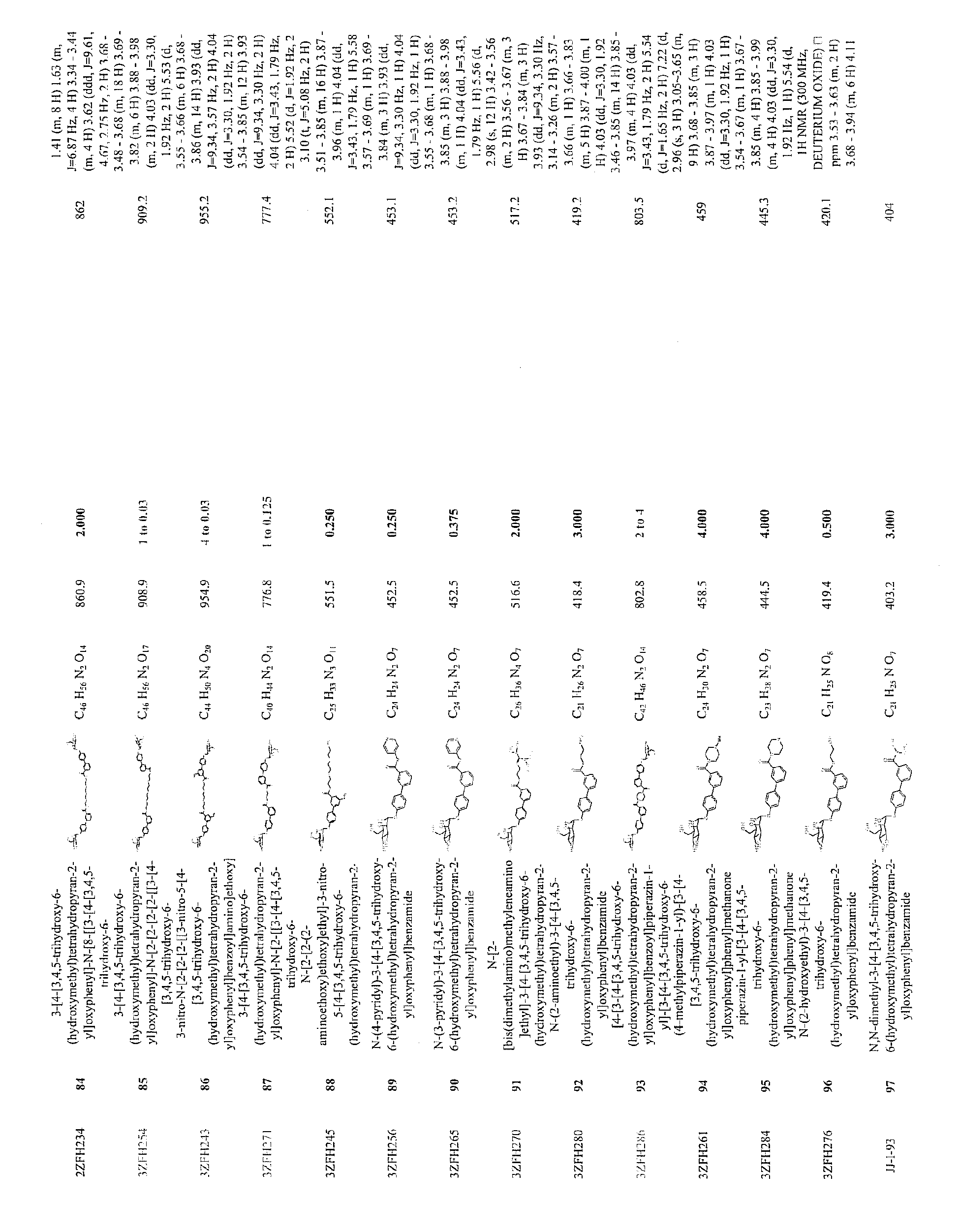

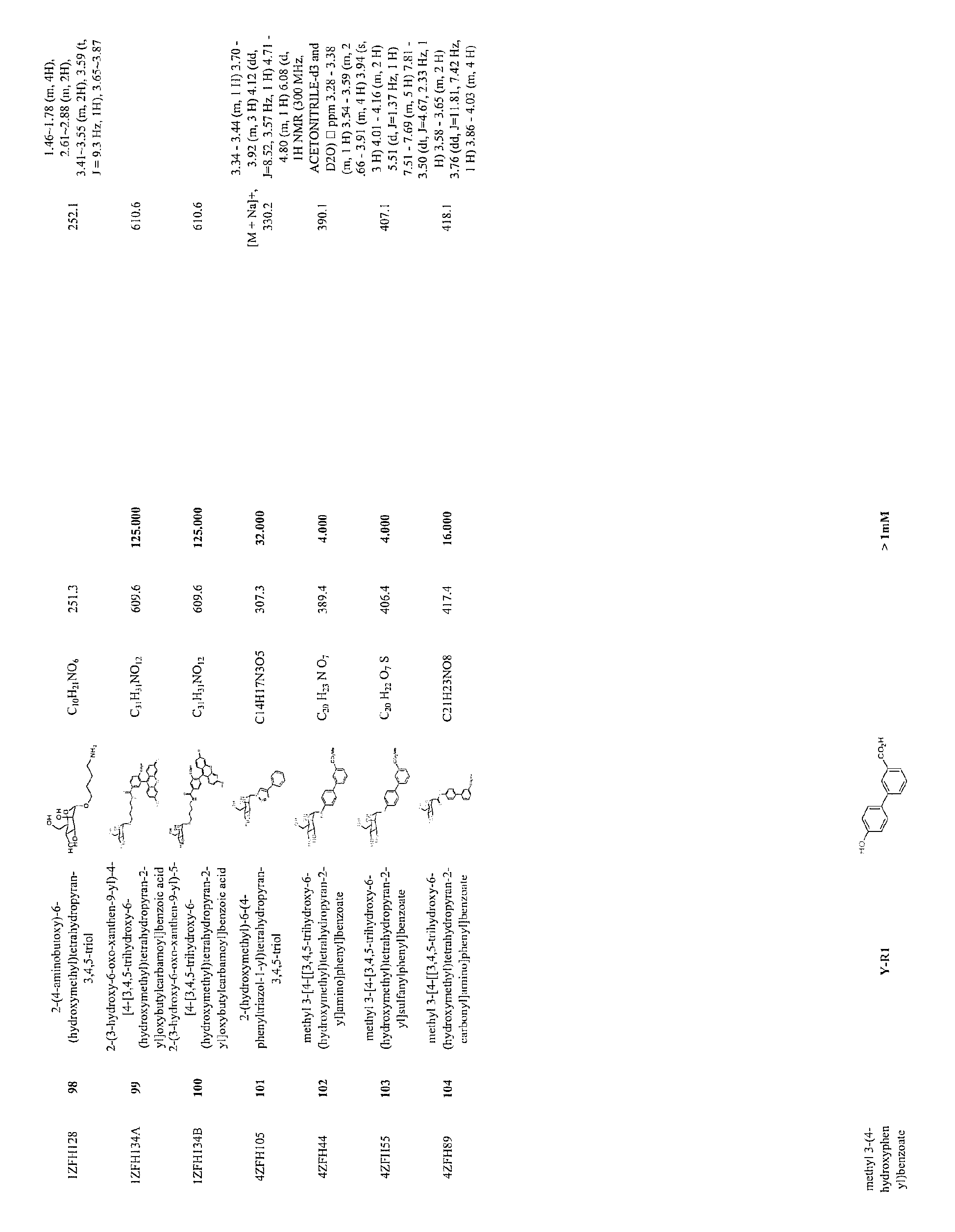

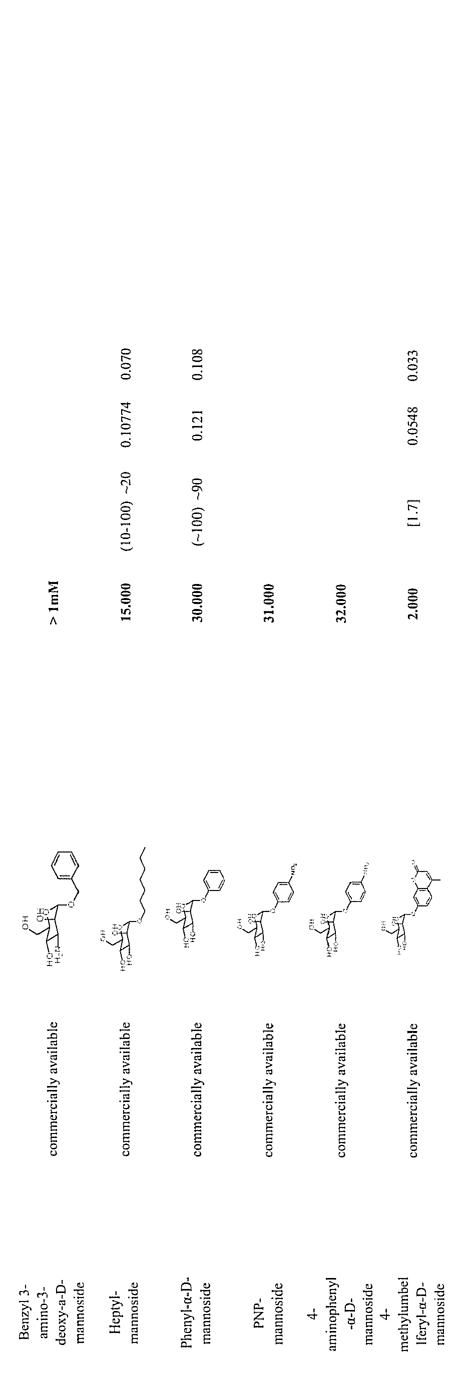

Some preferred aspects and embodiments of the invention are described in the clauses below:
1. A compound, the compound comprising Formula (I): 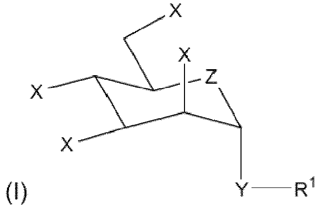 wherein:
   X is selected from the group consisting of hydrogen, OR², SR², and NR^{z};
   Z is selected from the group consisting of O, S, CR³ and NR⁴;
   Y is selected from the group consisting of oxygen, sulfur, CR³, NR⁴, -N(R⁵)CO-, -CH₂N(R⁵)-, -CH₂N(R⁵)CO-, CO₂, SO₂, -CH₂O-, -CH₂S-, CO, -CON(R⁵)-, -SO₂N(R⁵)-, -O(CH₂)ₙ-, -S(CH₂)ₙ-, -N(CH₂)ₙ-, -(CH₂)ₙ-, NR⁵, and an optionally substituted alkyl, alkene, alkyne, or heterocycle;
   R² is independently selected from the group consisting of hydrogen, COR^{x}, CONR^{x}, hydrocarbyl, and substituted hydrocarbyl;
   R³, R⁴, R⁵ are independently selected from the group consisting of hydrogen, hydrocarbyl, and substituted hydrocarbyl;
   R^{z} is independently selected from the group consisting of hydrogen, hydrocarbyl, substituted hydrocarbyl, -COR^{x}, -CONR^{x}R^{x}SO₂R^{x}, and -CO₂R^{x};
   R^{x} is independently selected from the group consisting of hydrogen, NR⁴R⁵, and an optionally substituted alkyl, cycloalkyl, heterocycle, or aryl;
   n is an integer from 1 to 10; and
   R¹ is selected from the group comprising Formulas (IIIB), (IV), (V), (VI), (VII) and (VIII): 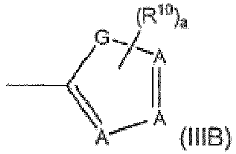 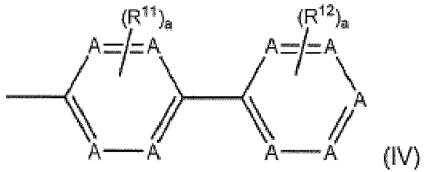 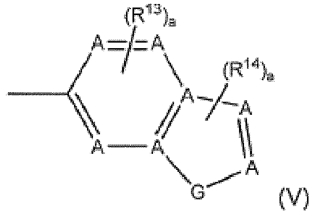 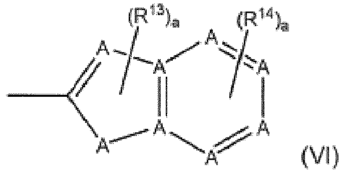 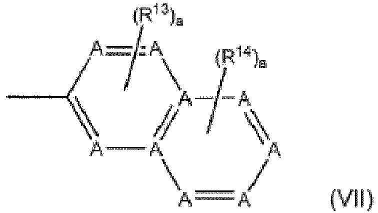 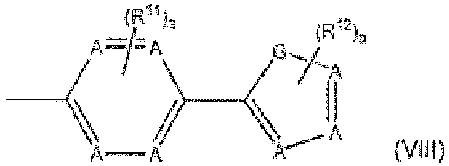 wherein:
      A is independently selected from the group consisting of CR⁶ and N;
      G is selected from the group consisting of S, O, CR⁸, and NR⁹;
      R⁶, R⁸ and R⁹ are independently selected from the group consisting of hydrogen, hydrocarbyl, and substituted hydrocarbyl;
      R¹⁰, R¹¹, R¹², R¹³ and R¹⁴ are independently selected from the group consisting of hydrogen, -OR¹⁵, -NR¹⁵R¹⁶, -NR¹⁵ COR¹⁶, -NR¹⁵CO₂R¹⁶, -NR¹⁵CONR¹⁶,-NR¹⁵SO₂R¹⁶, -COR¹⁵, -SO2R¹⁵, nitro, cyano, halogen, aryl, heterocycle,-COOR¹⁵, -CONR¹⁶R¹⁷, -SO₂NR¹⁸R¹⁹, alkenyl, alkynyl, hydrocarbyl, and substituted hydrocarbyl;
      R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
      a is either the integer 1 or the integer 2.
2. A compound of clause 1, wherein the compound is selected from the group consisting of a compound of formula (X), (XI), (XII), (XIII), (XIV), (XV), (XVI), (XVII), (XVIII), (XIX) and (XX).
3. A compound, the compound comprising Formula (II): 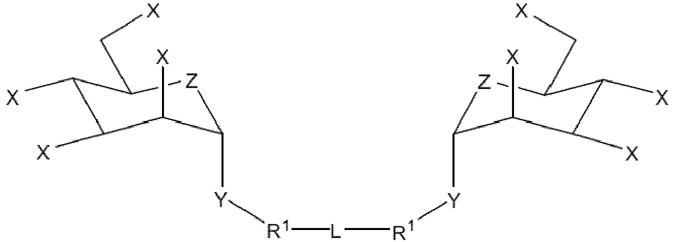
   X is selected from the group consisting of hydrogen, OR², SR², and NR^{z};
   Z is selected from the group consisting of O, S, CR³ and NR⁴;
   Y is selected from the group consisting of oxygen, sulfur, CR³, NR⁴, -N(R⁵)CO-,-CH₂N(R⁵)-, -CH₂N(R⁵)CO-, CO₂, SO₂, -CH₂O-, - CH₂S-, CO, -CON(R⁵)-,-SO₂N(R⁵)-, -O(CH₂)ₙ-, -S(CH₂)ₙ-, - N(CH₂)ₙ-, -(CH₂)ₙ-, NR⁵, and an optionally substituted alkyl, alkene, alkyne, or heterocycle;
   L is selected from -O(CH₂)ₙO-, -S(CH₂)ₙS-, -N(CH₂)ₙN-, -(CH₂)ₙ-,-O[(CH₂)ₘO(CH₂)ₙ]ₓO-, -N(CH₂)ₘO(CH₂)ₙN-, heterocycle, alkene, alkyne, -CON[(CH2)ₘO(CH2)ₙ]ₓNCO-, -SO₂N[(CH2)ₘO(CH2)n]xNO₂S-, and NCO[(CH₂)ₘO(CH₂)ₙ]ₓCON-, where L is bound to a ring of R¹ at a meta or para position;
   R² is independently selected from the group consisting of hydrogen, COR^{x}, CONR^{x}, hydrocarbyl, and substituted hydrocarbyl;
   R³, R⁴, R⁵ are independently selected from the group consisting of hydrogen, hydrocarbyl, and substituted hydrocarbyl,
   R^{z} is independently selected from the group consisting of hydrogen, hydrocarbyl, substituted hydrocarbyl, -COR^{x}, -CONR^{x}R^{x}SO₂R^{x}, and -CO₂R^{x};
   R^{x} is independently selected from the group consisting of hydrogen, NR⁴R⁵, and an optionally substituted alkyl, cycloalkyl, heterocycle, or aryl;
   m, n, and x are integers from 1 to 10; and
   R¹ is selected from the group comprising Formulas (IIIA), (IIIB), (IV), (V), (VI), (VII) and (VIII): 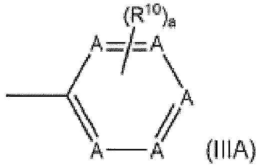 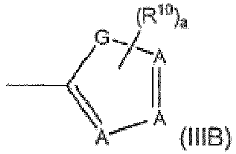 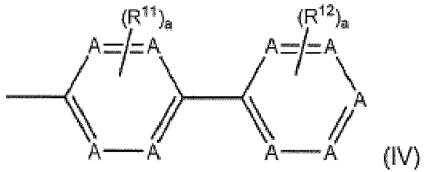 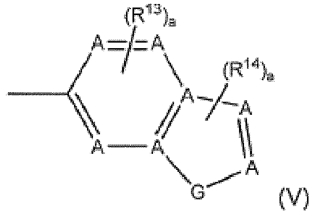 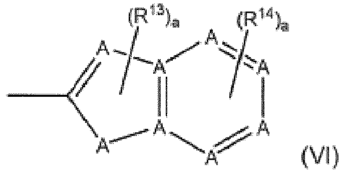 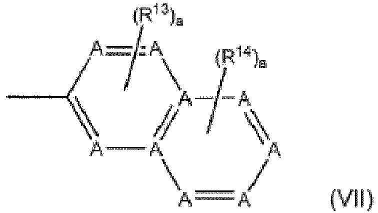 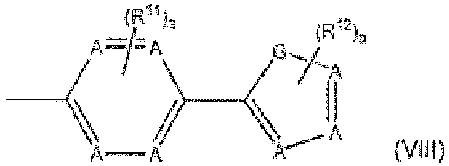 wherein:
      A is independently selected from the group consisting of CR⁶ and N;
      G is selected from the group consisting of S, O, CR⁸, and NR⁹;
      R⁶, R⁸ and R⁹ are independently selected from the group consisting of hydrogen, hydrocarbyl, and substituted hydrocarbyl;
      R¹⁰, R¹¹, R¹², R¹³ and R¹⁴ are independently selected from the group consisting of hydrogen, -OR¹⁵, -NR¹⁵R¹⁶, -NR¹⁵COR¹⁶, -NR¹⁵CO₂R¹⁶, -NR¹⁵CONR¹⁶,-NR¹⁵SO₂R¹⁶, -COR¹⁵, -SO₂R¹⁵, nitro, cyano, halogen, aryl, heterocycle,-COOR¹⁵, -CONR¹⁶R¹⁷, -SO₂NR¹⁸R¹⁹, alkenyl, alkynyl, hydrocarbyl, and substituted hydrocarbyl;
      R¹⁵, R¹⁶, R¹⁷, R¹⁸, R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring; and
      a is either the integer 1 or the integer 2.
4. A method of treating a urinary tract infection, the method comprising administering a compound of clause 1 or clause 2 to a subject in need thereof.
5. The method of clause 4, wherein the subject is further administered a bactericidal composition.
6. The method of clause 4, wherein the compound of clause 1 is selected from the group consisting of a compound of formula (X), (XI), (XII), (XIII), (XIV), (XV), (XVI), (XVII), (XVIII), (XIX) and (XX).
7. A method of reducing the resistance of a bacterium to a bactericidal compound, the method comprising administering a compound of clause 1 or clause 2 to a subject in need thereof.
8. The method of clause 7, wherein the compound of clause 1 is selected from the group consisting of a compound of formula (X), (XI), (XII), (XIII), (XIV), (XV), (XVI), (XVII), (XVIII), (XIX) and (XX).

## Claims

1. A compound of Formula (XVII): 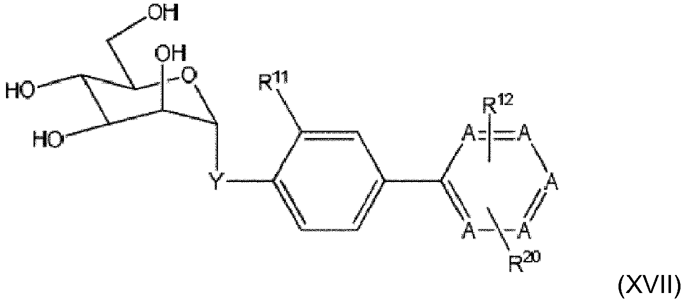 wherein:
Y is selected from the group consisting of oxygen, sulfur, and NR⁴;
A is independently selected from the group consisting of CR⁶ and N;
R⁴ and R⁶ are independently selected from the group consisting of hydrogen, hydrocarbyl, and substituted hydrocarbyl;
R¹¹ is selected from the group consisting of -OR¹⁵, nitro, cyano, chloro, bromo, iodo, fluoro, -COOR¹⁵, -CONR¹⁶R¹⁷, -SO₂R¹⁵, -SO₂NR¹⁸R¹⁹, hydrocarbyl, and substituted hydrocarbyl;
R¹² is selected from the group consisting of -OR¹⁵, -NR¹⁵R¹⁶, -NR¹⁵COR¹⁶,-NR¹⁵CO₂R¹⁶, -NR¹⁵CONR¹⁶, -NR¹⁵SO₂R¹⁶, -COR¹⁵, -SO₂R¹⁵, nitro, cyano, halogen, aryl, heterocycle, -COOR¹⁵, -CONR¹⁶R¹⁷, -SO₂NR¹⁸R¹⁹, hydrocarbyl, and substituted hydrocarbyl;
R²⁰ is selected from the group consisting of NR¹⁵SO₂R¹⁶, -COOR¹⁵,-NR¹⁵CONR¹⁶, -CONR¹⁶R¹⁷, -SO₂NR¹⁸R¹⁹; and
R¹⁵, R¹⁶, R¹⁷, R¹⁸, and R¹⁹ are independently selected from the group consisting of hydrogen, hydrocarbyl, substituted hydrocarbyl, aryl, and heterocycle, and R¹⁶ and R¹⁷ together can optionally form a ring, and R¹⁸ and R¹⁹ together can optionally form a ring.

2. A compound of claim 1 for use in therapy.

3. A compound of claim 1 for use in a method of treating a urinary tract infection, the method comprising administering the compound to a subject in need thereof.

4. The compound for use according to claim 3, wherein the method further comprises administering a bactericidal composition to the subject.

5. A compound of claim 1 for use in a method of reducing the resistance of a bacterium to a bactericidal compound, the method comprising administering the compound to a subject in need thereof.
